# EUROPEAN PATENT SPECIFICATION

(11) **EP 2 265 609 B1**
(45) Date of publication and mention of the grant of the patent: **05.09.2012**
(21) Application number: 09716688.8
(22) Date of filing: 27.02.2009
(51) Int. Cl.: C07D 471/04, C07C 311/08, A61K 31/437, A61P 35/00, A61P 13/12

(54) **IMDIZO [4. 5-B] PYRIDINE DERIVATIVES USED AS RAF INHIBITORS**
ALS RAF-INHIBITOREN VERWENDETE IMIDAZO[4,5-B]PYRIDINDERIVATE
DÉRIVÉS D'IMIDAZO [4. 5-B] PYRIDINE UTILISÉS COMME INHIBITEURS DE RAF

(30) Priority: 29.02.2008 US 32801
(43) Date of publication of application: 29.12.2010
(73) Proprietor: Array Biopharma, Inc., Boulder, CO 80301 (US); Genentech, Inc., South San Francisco, CA 94080-4990 (US)
(72) Inventor: AHRENDT, Kateri, A., Boulder Colorado 80301 (US); BUCKMELTER, Alexandre, J., Boulder Colorado 80301 (US); GRINA, Jonas, Boulder Colorado 80301 (US); HANSEN, Joshua, D., Boulder Colorado 80301 (US); LAIRD, Ellen, R., Boulder Colorado 80301 (US); NEWHOUSE, Brad, Boulder Colorado 80301 (US); REN, Li, Boulder Colorado 80301 (US); WENGLOWSKY, Steven, M., Boulder Colorado 80301 (US); FENG, Bainian, South San Francisco California 94080-4900 (US); MALESKY, Kim, South San Francisco Colorado 94080-4900 (US); MATHIEU, Simon, South San Francisco California 94080-4990 (US); RUDOLPH, Joachim, South San Francisco California 94080-4990 (US); WEN, Zhaoyang, South San Francisco California 94080-4990 (US); YOUNG, Wendy, B., South San Francisco California 94080-4990 (US); MORENO, David, A., Boulder CO 80301 (US)
(74) Representative: Bailey, Sam Rogerson
(86) International application number: PCT/US2009/035379
(87) International publication number: WO 2009/111277

(56) References cited:
- WO-A-2006/040039
- WO-A-2006/042599
- WO-A-2006/066913
- WO-A-2007/017143
- WO-A-2009/012283
- US-A- 4 328 155

## Description

### BACKGROUND OF THE INVENTION

FIELD OF THE INVENTION

The present invention relates to novel compounds, to pharmaceutical compositions comprising the compounds, to a process for making the compounds and to the compounds for use in therapy. More particularly, it relates to certain substituted 3H-imidazo[4,5-b]pyridine compounds useful for inhibiting Raf kinase and for treating disorders mediated thereby.

DESCRIPTION OF THE STATE OF THE ART

The Raf/MEK/ERK pathway is critical for cell survival, growth, proliferation and tumorigenesis. Li, Nanxin, et al. "B-Raf kinase inhibitors for cancer treatment." Current Opinion in Investigational Drugs. Vol. 8, No. 6 (2007): 452-456. Raf kinases exist as three isoforms, A-Raf, B-Raf and C-Raf. Among the three isoforms, studies have shown that B-Raf functions as the primary MEK activator. B-Raf is one of the most frequently mutated genes in human cancers. B-Raf kinase represents an excellent target for anticancer therapy based on preclinical target validation, epidemiology and drugability.

Small molecule inhibitors of B-Raf are being developed for anticancer therapy. Nexavar^{®} (sorafenib tosylate) is a multikinase inhibitor, which includes inhibition of B-Raf, and is approved for the treatment of patients with advanced renal cell carcinoma and unresectable hepatocellular carcinoma. Other Raf inhibitors have also been disclosed or have entered clinical trials, for example SB-590885, RAF-265, PLX-4032 and XL-281. Other B-Raf inhibitors are also known, see for example, U.S. Patent Application Publication 2006/0189627, U.S. Patent Application Publication 2006/0281751, U.S. Patent Application Publication 2007/0049603, International Patent Application Publication WO 2007/002325 and International Patent Application Publication WO 2007/002433.

Imidazopyridines are known, see for example, International Patent Application Publication WO 2007/017143, International Patent Application Publication WO 2006/066913, International Patent Application Publication WO 2004/108133 and International Patent Application Publication WO 2004/024897.

Kinase inhibitors are known, see for example, International Patent Application Publication WO 2005/062795 and International Patent Application Publication WO 2007/013896.

International Patent Application Publication WO 2006/066913, International Patent Application Publication WO 2008/028617 and International Patent Application Publication WO 2009/012283 also disclose kinase inhibitors.
WO 2006/042599 describes various phenylurea derivatives as inhibitors of tyrosine kinase for treatment of tumours. In particular TIE-2 and Raf kinase are targeted.
WO 2006/040039 also describes N,N'-diphenylurea derivatives and their activity also against TIE-2 and Raf kinases for treatment of tumours.
US 4,328,155 describes various meta-sulfonamido-benzamides and indicates some anti-emetic and psychotropic activity.

### SUMMARY OF THE INVENTION

In one aspect, the invention relates to compounds that are inhibitors of Raf kinases, particularly B-Raf inhibitors. Certain hyperproliferative disorders are characterized by the over activation of Raf kinase function, for example by mutations or over expression of the protein. Accordingly, the compounds of the invention are useful in the treatment of hyperproliferative disorders such as cancer.

More specifically, one aspect of the present invention provides compounds of Formula **I**: 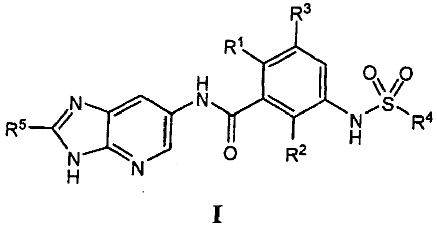 and stereoisomers, tautomers and pharmaceutically acceptable salts thereof, wherein R¹, R², R³, R⁴ and R⁵ are as defined herein.

Another aspect of the present invention provides an effective amount of a compound of this invention or a stereoisomer or pharmaceutically acceptable salt thereof for use in prevention or treatment of a disease or disorder modulated by B-Raf in a mammal. Examples of such diseases and disorders include, but are not limited to, hyperproliferative disorders (such as cancer, including melanoma and other cancers of the skin), neurodegeneration, cardiac hypertrophy, pain, migraine and neurotraumatic disease.

Another aspect of the present invention provides an effective amount of a compound of this invention, or a stereoisomer or pharmaceutically acceptable salt thereof, alone or in combination with one or more additional compounds having anti-cancer properties, for use in prevention or treatment of cancer in a mammal.

Another aspect of the present invention provides the compounds of the present invention for use in therapy.

Another aspect of the present invention provides the compounds of the present invention for use in the treatment of a hyperproliferative disease. In a further embodiment, the hyperproliferative disease may be cancer (or still further, a specific cancer as defined herein).

Another aspect of the present invention provides the compounds of the present invention or a stereoisomer or pharmaceutically acceptable salt thereof, alone or in combination with one or more additional compounds for use in the treatment of a kidney disease. In a further embodiment, the kidney disease may be polycystic kidney disease.

Another aspect of the present invention provides the use of a compound of this invention in the manufacture of a medicament for the treatment of a hyperproliferative disease. In a further embodiment, the hyperproliferative disease may be cancer (or still further, a specific cancer as defined herein).

Another aspect of the present invention provides the use of a compound of this invention in the manufacture of a medicament for the treatment of a kidney disease. In a further embodiment, the kidney disease may be polycystic kidney disease.

Another aspect of the present invention provides the use of a compound of the present invention in the manufacture of a medicament, for use as a B-Raf inhibitor in the treatment of a patient undergoing cancer therapy.

Another aspect of the present invention provides the use of a compound of the present invention in the manufacture of a medicament, for use as a B-Raf inhibitor in the treatment of a patient undergoing polycystic kidney disease therapy.

Another aspect of the present invention provides a pharmaceutical composition comprising a compound of the present invention for use in the treatment of a hyperproliferative disease.

Another aspect of the present invention provides a pharmaceutical composition comprising a compound of the present invention for use in the treatment of cancer.

Another aspect of the present invention provides a pharmaceutical composition comprising a compound of the present invention for use in the treatment of polycystic kidney disease.

Another aspect of the present invention provides a pharmaceutical composition comprising a compound of this invention or a pharmaceutically acceptable salt thereof, and a pharmaceutically acceptable carrier or excipient.

### DETAILED DESCRIPTION OF THE INVENTION

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the claims. One skilled in the art will recognize many methods and materials similar or equivalent to those described herein, which could be used in the practice of the present invention. The present invention is in no way limited to the methods and materials described. In the event that one or more of the incorporated literature and similar materials differs from or contradicts this application, including but not limited to defined terms, term usage, described techniques, or the like, this application controls.

### DEFINITIONS

The term "alkyl" includes linear or branched-chain radicals of carbon atoms. In one example, the alkyl radical is one to six carbon atoms (C₁-C₆). In other examples, the alkyl radical is C₁-C₅, C₁-C₄ or C₁-C₃. Some alkyl moieties have been abbreviated, for example, methyl ("Me"), ethyl ("Et"), propyl ("Pr") and butyl ("Bu"), and further abbreviations are used to designate specific isomers of compounds, for example, 1-propyl or n-propyl ("n-Pr"), 2-propyl or isopropyl ("i-Pr"), 1-butyl or n-butyl ("n-Bu"), 2-methyl-1-propyl or isobutyl ("i-Bu"), 1-methylpropyl or s-butyl ("s-Bu"), 1,1-dimethylethyl or t-butyl ("t-Bu") and the like. Other examples of alkyl groups include 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂) and 3,3-dimethyl-2-butyl (-CH(CH₃)C(CH₃)₃. The abbreviations are sometimes used in conjunction with elemental abbreviations and chemical structures, for example, methanol ("MeOH") or ethanol ("EtOH").

Additional abbreviations used throughout the application include, for example, benzyl ("Bn"), phenyl ("Ph") and acetyl ("Ac").

The term "alkenyl" refers to linear or branched-chain monovalent hydrocarbon radical with at least one site of unsaturation, i.e., a carbon-carbon double bond, wherein the alkenyl radical may be optionally substituted independently with one or more substituents described herein, and includes radicals having "cis" and "trans" orientations, or alternatively, "E" and "Z" orientations. In one example, the alkenyl radical is two to six carbon atoms (C₂-C₆). In other examples, the alkenyl radical is C₂-C₃. Examples include, but are not limited to, ethenyl or vinyl (-CH=CH₂), prop-1-enyl (-CH=CHCH₃), prop-2-enyl (-CH₂CH=CH₂), 2-methylprop-1-enyl, but-1-enyl, but-2-enyl, but-3-enyl, buta-1,3-dienyl, 2-methylbuta-1,3-diene, hex-1-enyl, hex-2-enyl, hex-3-enyl, hex-4-enyl, hexa-1,3-dienyl.

The term "alkynyl" refers to a linear or branched monovalent hydrocarbon radical with at least one site of unsaturation, i.e., a carbon-carbon, triple bond, wherein the alkynyl radical may be optionally substituted independently with one or more substituents described herein. In one example, the alkynyl radical is two to eighteen carbon atoms (C₂-C₆). In other examples, the alkynyl radical is C₂-C₃. Examples include, but are not limited to, ethynyl (-C≡CH), prop-1-ynyl (-C≡CCH₃), prop-2-ynyl (propargyl, CH₂C≡CH), but-1-ynyl, but-2-ynyl and but-3-ynyl.

The terms "alkenyl" and "alkynyl" also include linear or branched-chain radicals of carbon atoms containing at least one unsaturated bond.

"Cycloalkyl" refers to a non-aromatic, saturated or partially unsaturated hydrocarbon ring group wherein the cycloalkyl group may be optionally substituted independently with one or more substituents described herein. In one example, the cycloalkyl group is 3 to 6 carbon atoms (C₃-C₆). In other examples, cycloalkyl is C₃-C₄ or C₃-C₅. In other examples, the cycloalkyl group, as a monocycle, is C₃-C₆ or C₅-C₆. In another example, the cycloalkyl group, as a bicycle, is C₇-C₁₂. Examples of monocyclic cycloalkyl include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, and cyclododecyl. Exemplary arrangements of bicyclic cycloalkyls having 7 to 12 ring atoms include, but are not limited to, [4,4], [4,5], [5,5], [5,6] or [6,6] ring systems. Exemplary bridged bicyclic cycloalkyls include, but are not limited to, bicyclo[2.2.1]heptane, bicyclo[2.2.2]octane, and bicyclo[3.2.2]nonane.

The terms "heterocyclic" or "heterocycle" or "heterocyclyl" refers to a saturated or a partially unsaturated (i.e., having one or more double and/or triple bonds within the ring) cyclic group in which at least one ring atom is a heteroatom independently selected from nitrogen, oxygen, and sulfur, the remaining ring atoms being carbon. In one embodiment, heterocyclyl includes saturated or partially unsaturated 4-6 membered heterocyclyl groups. The heterocyclyl group may be optionally substituted with one or more substituents described herein. Exemplary heterocyclyl groups include, but are not limited to, oxiranyl, aziridinyl, thiiranyl, azetidinyl, oxetanyl, thietanyl, 1,2-dithietanyl, 1,3-dithietanyl, pyrrolidinyl, piperidinyl, dihydropyridinyl, tetrahydropyridinyl, morpholinyl, thiomorpholinyl, thioxanyl, piperazinyl, homopiperazinyl, homopiperidinyl, azepanyl, oxepanyl, thiepanyl, 1,4-oxathianyl, 1,4-dioxepanyl, 1,4-oxathiepanyl, 1,4-oxaazepanyl, 1,4-dithiepanyl, 1,4-thiazepanyl and 1,4-diazepane 1,4-dithianyl, 1,4-azathianyl, oxazepinyl, diazepinyl, thiazepinyl, dihydrothienyl, dihydropyranyl, dihydrofuranyl, tetrahydrofuranyl, tetrahydrothienyl, tetrahydropyranyl, tetrahydrothiopyranyl, 1-pyrrolinyl, 2-pyrrolinyl, 3-pyrrolinyl, indolinyl, 2H-pyranyl, 4H-pyranyl, 1,4-dioxanyl, 1,3-dioxolanyl, pyrazolinyl, pyrazolidinyl, dithianyl, dithiolanyl, pyrazolidinylimidazolinyl, imidazolidinyl, pyrimidinonyl, 1,1-dioxo-thiomorpholinyl, 3-azabicyco[3.1.0]hexanyl, 3-azabicyclo[4.1.0]heptanyl and azabicyclo[2.2.2]hexanyl. Heterocycles include 4 to 6 membered rings containing one or two heteroatoms selected from oxygen, nitrogen and sulfur.

The term "heteroaryl" refers to an aromatic cyclic group in which at least one ring atom is a heteroatom independently selected from nitrogen, oxygen and sulfur, the remaining ring atoms being carbon. Heteroaryl groups may be optionally substituted with one or more substituents described herein. In one example, heteroaryl includes 5-6 membered heteroaryl groups. Other examples of heteroaryl groups include, but are not limited to, pyridinyl, imidazolyl, imidazopyridinyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, 1,2,3-triazolyl, 1,3,4-triazolyl, 1-oxa-2,3-diazolyl, 1-oxa-2,4-diazolyl, 1-oxa-2,5-diazolyl, 1-oxa-3,4-diazolyl, 1-thia-2,3-diazolyl, 1-thia-2,4-diazolyl, 1-thia-2,5-diazolyl, 1-thia-3,4-diazolyl, furazanyl, benzofurazanyl, benzothiophenyl, benzothiazolyl, benzoxazolyl, quinazolinyl, quinoxalinyl, naphthyridinyl, and furopyridinyl. Heteroaryls include 5 to 6 membered aromatic rings containing one, two or three heteroatoms selected from oxygen, nitrogen and sulfur.

"Halogen" refers to F, Cl, Br or I.

The terms "treat" or "treatment" refer to therapeutic, prophylactic, palliative or preventative measures. In one example, treatment includes therapeutic and palliative treatment. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The phrases "therapeutically effective amount" or "effective amount" mean an amount of a compound of the present invention that, when administered to a mammal in need of such treatment, sufficient to (i) treat or prevent the particular disease, condition, or disorder, (ii) attenuate, ameliorate, or eliminate one or more symptoms of the particular disease, condition, or disorder, or (iii) prevent or delay the onset of one or more symptoms of the particular disease, condition, or disorder described herein. The amount of a compound that will correspond to such an amount will vary depending upon factors such as the particular compound, disease condition and its severity, the identity (e.g., weight) of the mammal in need of treatment, but can nevertheless be routinely determined by one skilled in the art.

The terms "cancer" and "cancerous" refer to or describe the physiological condition in mammals that is typically characterized by abnormal or unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small-cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer. The term cancer may be used generically to include various types of cancer or specifically (as listed above).

The phrase "pharmaceutically acceptable" indicates that the substance or composition is compatible chemically and/or toxicologically, with the other ingredients comprising a formulation, and/or the mammal being treated therewith.

The phrase "pharmaceutically acceptable salt," as used herein, refers to pharmaceutically acceptable organic or inorganic salts of a compound of the invention.

The compounds of this invention also include other salts of such compounds which are not necessarily pharmaceutically acceptable salts, and which may be useful as intermediates for preparing and/or purifying compounds of this invention and/or for separating enantiomers of compounds of this invention.

The term "mammal" means a warm-blooded animal that has or is at risk of developing a disease described herein and includes, but is not limited to, guinea pigs, dogs, cats, rats, mice, hamsters, and primates, including humans.

### B-RAF INHIBITOR COMPOUNDS

The present invention provides compounds, and pharmaceutical formulations thereof, that are potentially useful in the treatment of diseases, conditions and/or disorders modulated by B-Raf.

One embodiment of this invention provides compounds of Formula I: 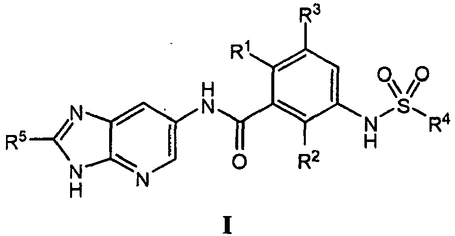 and stereoisomers and pharmaceutically acceptable salts thereof, wherein:
R¹ and R² are independently selected from hydrogen, halogen, CN, C₁-C₃ alkyl, and C₁-C₃ alkoxy;
R³ is hydrogen, halogen or C₁-C₃ alkyl;
R⁴ is C₃-C₅ cycloalkyl, C₁-C₆ alkyl, C₂-C₆ alkenyl, or C₂-C₆ alkynyl, wherein the cycloalkyl, alkyl, alkenyl and alkynyl are optionally substituted with OR^{c}, halogen or C₃-C₄ cycloalkyl;
R⁵ is hydrogen, phenyl optionally substituted with one to three R^{a} groups, -N(R^{c})-phenyl optionally substituted with R^{a}, -CH₂-phenyl optionally substituted with one to three R^{b} groups, a 5-6 membered heteroaryl optionally substituted with one to three R^{e} groups, saturated or partially unsaturated C₃-C₆ cycloalkyl optionally substituted with halogen or C₁-C₄ alkyl, a 5-6 membered heterocyclyl, or C₁-C₆ alkyl optionally substituted with one or more R^{g} groups;
each R^{a} is independently selected from halogen, CN, a 5-6 membered heterocyclyl, NR^{c}R^{d}, -S(O)₂R^{f}, -O(C₁-C₄ alkyl), and C₁-C₄ alkyl, wherein the alkyl or alkoxy are optionally substituted with halogen;
each R^{b} is independently selected from halogen, OH or OCH₃;
each R^{c} and R^{d} are independently selected from hydrogen or C₁-C₄ alkyl;
R^{e} is selected from a 5-6 membered heterocyclyl or NR^{c}R^{d};
R^{f} is selected from C₁-C₄ alkyl or NR^{c}R^{d}; and
each R^{g} is independently selected from halogen, CN, OR^{c}, C₃-C₆ cycloalkyl or NR^{c}R^{d}.

Compounds of Formula I include compounds wherein:
R¹, R² and R³ are independently selected from H, halogen or C₁-C₃ alkyl;
R⁴ is C₃-C₄ cycloalkyl, or C₁-C₆ alkyl optionally substituted with OH, halogen or C₃-C₄ cycloalkyl;
R⁵ is hydrogen, phenyl optionally substituted with one to three R^{a} groups, -NH-phenyl, -CH₂-phenyl optionally substituted with one to three R^{b} groups, a 5-6 membered heteroaryl optionally substituted with one to three R^{e} groups, C₃-C₆ cycloalkyl, a 5-6 membered heterocyclyl, or C₁-C₆ alkyl;
each R^{a} is independently selected from halogen, CN, a 5-6 membered heterocyclyl, NR^{c}R^{d}, -S(O)₂R^{f}, -O(C₁-C₄ alkyl) and C₁-C₄ alkyl, wherein the alkyl or alkoxy are optionally substituted with halogen;
each R^{b} is independently selected from halogen, OH or OCH₃;
each R^{c} and R^{d} are independently selected from hydrogen or C₁-C₄ alkyl;
R^{e} is selected from a 5-6 membered heterocyclyl or NR^{c}R^{d}; and
R^{f} is selected from C₁-C₄ alkyl or NR^{c}R^{d}.

In certain embodiments, R¹ and R² are independently selected from hydrogen, halogen, CN, C₁-C₃ alkyl or C₁-C₃ alkoxy.

In certain embodiments, R¹, R² and R³ are independently selected from hydrogen, halogen or C₁-C₃ alkyl.

In certain embodiments, R¹, R² and R³ are independently selected from hydrogen, F and Cl.

In certain embodiments, R¹ is hydrogen, halogen, CN, C₁-C₃ alkyl or C₁-C₃ alkoxy.

In certain embodiments, R¹ is hydrogen.

In certain embodiments, R¹ is halogen. In certain embodiments, R¹ is F or Cl.

In certain embodiments, R¹ is C₁-C₃ alkyl. In certain embodiments, R¹ is methyl.

In certain embodiments, R² is hydrogen, halogen, CN, C₁-C₃ alkyl or C₁-C₃ alkoxy.

In certain embodiments, R² is hydrogen.

In certain embodiments, R² is halogen. In certain embodiments, R² is F or Cl.

In certain embodiments, R² is C₁-C₃ alkyl. In certain embodiments, R² is methyl.

In certain embodiments of Formula **I**, R² is Cl.

In certain embodiments of Formula **I**, R² is hydrogen.

In certain embodiments, R³ is hydrogen, halogen or C₁-C₃ alkyl.

In certain embodiments, R³ is hydrogen.

In certain embodiments, R³ is halogen. In certain embodiments, R³ is F or Cl.

In certain embodiments, R¹ and R² are F and R³ is hydrogen.

In certain embodiments, R¹ is F and R² is Cl and R³ is hydrogen.

In certain embodiments, R¹ is Cl and R² is F and R³ is hydrogen.

In certain embodiments, R¹ is F and R² and R³ are hydrogen.

In certain embodiments, R¹ and R³ are hydrogen and R² is F.

In certain embodiments, R² and R³ are F and R¹ is hydrogen.

In certain embodiments, R¹ is Cl and R² and R³ are hydrogen.

In certain embodiments, R¹, R² and R³ are F.

In certain embodiments, R¹ is F and R² is methyl and R³ is hydrogen.

In certain embodiments, R¹ is methyl and R² is F and R³ is hydrogen.

In certain embodiments, R¹ is F and R² and R³ are hydrogen.

In certain embodiments, R¹ is Cl and R² and R³ are hydrogen.

In certain embodiments, R² is F and R¹ and R³ are hydrogen.

In certain embodiments, the residue: 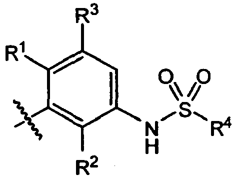 of Formula **I**, wherein the wavy line represents the point of attachment of the residue in Formula **I**, is selected from:

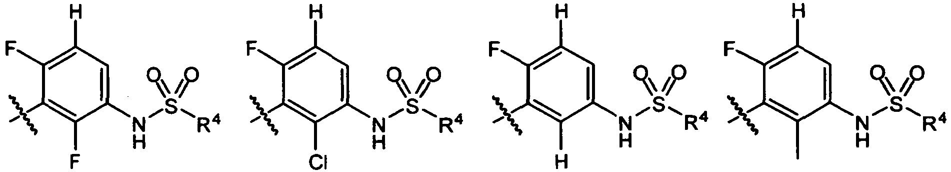

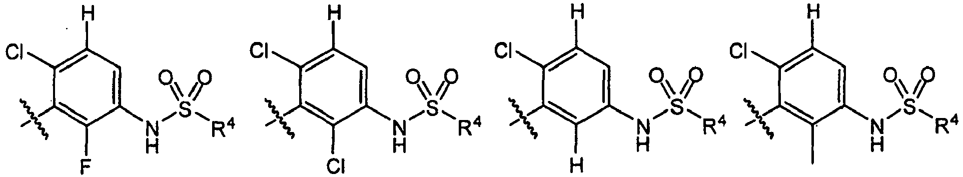

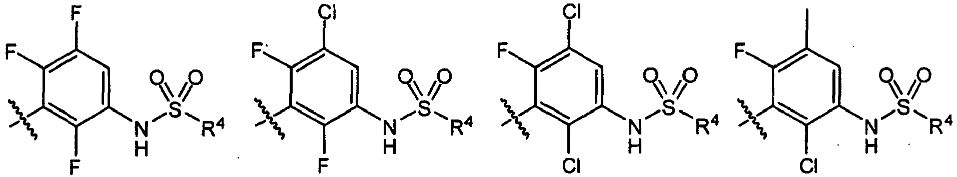

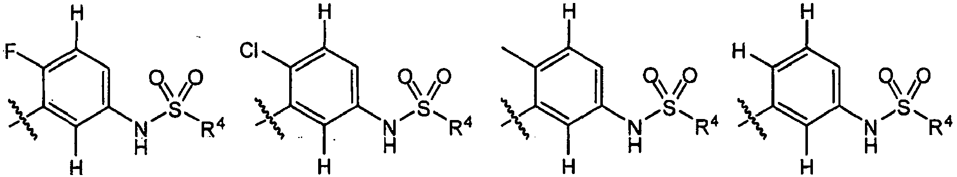

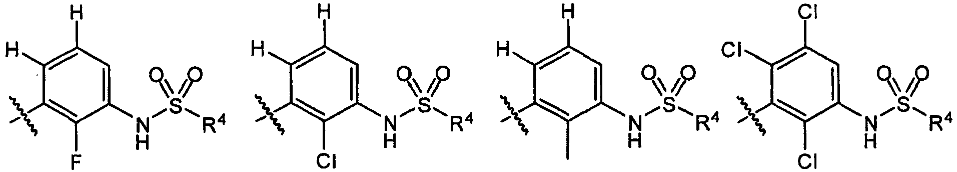

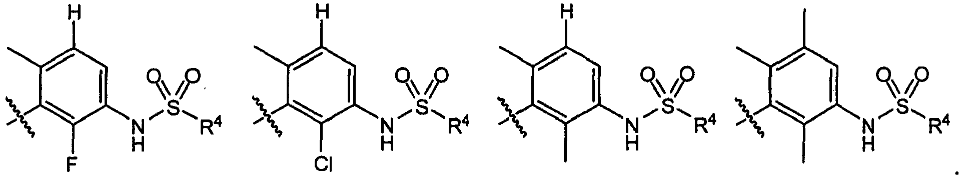

In certain embodiments, R⁴ is C₃-C₅ cycloalkyl, C₁-C₆ alkyl, C₂-C₆ alkenyl, or C₂-C₆ alkynyl, wherein the cycloalkyl, alkyl, alkenyl and alkynyl are optionally substituted with OR^{c}, halogen, or C₃-C₄ cycloalkyl.

In certain embodiments, R^{c} is independently selected from hydrogen, and C₁-C₄ alkyl. In certain embodiments, R^{c} is hydrogen.

In certain embodiments, R⁴ is C₃-C₅ cycloalkyl, C₁-C₆ alkyl, C₂-C₆ alkenyl, or C₂-C₆ alkynyl, wherein the cycloalkyl, alkyl, alkenyl, and alkynyl are optionally substituted with OH, halogen, or C₃₋C₄ cycloalkyl.

In certain embodiments, R⁴ is C₃-C₅ cycloalkyl, C₁-C₆ alkyl, C₂-C₆ alkenyl, or C₂-C₆ alkynyl, wherein the cycloalkyl, alkyl, alkenyl and alkynyl are optionally substituted with OR^{c}, halogen or C₃-C₄ cycloalkyl.

In certain embodiments, R⁴ is cyclopropyl, ethyl, propyl, butyl, isobutyl, -CH₂CH₂CH₂OH, -CH₂Cl, -CH₂CF₃, -CH₂CH₂CH₂F, -CH₂CH₂CF₃, or cyclopropylmethyl.

In certain embodiments, R⁴ is cyclopropyl, ethyl, propyl, isobutyl, -CH₂CH₂CH₂OH, -CH₂CH₂CH₂F, or cyclopropylmethyl.

In certain embodiments, R⁴ is propyl, butyl, isobutyl, -CH₂CH₂CH₂F, -CH₂CH₂CF₃ or cyclopropylmethyl.

In certain embodiments, R⁴ is C₃-C₅ cycloalkyl, or C₁-C₆ alkyl optionally substituted with OH, halogen or C₃-C₄ cycloalkyl.

In certain embodiments, R⁴ is C₃-C₅ cycloalkyl. In certain embodiments, R⁴ is C₃-C₄ cycloalkyl. In certain embodiments, R⁴ is cyclopropyl or cyclobutyl.

In certain embodiments, R⁴ is C₃-C₅ cycloalkyl. In certain embodiments, R⁴ is C₃-C₄ cycloalkyl. In certain embodiments, R⁴ is cyclopropyl.

In certain embodiments, R⁴ is C₁-C₆ alkyl. In certain embodiments, R⁴ is ethyl, propyl, butyl or isobutyl.

In certain embodiments, R⁴ is C₁-C₆ alkyl. In certain embodiments, R⁴ is propyl, butyl or isobutyl.

In certain embodiments, R⁴ is C₁-C₆ alkyl optionally substituted with OR^{c}. In certain embodiments, R^{c} is hydrogen. In certain embodiments, R⁴ is C₁-C₆ alkyl optionally substituted with OH. In certain embodiments, R⁴ is -CH₂CH₂CH₂OH.

In certain embodiments, R⁴ is C₁-C₆ alkyl optionally substituted with halogen. In certain embodiments, R⁴ is -CF₃, -CH₂Cl, -CH₂CF₃, -CH₂CH₂CH₂F, -CH₂CH₂CF₃, -CF₂CF₃ or -CF₂CF₂CF₃.

In certain embodiments, R⁴ is C₁-C₆ alkyl optionally substituted with halogen. In certain embodiments, R⁴ is -CF₃, -CH₂CF₃, -CH₂CH₂CH₂F, -CH₂CH₂CF₃, -CF₂CF₃ or -CF₂CF₂CF₃. In certain embodiments, R⁴ is -CH₂CH₂CH₂F or -CH₂CH₂CF₃.

In certain embodiments, R⁴ is C₁-C₆ alkyl optionally substituted with OR^{c}, halogen or C₃-C₄ cycloalkyl. In certain embodiments, R⁴ is C₁-C₆ alkyl optionally substituted with OH, halogen or C₃-C₄ cycloalkyl. In certain embodiments, R⁴ is cyclopropylmethyl (-CH₂-cyclopropyl) or cyclobutylmethyl (-CH₂-cyclobutyl). In certain embodiments, R⁴ is cyclopropylmethyl (-CH₂-cyclopropyl).

In certain embodiments, R¹ and R² are F, R³ is hydrogen and R⁴ is propyl, such that the compounds of Formula **I**, have the structure of Formula **Ia**: 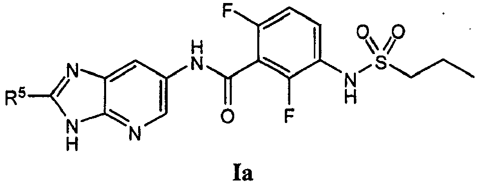 wherein R⁵ is as defined herein.

In certain embodiments, R¹ is Cl, R² is F, R³ is hydrogen and R⁴ is propyl, such that the compounds of Formula **I**, have the structure of Formula **Ia1**: 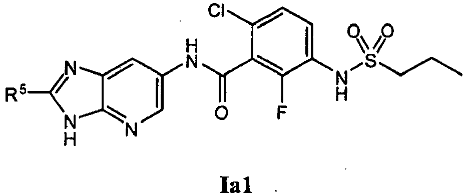 wherein R⁵ is as defined herein.

In certain embodiments, R¹ is F, R² is Cl, R³ is hydrogen and R⁴ is propyl, such that the compounds of Formula **I**, have the structure of Formula **Ia2**: 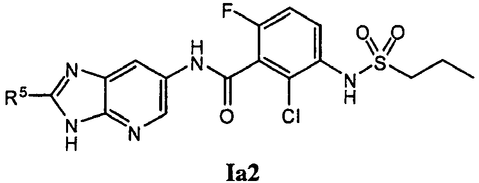 wherein R⁵ is as defined herein.

In certain embodiments, R⁵ is hydrogen, phenyl optionally substituted with one to three R^{a} groups, -N(R^{c})-phenyl optionally substituted with R^{a}, -CH₂-phenyl optionally substituted with one to three R^{b} groups, a 5-6 membered heteroaryl optionally substituted with one to three R^{e} groups, saturated or partially unsaturated C₃-C₆ cycloalkyl optionally substituted with halogen or C₁-C₄ alkyl, a 5-6 membered heterocyclyl, or C₁-C₆ alkyl optionally substituted with one or more R^{g} groups.

In certain embodiments, R⁵ is hydrogen, phenyl optionally substituted with one to three R^{a} groups, -NH-phenyl, -CH₂-phenyl optionally substituted with one to three R^{b} groups, a 5-6 membered heteroaryl optionally substituted with one to three R^{e} groups, C₃-C₆ cycloalkyl, a 5-6 membered heterocyclyl, or C₁-C₆ alkyl.

In certain embodiments, R⁵ is selected from hydrogen, phenyl, 2-fluorophenyl, 2-chlorophenyl, 2-bromophenyl, 3-fluorophenyl, 3-chlorophenyl, 3-bromophenyl, 3-trifluoromethylphenyl, 3-methoxyphenyl, 4-fluorophenyl, 4-chlorophenyl, 4-bromophenyl, 4-cyanophenyl, 4-methylphenyl, 4-trifluoromethylphenyl, 4-methoxyphenyl, 4-trifluoromethoxyphenyl, 4-dimethylaminophenyl, 4-(methylsulfonyl)phenyl, 4-morpholinophenyl, 3,4-dichlorophenyl, 3-trifluoromethyl-4-methylphenyl, 3-trifluoromethyl-4-chlorophenyl, 2-methyl-4-bromophenyl, NH-phenyl, -CH₂-4-chlorophenyl, -CH₂-3,5-difluorophenyl, -CH₂-4-methoxyphenyl, -CH₂-4-bromophenyl, -CH₂-3,4-difluorophenyl, pyridin-3-yl, pyridin-4-yl, 6-morpholinopyridin-3-yl, 6-(dimethylamino)pyridin-3-yl, cyclobutyl, piperdin-3-yl, piperdin-4-yl, methyl, ethyl, isopropyl and tert-butyl.

In certain embodiments, R⁵ is hydrogen.

In certain embodiments, R⁵ is phenyl optionally substituted with one to three R^{a} groups. In certain embodiments, each R^{a} is independently selected from halogen, CN, a 5-6 membered heterocyclyl, NR^{c}R^{d}, -S(O)₂R^{f}, -O(C₁-C₄ alkyl), and C₁-C₄ alkyl, wherein the alkyl or alkoxy are optionally substituted with halogen. In certain embodiments, R^{a} is a 5-6 membered heterocyclyl, wherein the heterocyclyl is morpholinyl. In certain embodiments, R⁵ is selected from phenyl, 2-fluorophenyl, 2-chlorophenyl, 2-bromophenyl, 3-fluorophenyl, 3-chlorophenyl, 3-bromophenyl, 3-trifluoromethylphenyl, 3-methoxyphenyl, 4-fluorophenyl, 4-chlorophenyl, 4-bromophenyl, 4-cyanophenyl, 4-methylphenyl, 4-trifluoromethylphenyl, 4-methoxyphenyl, 4-trifluoromethoxyphenyl, 4-dimethylaminophenyl, 4-(methylsulfonyl)phenyl, 4-morpholinophenyl, 3,4-dichlorophenyl, 3-trifluoromethyl-4-methylphenyl, 3-trifluoromethyl-4-chlorophenyl and 2-methyl-4-bromophenyl.

In certain embodiments, R¹ and R² are F, R³ is hydrogen, R⁴ is propyl and R⁵ is phenyl optionally substituted with one to three R^{a} groups, such that the compounds of Formula **I**, have the structure of Formula **Ib**: 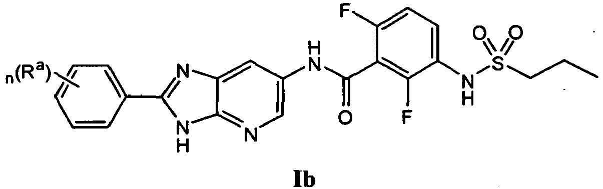 wherein n is 0, 1, 2 or 3, and R^{a} is as defined herein.

In certain embodiments, R⁵ is phenyl.

In certain embodiments, R⁵ is phenyl optionally substituted with one R^{a} group. In certain embodiments, R^{a} is selected from halogen, CN, a 5-6 membered heterocyclyl, NR^{c}R^{d}, -S(O)₂R^{f}, -O(C₁-C₄ alkyl), and C₁-C₄ alkyl, wherein the alkyl or alkoxy are optionally substituted with halogen. In certain embodiments, R^{a} is -O(C₁-C₄ alkyl), wherein R^{a} is methoxy. In certain embodiments, R^{a} is -O(C₁-C₄ alkyl) optionally substituted with halogen, wherein R^{a} is trifluoromethoxy. In certain embodiments, R^{a} is C₁-C₄ alkyl optionally substituted with halogen, wherein R^{a} is trifluoromethyl. In certain embodiments, R^{a} is a 5-6 membered heterocyclyl, wherein the heterocyclyl is morpholinyl. In certain embodiments, R^{c} and R^{d} are C₁-C₄ alkyl. In certain embodiments, R^{c} and R^{d} are methyl. In certain embodiments, R^{a} is -S(O)₂R^{f}. In certain embodiments, R^{f} is C₁-C₄ alkyl. In certain embodiments, R⁵ is selected from 2-fluorophenyl, 2-chlorophenyl, 2-bromophenyl, 3-fluorophenyl, 3-chlorophenyl, 3-bromophenyl, 3-trifluoromethylphenyl, 3-methoxyphenyl, 4-fluorophenyl, 4-chlorophenyl, 4-bromophenyl, 4-cyanophenyl, 4-methylphenyl, 4-trifluoromethylphenyl, 4-methoxyphenyl, 4-trifluoromethoxyphenyl, 4-dimethylaminophenyl, 4-(methylsulfonyl)phenyl and 4-morpholinophenyl.

In certain embodiments, R⁵ is phenyl optionally substituted with two R^{a} groups. In certain embodiments, R^{a} is selected from halogen and C₁-C₄ alkyl, wherein the alkyl is optionally substituted with halogen. In certain embodiments, R^{a} is C₁-C₄ alkyl, wherein R^{a} is methyl. In certain embodiments, R^{a} is C₁-C₄ alkyl optionally substituted with halogen, wherein R^{a} is trifluoromethyl. In certain embodiments, R⁵ is selected from 3,4-dichlorophenyl, 3-trifluoromethyl-4-methylphenyl, 3-trifluoromethyl-4-chlorophenyl and 2-methyl-4-bromophenyl.

In certain embodiments, R⁵ is -N(R^{c})-phenyl optionally substituted with R^{a}. In certain embodiments, R^{c} is selected from hydrogen or C₁-C₄ alkyl. In certain embodiments, R^{c} is hydrogen. In certain embodiments, R⁵ is NH-phenyl optionally substituted with R^{a}.

In certain embodiments, R⁵ is -NH-phenyl optionally substituted with R^{a}. In certain embodiments, R⁵ is NH-phenyl.

In certain embodiments, R⁵ is -CH₂-phenyl optionally substituted with one to three R^{b} groups. In certain embodiments, each R^{b} is independently selected from halogen, OH or OCH₃. In certain embodiments, R⁵ is selected from -CH₂-4-chlorophenyl, -CH₂-3,5-difluorophenyl, -CH₂-4-methoxyphenyl, -CH₂₋4-bromophenyl and -CH₂-3,4-difluorophenyl.

In certain embodiments, R⁵ is a 5-6 membered heteroaryl optionally substituted with one to three R^{e} groups. In certain embodiments, R^{e} is selected from a 5-6 membered heterocyclyl or NR^{c}R^{d}. In certain embodiments, R^{c} and R^{d} are independently selected from hydrogen and C₁-C₄ alkyl. In certain embodiments, R⁵ is a 5-6 membered heteroaryl optionally substituted with a 5-6 membered heterocyclyl, wherein the heteroaryl is pyridinyl. In certain embodiments, R⁵ is a 5-6 membered heteroaryl optionally substituted with a 5-6 membered heterocyclyl, wherein the heterocyclyl is morpholinyl. In certain embodiments, R⁵ is a 5-6 membered heteroaryl optionally substituted with a 5-6 membered heterocyclyl, wherein the heteroaryl is pyridinyl and the heterocyclyl is morpholinyl. In certain embodiments, R⁵ is selected from pyridin-3-yl, pyridin-4-yl, 6-morpholinopyridin-3-yl and 6-(dimethylamino)pyridin-3-yl.

In certain embodiments, R⁵ is saturated or partially unsaturated C₃-C₆ cycloalkyl optionally substituted with halogen or C₁-C₄ alkyl. In certain embodiments, R⁵ is saturated or partially unsaturated C₃-C₆ cycloalkyl. In certain embodiments, R⁵ is saturated C₃-C₆ cycloalkyl. In certain embodiments, R⁵ is C₃-C₆ cycloalkyl, wherein the cycloalkyl is cyclobutyl. In certain embodiments, R⁵ is cyclobutyl.

In certain embodiments, R⁵ is a 5-6 membered heterocyclyl. In certain embodiments, R⁵ is a 5-6 membered heterocyclyl, wherein the heterocyclyl is piperdinyl. In certain embodiments, R⁵ is selected from piperdin-3-yl and piperdin-4-yl.

In certain embodiments, R⁵ is C₁-C₆ alkyl optionally substituted with one or more R^{g} groups. In certain embodiments, each R^{g} is independently selected from halogen, CN, OR^{c}, C₃-C₆ cycloalkyl or NR^{c}R^{d}

In certain embodiments, R⁵ is C₁-C₆ alkyl. In certain embodiments, R⁵ is selected from methyl, ethyl, isopropyl and tert-butyl.

It will be appreciated that certain compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

It will also be appreciated that compounds of Formula **I** include tautomeric forms. Tautomers are compounds that are interconvertible by tautomerization. This commonly occurs due to the migration of a hydrogen atom or proton, accompanied by the switch of a single bond and adjacent double bond. For instance, 3H-imidazo[4,5-b]pyridine is one tautomeric form, while 4H-imidazo[4,5-b]pyridine is another tautomeric form. Other tautomers of Formula **I** may also form at other positions, including, but not limited to, the sulfonamide or R⁵ position depending on the substitution. The compounds of Formula **I** are intended to include all tautomeric forms.

The compounds of Formula **I** include the tautomer 4H-imidazo[4,5-b]pyridine, shown as Formula **II**: 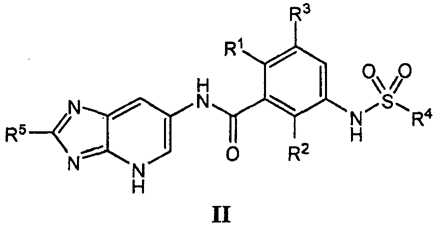 wherein R¹, R², R³, R⁴ and R⁵ are as defined herein.

It will also be appreciated that certain compounds of Formula **I** may be used as intermediates for further compounds of Formula **I.**

It will be further appreciated that the compounds of the present invention may exist in unsolvated, as well as solvated forms with pharmaceutically acceptable solvents, such as water, ethanol, and the like, and it is intended that the invention embrace both solvated and unsolvated forms.

### SYNTHESIS OF COMPOUNDS

Compounds of the present invention may be synthesized by synthetic routes that include processes analogous to those well-known in the chemical arts, particularly in light of the description contained herein. The starting materials are generally available from commercial sources such as Sigma-Aldrich (St. Louis, MO), Alfa Aesar (Ward Hill, MA), or TCI (Portland, OR), or are readily prepared using methods well known to those skilled in the art (e.g., prepared by methods generally described in Louis F. Fieser and Mary Fieser, Reagents for Organic Synthesis. v. 1-23, New York: Wiley 1967-2006 ed. (also available via the Wiley InterScience® website), or Beilsteins Handbuch der organischen Chemie, 4, Aufl. ed. Springer-Verlag, Berlin, including supplements (also available via the Beilstein online database)).

For illustrative purposes, Schemes 1-4 show a general method for preparing the compounds of the present invention, as well as key intermediates. For a more detailed description of the individual reaction steps, see the Examples section below. Those skilled in the art will appreciate that other synthetic routes may be used to synthesize the inventive compounds. Although specific starting materials and reagents are depicted in the Schemes and discussed below, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

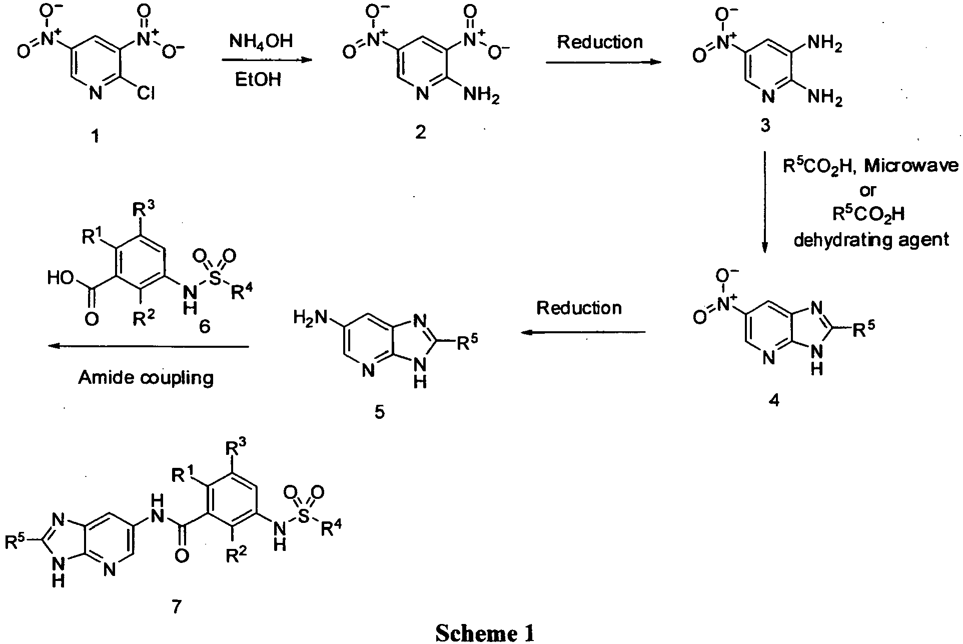

Scheme 1 shows a general scheme for the synthesis of imidazopyridine 7, wherein R¹, R², R³, R⁴, and R⁵ are as defmed herein. Dinitropyridine 1 can be treated with ammonium hydroxide to provide compound 2, which then can be selectively reduced to compound 3 using ammonium sulfide as the reducing agent. Imidazopyridine 4 may be prepared using carboxylic acids in a suitable solvent with various additives, such as triphenylphosphite, under microwave conditions or with a carboxylic acid and a dehydrating agent, such as POCl₃. The nitro group may be reduced by hydrogenation with a suitable catalyst, such as palladium on carbon, tin (II) chloride dehydrate in refluxing methanol, or by zinc or iron in aqueous ammonium chloride, to give compound 5. Compound 5 may be coupled with benzoic acid 6 in the presence of a coupling reagent, such as 2-(1H-benzotriazole-1-yl)-1,1,3,3-tetramethyluronium hexafluorophosphate ("HBTU") or 1-ethyl-(3-dimethylaminopropyl)carbodiimide hydrochloride ("EDCI"), with additives, such as hydroxybenzotriazole monohydrate, in a suitable solvent, such as dichloromethane ("DCM"), N,N-dimethylfonnamide ("DMF") or mixtures thereof, to prepare compound 7.

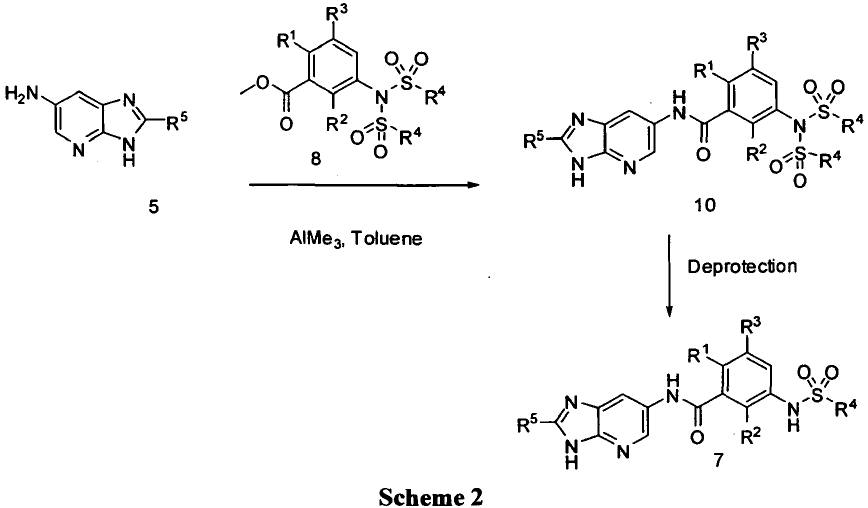

Scheme 2 shows another preparation of imidazopyridine 7, wherein R¹ , R², R³, R⁴ and R⁵ are as defined herein. Imidazopyridine 5 may be coupled with bis-sulfonylated benzoate 8 using Weinreb conditions (trimethylaluminum in toluene) to provide compound 10. Hydrolysis with a suitable base, such as aqueous sodium hydroxide, potassium carbonate, or sodium carbonate, and deprotection provides compound 7.

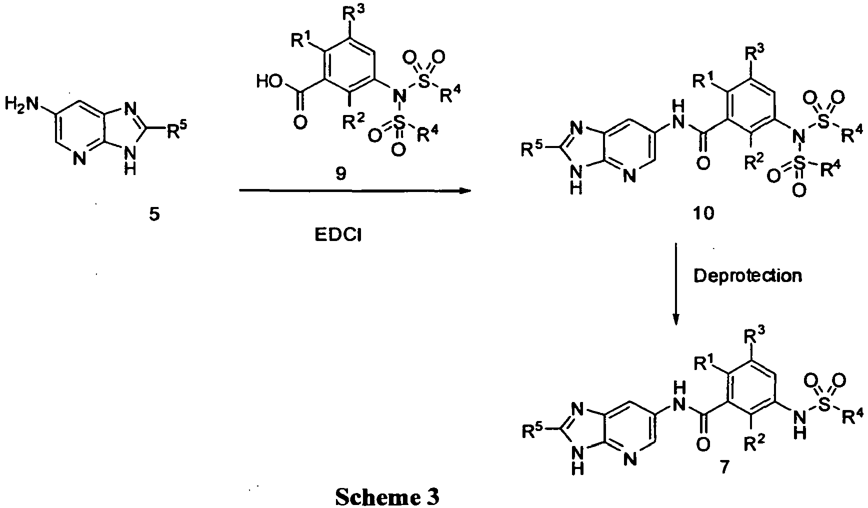

Scheme 3 illustrates yet another preparation of imidazopyridine 7, wherein R¹, R², R³, R⁴ and R⁵ are as defined herein. Imidazopyridine 5 may be coupled with bis-sulfonylated benzoic acid 9 using standard amide coupling conditions, such as those described in Scheme 1, to provide compound 10. Hydrolysis with a suitable base, such as aqueous sodium hydroxide or sodium carbonate, provides compound 7.

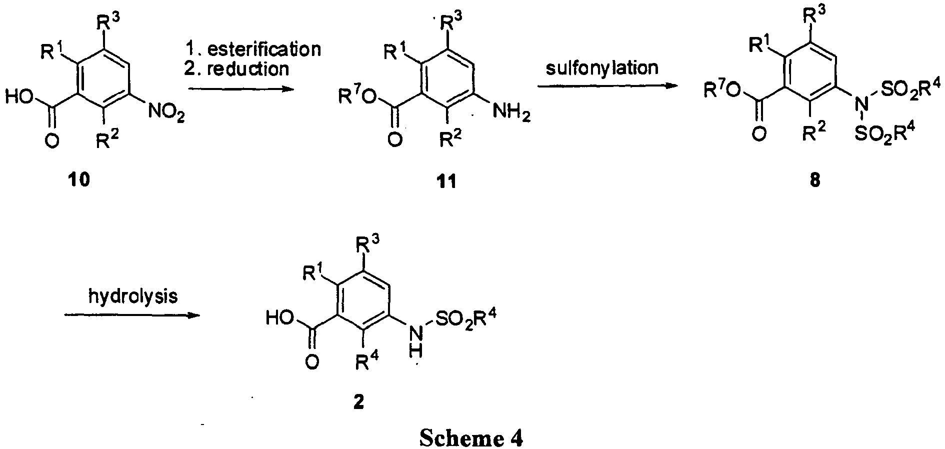

Scheme 4 shows a general method for preparing benzoate 8 and benzoic acid 2, wherein R¹, R², R³ and R⁴ are as defined herein. Benzoic acid 10 is esterified by standard methods, such as by Fischer esterification conditions. The nitro group may be reduced by hydrogenation with a suitable catalyst, such as palladium on carbon. Aniline 11 may be sulfonylated with a substituted sulfonyl chloride in the presence of a suitable base, such as triethylamine, to provide benzoate 8. Hydrolysis of benzoate 8 with a base, such as aqueous sodium hydroxide, in an optional solvent, such as an alcohol (e.g., methanol), tetrahydrofuran ("THF")or a mixture thereof, provides benzoic acid 2.

Accordingly, a process for preparing compounds of Formula **I** may comprise:
(a) coupling a compound of Formula **5**: 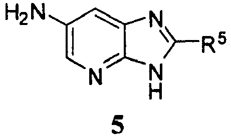 wherein R⁵ is as defined herein, with a compound of Formula **6**: 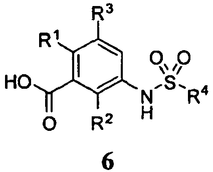 wherein R¹, R², R³ and R⁴ are as defined herein, to provide a compound of Formula **I**; or
(b) coupling a compound of Formula **5**: 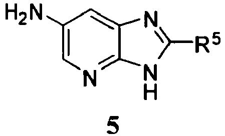 wherein R⁵ is as defined herein, with a compound of Formula **III**: 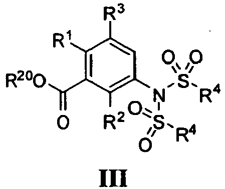 wherein R¹, R², R³, R⁴ and R²⁰ are as defined herein, to provide a compound of Formula **I**.

In preparing compounds of Formula **I**, protection of remote functionalities (e.g., primary or secondary amines, etc.) of intermediates may be necessary. The need for such protection will vary depending on the nature of the remote functionality and the conditions of the preparation methods. Suitable amino-protecting groups (NH-Pg) include acetyl, trifluoroacetyl, t-butyloxycarbonyl ("Boc"), benzyloxycarbonyl ("CBz") and 9-fluorenylmethyleneoxycarbonyl ("Fmoc"). The need for such protection is readily determined by one skilled in the art. For a general description of protecting groups and their use, see T. W. Greene, et al. Greene's Protective Groups in Organic Synthesis. New York: Wiley Interscience, 2006.

### METHODS OF SEPARATION

It may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium and low pressure liquid chromatography methods and apparatus; small scale analytical; simulated moving bed (SMB) and preparative thin or thick layer chromatography, as well as techniques of small scale thin layer and flash chromatography. One skilled in the art will apply techniques most likely to achieve the desired separation.

Diastereomeric mixtures can be separated into their individual diastereomers on the basis of their physical chemical differences by methods well known to those skilled in the art, such as by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereoisomers to the corresponding pure enantiomers. Enantiomers can also be separated by use of a chiral HPLC column.

A single stereoisomer, e.g., an enantiomer, substantially free of its stereoisomer may be obtained by resolution of the racemic mixture using a method such as formation of diastereomers using optically active resolving agents (Eliel, E. and Wilen, S. Stereochemistry of Organic Compounds. New York: John Wiley & Sons, Inc., 1994; Lochmuller, C. H., et al. "Chromatographic resolution of enantiomers: Selective review." J. Chromatogr., 113(3) (1975): pp. 283-302). Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: Wainer, Irving W., Ed. Drug Stereochemistry: Analytical Methods and Pharmacology. New York: Marcel Dekker, Inc., 1993.

Under method (1), diastereomeric salts can be formed by reaction of enantiomerically pure chiral bases such as brucine, quinine, ephedrine, strychnine, α-methyl-β-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, by method (2), the substrate to be resolved is reacted with one enantiomer of a chiral compound to form a diastereomeric pair (Eliel, E. and Wilen, S. Stereochemistry of Organic Compounds. New York: John Wiley & Sons, Inc., 1994, p. 322). Diastereomeric compounds can be formed by reacting asymmetric compounds with enantiomerically pure chiral derivatizing reagents, such as menthyl derivatives, followed by separation of the diastereomers and hydrolysis to yield the pure or enriched enantiomer. A method of determining optical purity involves making chiral esters, such as a menthyl ester, e.g., (-) menthyl chloroformate in the presence of base, or Mosher ester, α-methoxy-a-(trifluoromethyl)phenyl acetate (Jacob III, Peyton. "Resolution of (±)-5-Bromonornicotine. Synthesis of (R)- and (S)-Nornicotine of High Enantiomeric Purity." J. Orig. Chem. Vol. 47, No. 21 (1982): pp. 4165-4167), of the racemic mixture, and analyzing the ¹H NMR spectrum for the presence of the two atropisomeric enantiomers or diastereomers. Stable diastereomers of atropisomeric compounds can be separated and isolated by normal- and reverse-phase chromatography following methods for separation of atropisomeric naphthyl-isoquinolines (WO 96/15111).

By method (3), a racemic mixture of two enantiomers can be separated by chromatography using a chiral stationary phrase Lough, W.J., Ed. Chiral Liquid Chromatography. New York: Chapman and Hall, 1989; Okamoto, Yoshio, et al. "Optical resolution of dihydropyridine enantiomers by high-performance liquid chromatography using phenylcarbamates of polysaccharides as a chiral stationary phase." J. of Chromatogr. Vol. 513 (1990): pp. 375-378). Enriched or purified enantiomers can be distinguished by methods used to distinguish other chiral molecules with asymmetric carbon atoms, such as optical rotation and circular dichroism.

### BIOLOGICAL EVALUATION

B-Raf mutant protein 447-717 (V600E) was co-expressed with the chaperone protein Cdc37, complexed with Hsp90 (Roe, S. Mark, et al. "The Mechanism of Hsp90 Regulation by the Protein Kinase-Specific Cochaperone p50cdc37." Cell. Vol. 116 (2004): pp. 87-98; Stancato, LF, et al. "Raf exists in a native heterocomplex with Hsp90 and p50 that can be reconstituted in a cell free system." J. Biol. Chem. 268(29) (1993): pp. 21711-21716).

Determining the activity of Raf in the sample is possible by a number of direct and indirect detection methods (US 2004/0082014). Activity of human recombinant B-Raf protein may be assessed *in vitro* by assay of the incorporation of radio labeled phosphate to recombinant MAP kinase (MEK), a known physiologic substrate of B-Raf, according to US 2004/0127496 and WO 03/022840. The activity/inhibition of V600E full-length B-Raf was estimated by measuring the incorporation of radio labeled phosphate from [γ³³]ATP into FSBA-modified wild-type MEK (see Example A).

### ADMINISTRATION AND PHARMACEUTICAL FORMULATIONS

The compounds of the invention may be administered by any convenient route appropriate to the condition to be treated. Suitable routes include oral, parenteral (including subcutaneous, intramuscular, intravenous, intraarterial, intradermal, intrathecal and epidural), transdermal, rectal, nasal, topical (including buccal and sublingual), vaginal; intraperitoneal, intrapulmonary and intranasal.

The compounds may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g. diluents, carriers, pH modifiers, sweeteners, bulking agents, and further active agents. If parenteral administration is desired, the compositions will be sterile and in a solution or suspension form suitable for injection or infusion.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

One embodiment of the present invention includes a pharmaceutical composition comprising a compound of Formula **I**, or a stereoisomer or pharmaceutically acceptable salt thereof. In a further embodiment, the present invention provides a pharmaceutical composition comprising a compound of Formula **I**, or a stereoisomer or pharmaceutically acceptable salt thereof, together with a pharmaceutically acceptable carrier or excipient.

Another embodiment of the present invention provides a pharmaceutical composition comprising a compound of Formula **I** for use in the treatment of a hyperproliferative disease.

Another embodiment of the present invention provides a pharmaceutical composition comprising a compound of Formula I for use in the treatment of cancer.

### COMPOUND OF THE INVENTION FOR USE IN TREATMENT

The invention includes one or more compounds ot this invention, or a stereoisomer or pharmaceutically acceptable salt thereof for use in treament or prevention of disease or condition. In one embodiment, a compound of Formula **I**, or a stereoisomer or pharmaceutically acceptable salt thereof, and a pharmaceutically acceptable carrier, adjuvant, or vehicle is used to treat a human patient in an amount to detectably inhibit B-Raf activity.

In another embodiment, a compound of Formula **I**, or a stereoisomer, tautomer or pharmaceutically acceptable salt thereof, and a pharmaceutically acceptable carrier, adjuvant, or vehicle is used to treat a human patient in an amount to detectably inhibit B-Raf activity.

In another embodiment of the present invention, a therapeutically effective amount of the compound of Formula **I**, or a stereoisomer, tautomer or pharmaceutically acceptable salt thereof is provided for use in treatment of a hyperproliferative disease in a mammal.

In another embodiment of the present invention, a therapeutically effective amount of the compound of Formula **I**, or a stereoisomer or pharmaceutically acceptable salt thereof is provided, for use in treatment of a hyperproliferative disease in a mammal.

In another embodiment of the present invention, a therapeutically effective amount of the compound of Formula **I**, or a stereoisomer or pharmaceutically acceptable salt thereof is provided for use in treatment of kidney disease in a mammal. In a further embodiment, the kidney disease is polycystic kidney disease.

In another embodiment, a therapeutically effective amount of the compound of Formula **I**, or a stereoisomer or pharmaceutically acceptable salt thereof is provided for treatment or prevention of cancer in a mammal in need of such treatment. The cancer is selected from breast, ovary, cervix, prostate, testis, genitourinary tract, esophagus, larynx, glioblastoma, neuroblastoma, stomach, skin, keratoacanthoma, lung, epidermoid carcinoma, large cell carcinoma, NSCLC, small cell carcinoma, lung adenocarcinoma, bone, colon, adenoma, pancreas, adenocarcinoma, thyroid, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, hairy cells, buccal cavity and pharynx (oral), lip, tongue, mouth, pharynx, small intestine, colon-rectum, large intestine, rectum, brain and central nervous system, Hodgkin's and leukemia. Another embodiment of the present invention provides the use of a compound of Formula **I**, or a stereoisomer or pharmaceutically acceptable salt thereof, in the manufacture of a medicament for the treatment of cancer.

In another embodiment, a therapeutically effective amount of a compound of Formula **I**, or a stereoisomer, tautomer or pharmaceutically acceptable salt thereof is provided for treatment or prevention of cancer in a mammal in need of such treatment.

Another embodiment of the present invention provides the use of a compound of Formula **I**, or a stereoisomer, tautomer or pharmaceutically acceptable salt thereof, in the manufacture of a medicament for the treatment of cancer.

Another embodiment of the present invention provides the use of a compound of Formula **I**, or a stereoisomer, tautomer or pharmaceutically acceptable salt thereof, in the manufacture of a medicament for the treatment of kidney disease. In a further embodiment, the kidney disease is polycystic kidney disease.

In another embodiment, an effective amount of a compound of Formula **I**, or a stereoisomer, tautomer or pharmaceutically acceptable salt thereof, alone or in combination with one or more additional compounds having anti-cancer properties is provided for prevention or treatment of cancer in a mammal in need of such treatment.

In another embodiment, an effective amount of a compound of Formula **I**, or a stereoisomer or pharmaceutically acceptable salt thereof, alone or in combination with one or more additional compounds having anti-cancer properties is provided for prevention or treatment of cancer in a mammal in need of such treatment.

In one further embodiment, the cancer is a sarcoma.

In another further embodiment, the cancer is a carcinoma. In one further embodiment, the carcinoma is squamous cell carcinoma. In another further embodiment, the carcinoma is an adenoma or adenocarcinoma.

In another embodiment, an effective amount of a compound of Formula **I**, or a stereoisomer or pharmaceutically acceptable salt thereof is provided for treatment or prevention of a disease or disorder modulated by B-Raf in a mammal in need of such treatment. Examples of such diseases and disorders include, but are not limited to, cancer. The cancer is selected from breast, ovary, cervix, prostate, testis, genitourinary tract, esophagus, larynx, glioblastoma, neuroblastoma, stomach, skin, keratoacanthoma, lung, epidermoid carcinoma, large cell carcinoma, NSCLC, small cell carcinoma, lung adenocarcinoma, bone, colon, adenoma, pancreas, adenocarcinoma, thyroid, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, hairy cells, buccal cavity and pharynx (oral), lip, tongue, mouth, pharynx, small intestine, colon-rectum, large intestine, rectum, brain and central nervous system, Hodgkin's and leukemia.

In another embodiment, an effective amount of a compound of Formula I, or a stereoisomer, tautomer or pharmaceutically acceptable salt thereof is provided for treatment or prevention a disease or disorder modulated by B-Raf in a mammal in need of such treatment.

In another embodiment of the present invention, an effective amount of Formula **I**, or a stereoisomer, tautomer or pharmaceutically acceptable salt thereof, alone or in combination with one or more additional compounds is provided for prevention or treatment of kidney disease in a mammal in need of such treatment. In another embodiment of the present invention, an effective amount of a compound of Formula **I**, or a stereoisomer, tautomer or pharmaceutically acceptable salt thereof, alone or in combination with one or more additional compounds is provided for prevention or treatment of polycystic kidney desease in a mammal in need of such treatment.

Another embodiment of the present invention provides the use of a compound of Formula **I**, or a stereoisomer or pharmaceutically acceptable salt thereof, in the manufacture of a medicament for the treatment of cancer. The cancer is selected from breast, ovary, cervix, prostate, testis, genitourinary tract, esophagus, larynx, glioblastoma, neuroblastoma, stomach, skin, keratoacanthoma, lung, epidermoid carcinoma, large cell carcinoma, NSCLC, small cell carcinoma, lung adenocarcinoma, bone, colon, adenoma, pancreas, adenocarcinoma, thyroid, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, hairy cells, buccal cavity and pharynx (oral), lip, tongue, mouth, pharynx, small intestine, colon-rectum, large intestine, rectum, brain and central nervous system, Hodgkin's and leukemia. In a further embodiment, the use of a compound of Formula **I** in the manufacture of a medicament, for use as a b-Raf inhibitor in the treatment of a patient undergoing cancer therapy.

Another embodiment of the present invention provides the use of a compound of Formula **I**, or a stereoisomer, tautomer or pharmaceutically acceptable salt thereof, in the manufacture of a medicament for the treatment of cancer.

Another embodiment of the present invention provides the use of a compound of Formula **I**, or a stereoisomer, tautomer or pharmaceutically acceptable salt thereof, in the manufacture of a medicament for the treatment of polycystic kidney disease. In a further embodiment, the kidney disease is polycystic kidney disease.

Another embodiment of the present invention provides the compounds of Formula **I** for use in therapy.

Another embodiment of the present invention provides the compounds of Formula **I** for use in the treatment of a hyperproliferative disease. In a further embodiment, the hyperproliferative disease is cancer (as further defined and may be individually selected from those above).

Another embodiment of the present invention provides the compounds of Formula **I** for use in the treatment of kidney disease. In a further embodiment, the kidney disease is polycystic kidney disease.

### COMBINATION THERAPY

The compounds of this invention and stereoisomers and pharmaceutically acceptable salts thereof may be employed alone or in combination with other therapeutic agents for treatment. The compounds of the present invention can be used in combination with one or more additional drugs, for example an anti-hyperproliferative, anti-cancer or chemotherapeutic agent. The second compound of the pharmaceutical combination formulation or dosing regimen preferably has complementary activities to the compound of this invention such that they do not adversely affect each other. Such agents are suitably present in combination in amounts that are effective for the purpose intended. The compounds may be administered together in a unitary pharmaceutical composition or separately and, when administered separately this may occur simultaneously or sequentially in any order. Such sequential administration may be close in time or remote in time.

A "chemotherapeutic agent" is a chemical compound useful in the treatment of cancer, regardless of mechanism of action. Chemotherapeutic agents include compounds used in "targeted therapy" and conventional chemotherapy. A number of suitable chemotherapeutic agents to be used as combination therapeutics are contemplated for use in the methods of the present invention. The present invention contemplates, but is not limited to, administration of numerous anticancer agents, such as: agents that induce apoptosis; polynucleotides (e.g., ribozymes); polypeptides (e.g., enzymes); drugs; biological mimetics; alkaloids; alkylating agents; antitumor antibiotics; antimetabolites; hormones; platinum compounds; monoclonal antibodies conjugated with anticancer drugs; toxins, and/or radionuclides; biological response modifiers (e.g., interferons [e.g., IFN-a, etc.] and interleukins [e.g., IL-2, etc.], etc.); adoptive immunotherapy agents; hematopoietic growth factors; agents that induce tumor cell differentiation (e.g., all-trans-retinoic acid, etc.); gene therapy reagents; antisense therapy reagents and nucleotides; tumor vaccines; inhibitors of angiogenesis, and the like.

Examples of chemotherapeutic agents include Erlotinib (TARCEVA®, Genentech/OSI Pharm.), Bortezomib (VELCADE®, Millennium Pharm.), Fulvestrant (FASLODEX®, AstraZeneca), Sunitinib (SUTENT®, Pfizer), Letrozole (FEMARA®, Novartis), Imatinib mesylate (GLEEVEC®, Novartis), PTK787/ZK 222584 (Novartis), Oxaliplatin (Eloxatin®, Sanofi), 5-FU (5-fluorouracil), Leucovorin, Rapamycin (Sirolimus, RAPAMUNE®, Wyeth), Lapatinib (TYKERB®, GSK572016, Glaxo Smith Kline), Lonafarnib (SCH 66336), Sorafenib (NEXAVAR®, Bayer), Irinotecan (CAMPTOSAR®, Pfizer) and Gefitinib (IRESSA®, AstraZeneca), AG1478, AG1571 (SU 5271; Sugen), alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaI1 (Angew Chem. Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® (paclitaxel; Bristol-Myers Squibb Oncology, Princeton, N.J.), ABRAXANE™ (Cremophor-free), albumin-engineered nanoparticle formulations of paclitaxel (American Pharmaceutical Partners, Schaumberg, Illinois), and TAXOTERE® (doxetaxel; Rhone-Poulenc Rorer, Antony, France); chloranmbucil; GEMZAR® (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE® (vinorelbine); novantrone; teniposide; edatrexate; daunomycin; aminopterin; capecitabine (XELODA®); ibandronate; CPT-11; topoisomerase inhibitor RFS 2000; difluoromethylornithine (DMFO); retinoids such as retinoic acid; and pharmaceutically acceptable salts, acids and derivatives of any of the above.

Also included in the definition of "chemotherapeutic agent" are: (i) antihormonal agents that act to regulate or inhibit hormone action on tumors such as antiestrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN® rIL-2; a topoisomerase 1 inhibitor such as LURTOTECAN®; ABARELIX® rmRH; (ix) anti-angiogenic agents such as bevacizumab (AVASTIN®, Genentech); and (x) pharmaceutically acceptable salts, acids and derivatives of any of the above.

Also included in the definition of "chemotherapeutic agent" are therapeutic antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth).

Humanized monoclonal antibodies with therapeutic potential as chemotherapeutic agents in combination with the Raf inhibitors of the invention include: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

### EXAMPLES

In order to illustrate the invention, the following Examples are included. However, it is to be understood that these Examples do not limit the invention and are only meant to suggest a method of practicing the invention. Persons skilled in the art will recognize that the chemical reactions described may be readily adapted to prepare a number of other compounds of the invention. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds of the invention.

In the Examples described below, unless otherwise indicated all temperatures are set forth in degrees Celsius. Reagents were purchased from commercial suppliers such as Sigma-Aldrich, Alfa Aesar, or TCI, and were used without further purification unless otherwise indicated.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried.

Column chromatography purification was done on a Biotage system (Manufacturer: Dyax Corporation) having a silica gel column or on a silica SepPak cartridge (Waters) or on a Teledyne Isco Combiflash purification system using prepacked silica gel cartridges. ¹H NMR spectra were recorded on a Varian instrument operating at 400 MHz. ¹H-NMR spectra were obtained as CDCl₃, CD₂Cl₂, CD₃OD D₂O, d₆-DMSO or d₆-acetone solutions (reported in ppm), using tetramethylsilane (0.00 ppm) or residual solvent (CDCl₃: 7.25 ppm; CD₃OD: 3.31 ppm; D₂O: 4.79 ppm; d₆-DMSO: 2.50 ppm; d₆-acetone: 2.05 ppm) as the reference standard. When peak multiplicities are reported, the following abbreviations are used: s (singlet), d (doublet), t (triplet), q (quartet), qn (quintuplet), sx (sextuplet), m (multiplet), br (broadened), dd (doublet of doublets), dt (doublet of triplets). Coupling constants, when given, are reported in Hertz (Hz).

### Example A

### B-Raf IC₅₀ Assay Protocol

Activity of human recombinant B-Raf protein may be assessed *in vitro* by assay of the incorporation of radio labeled phosphate to recombinant MAP kinase (MEK), a known physiologic substrate of B-Raf, according to US 2004/0127496 and WO 03/022840. Catalytically active human recombinant B-Raf protein is obtained by purification from sf9 insect cells infected with a human B-Raf recombinant baculovirus expression vector.

The activity/inhibition of V600E full-length B-Raf was estimated by measuring the incorporation of radio labeled phosphate from [γ-³³P]ATP into FSBA-modified wild-type MEK. The 30-µL assay mixtures contained 25mM Na Pipes, pH 7.2, 100mM KCl, 10mM MgCl₂, 5mM β-glycerophosphate, 100µM Na Vanadate, 4µM ATP, 500 nCi [γ-³³P]ATP, 1µM FSBA-MEK and 20nM V600E full-length B-Raf. Incubations were carried out at 22°C in a Costar 3365 plate (Coming). Prior to the assay, the B-Raf and FSBA-MEK were preincubated together in assay buffer at 1.5x (20 µL of 30nM and 1.5µM, respectively) for 15 minutes, and the assay was initiated by the addition of 10 µL of 10µM ATP. Following the 60-minute incubation, the assay mixtures were quenched by the addition of 100 µL of 25% TCA, the plate was mixed on a rotary shaker for 1 minute, and the product was captured on a Perkin-Elmer GF/B filter plate using a Tomtec Mach III Harvester. After sealing the bottom of the plate, 35 µL of Bio-Safe II (Research Products International) scintillation cocktail were added to each well and the plate was top-sealed and counted in a Topcount NXT (Packard).

The compounds of Examples 1-42 were tested in the above assay and found to have an IC₅₀ of less than 1 µM.

### Reference Example B

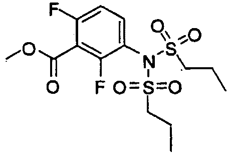

### methyl 2,6-difluoro-3-(N-(propylsulfonyl)propylsulfonamido)benzoate

Step A: A 1 L flask was charged with 2,6-difluoro-3-nitrobenzoic acid (17.0 g, 83.7 mmol) and MeOH (170 mL, 0.5M). The flask was placed in a cold water bath, and an addition funnel charged with a 2M solution of trimethylsilyl ("TMS") diazomethane in hexanes (209 mL, 419 mmol) was attached to the flask. The TMS diazomethane solution was added slowly to the reaction flask over the course of 2 hours. A large excess of reagent was required in order for the reaction to reach completion as determined by the ceased evolution of N₂ upon further addition of reagent. The volatiles were removed in vacuo to afford methyl 2,6-difluoro-3-nitrobenzoate as a solid (18.2 g, 99%). The material was taken directly onto Step B.

Step B: 10% (wt.) Pd on activated carbon (4.46 g, 4.19 mmol) was added to a 1 L flask charged with methyl 2,6-difluoro-3-nitrobenzoate (18.2 g, 83.8 mmol) under a nitrogen atmosphere. EtOH (350 mL, 0.25 M) was added, and then H₂ was passed through the reaction mixture for 15 minutes. The reaction mixture was stirred under two H₂ balloons overnight. The following day the reaction mixture was re-flushed with fresh H₂ balloons and stirred an additional 4 hours. Upon consumption of the starting material and intermediate hydroxylamine as determined by thin layer chromatography ("TLC"), N₂ gas was flushed through the reaction mixture. The mixture was then filtered through glass microfibre filter ("GF/F") paper twice. The volatiles were removed to afford methyl 3-amino-2,6-difluorobenzoate as an oil (15.66 g, 99%). The material was taken directly onto the next step.

Step C: Propane-1-sulfonyl chloride (23.46 mL, 209.3 mmol) was slowly added to a solution of methyl 3-amino-2,6-difluorobenzoate (15.66 g, 83.7 mmol) and triethylamine (35.00 mL, 251.1 mmol) in CH₂Cl₂ (175 mL, 0.5M) maintained in a cool water bath. The reaction mixture was stirred for 1 hour at room temperature. Water (300 mL) was added and the organic layer was separated, washed with water (2 X 300 mL) and brine (200 mL), then dried (Na₂SO₄) filtered and concentrated to an oil. The crude product was purified by column chromatography, eluting with 15% ethyl acetate/hexanes. The isolated fractions were triturated with hexanes to afford methyl 2,6-difluoro-3-(N-(propylsulfonyl) propylsulfonamido)benzoate as a solid (24.4 g, 73% yield for 3 steps). ¹H NMR (400 MHz, CDCl₃) δ 7.52-7.45 (m, 1H), 7.08-7.02 (m, 1H), 3.97 (s, 3H), 3.68-3.59 (m, 2H), 3.53-3.45 (m, 2H), 2.02-1.89 (m, 4H), 1.10 (t, J = 7.4 Hz, 6H). *mlz* (APCI-neg) M-(SO₂Pr = 292.2.

### Example C

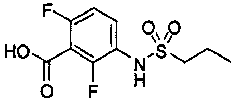

### 2,6-difluoro-3-(propylsulfonamido)benzoic acid

A 1N aqueous NaOH solution (150 mL, 150 mmol) was added to a solution of methyl 2,6-difluoro-3-(N-(propylsulfonyl)propylsulfonamido)benzoate (20.0 g, 50.1 mmol) in 4:1 THF/MeOH (250 mL, 0.2M). The reaction mixture was stirred at room temperature overnight. The majority of the organic solvents were then removed in vacuo (water bath temperature 35°C). 1N HCl (150 mL) was slowly added to the mixture, and the resulting solid was filtered and rinsed with water (4 X 50 mL). The material was then washed with Et₂O (4 X 15 mL) to give 2,6-difluoro-3-(propylsulfonamido)benzoic acid as a solid (10.7 g, 77% yield). ¹H NMR (400 MHz, d₆-DMSO) δ 9.74 (s, 1H), 7.57-7.50 (m, 1H), 7.23-7.17 (m, 1H), 3.11-3.06 (m, 2H), 1.79-1.69 (m, 2H), 0.98 (t, J = 7.4 Hz, 3H). *mlz* (APCI-neg) M-1 = 278.0.

### Reference Example D

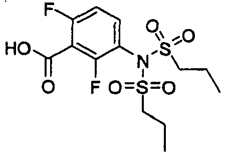

### 2,6-difluoro-3-(N-(pronylsulfonyl)propylsulfonamido)benzoic acid

Propane-1-sulfonyl chloride (1.225 mL, 10.92 mmol) was added to a mixture of 3-amino-2,6-difluorobenzoic acid (0.573 g, 3.310 mmol), triethylamine (2.030 mL, 14.56 mmol) and CH₂Cl₂ (17 mL, 0.2M) cooled to 0°C. The reaction mixture was allowed to warm to room temperature and stirred for 1 hour. The mixture was then partitioned between saturated NaHCO₃ (100 mL) and ethyl acetate (75 mL). The aqueous layer was washed with ethyl acetate (50 mL) and then acidified with concentrated HCl to a pH of about 1. The acidified aqueous layer was extracted with ethyl acetate (2 X 50 mL), and the combined ethyl acetate extracts were dried (Na₂SO₄), filtered and concentrated. The resulting residue was triturated with hexanes to afford 2,6-difluoro-3-(N-(propylsulfonyl)propylsulfonamido)benzoic acid as a solid (0.948 g, 74% yield). ¹H NMR (400 MHz, d₆-DMSO) δ 7.90-7.84 (m, 1H), 7.39-7.34 (m, 1H), 3.73-3.58 (m, 4H), 1.88-1.74 (m, 4H), 1.01 (t, J = 7.5 Hz, 6H). *m*/*z* (APCI-neg) M-(SO₂Pr) = 278.1.

### Example E

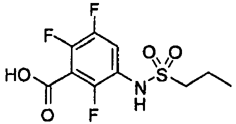

### 2,3,6-trifluoro-5-(propylsulfonamido)benzoic acid

2,3,6-Trifluoro-5-(propylsulfonamido)benzoic acid (8.5%) was prepared according to the general procedure of Example D, substituting 3-amino-2,5,6-trifluorobenzoic acid for 3-amino-2,6-difluorobenzoic acid.

### Reference Example F

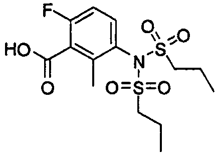

### 6-fluoro-2-methyl-3-(N-(propylsulfonyl)propylsulfonamido)benzoic acid

6-Fluoro-2-methyl-3-(N-(propylsulfonyl)propylsulfonamido)benzoic acid (11%) was prepared according to the general procedure of Example D, substituting 3-amino-6-fluoro-2-methylbenzoic acid for 3-amino-2,6-difluorobenzoic acid.

### Example G

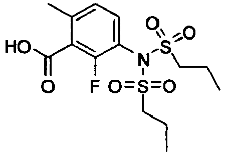

### 2-fluoro-6-methyl-3-(N-(propylsulfonyl)propylsulfonamido)benzoic acid

2-Fluoro-6-methyl-3-(N-(propylsulfonyl)propylsulfonamido)benzoic acid (3%) was prepared according to the general procedure of Example D, substituting 3-amino-2-fluoro-6-methylbenzoic acid for 3-amino-2,6-difluorobenzoic acid.

### Example H

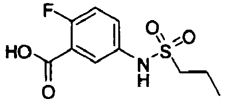

### 2-fluoro-5-(propylsulfonamido)benzoic acid

Propane-1-sulfonyl chloride (0.0871 mL, 0.774 mmol) was dissolved in 10% Na₂CO₃ (1.65 mL, 1.55 mmol) at room temperature. 5-Amino-2-fluorobenzoic acid (0.100 g, 0.645 mmol) was added and heated to 60°C overnight. Propane-1-sulfonyl chloride (0.0871 mL, 0.774 mmol) was added again, and the reaction mixture was heated at 60°C for another hour. The reaction mixture was cooled to room temperature, diluted with water, taken to a pH of 10 with 10% Na₂CO₃ and extracted with DCM (2 X). The reaction mixture was then taken to a pH of 2 with 1N HCl, extracted with DCM (3 X) and concentrated to a solid, 2-fluoro-5-(propylsulfonamido)benzoic acid (29%).

### Example I

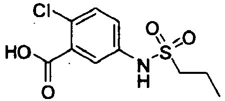

### 2-chloro-5-(propylsulfonamido)benzoic acid

2-Chloro-5-(propylsulfonamido)benzoic acid (14%) was prepared according to the general procedure for Example H, substituting 5-amino-2-chlorobenzoic acid for 5-amino-2-fluorobenzoic acid.

### Example J

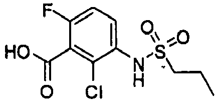

### 2-chloro-6-fluoro-3-(propylsulfonamido)benzoic acid

Step A: 2-Chloro-6-fluorobenzoic acid (2.00 g, 11.5 mmol) was dissolved in sulfuric acid (20 mL) and cooled to 0°C. Nitric acid (0.529 mL, 12.6 mmol) was added, and the reaction mixture was warmed to room temperature for one hour. The reaction mixture was diluted with water, and the aqueous portion was extracted with DCM (3 X), dried over Na₂SO₄, concentrated to a solid, 2-chloro-6-fluoro-3-nitrobenzoic acid (97%), which was used directly in the next step without further purification.

Step B: 2-Chloro-6-fluoro-3-nitrobenzoic acid (0.100 g, 0.455 mmol) and Zn dust (0.298 g, 4.55 mmol) were taken up in tetrahydrofuran (4 mL) and saturated aqueous NH₄Cl (2 mL) and stirred at room temperature overnight. The reaction mixture was filtered through Celite, concentrated to a solid, and dissolved in water. The pH was adjusted to 2 with 1N HCl, and the aqueous portion was extracted with DCM (3 X). The organic portion was dried over Na₂SO₄ and concentrated to a solid, 3-amino-2-chloro-6-fluorobenzoic acid (49%), which was used directly in the next step without further purification.

Step C: 2-Chloro-6-fluoro-3-(propylsulfonamido)benzoic acid (13%) was prepared according to the general procedure for Example H, substituting 3-amino-2-chloro-6-fluorobenzoic acid for 5-amino-2-fluorobenzoic acid.

### Reference Example K

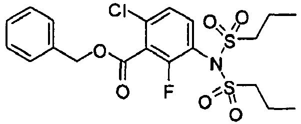

### benzyl 6-chloro-2-fluoro-3-(N-(propylsulfonyl)propylsulfonamido)benzoate

Step A: A flame dried flask equipped with a stir bar and rubber septum was charged with 4-chloro-2-fluoroaniline (5.00 g, 34.35 mmol) and dry THF (170 mL). This solution was chilled to -78°C, and n-BuLi (14.7 mL, 1.07 eq. of 2.5M solution in hexanes) was then added over a 15 minute period. This mixture was stirred at -78°C for 20 minutes, and then a THF solution (25 mL) of 1,2-bis(chlorodimethylsilyl)ethane (7.76 g, 1.05 eq.) was added slowly (over a 10 minute period) to the reaction mixture. This was stirred for 1 hour, and then 2.5M n-BuLi in hexanes (15.11 mL, 1.1 eq.) was added slowly. After allowing the mixture to warm to room temperature for one hour, the mixture was chilled back to -78°C. A third allotment of n-BuLi (15.66 mL, 1.14 eq.) was added slowly, and the mixture was stirred at -78°C for 75 minutes. Benzyl chloroformate (7.40 g, 1.2 eq.) was then added slowly, and the mixture was stirred at -78°C for one hour. The cooling bath was then removed. The mixture was allowed to warm for 30 minutes and then quenched with water (70 mL) and concentrated HCl (25 mL). The mixture was allowed to continue to warm to room temperature. The mixture was then extracted with EtOAc. The extracts were washed twice with a saturated NaHCO₃ solution, once with water, dried over sodium sulfate and concentrated. The resulting residue was flashed on a 65 Biotage (30% ethyl acetate/hexane) to produce benzyl 3-amino-6-chloro-2-fluorobenzoate (4.3 g, 45%) as an oil. ¹H NMR (DMSO-d₆, 400 MHz) δ 7.37-7.48 (m, 5H), 7.07 (dd, 1H, J = 8, 2), 6.87 (t, 1H, J = 8), 5.61 (br s, 2H), 5.40 (s, 2H).

Step B: Benzyl 3-amino-6-chloro-2-fluorobenzoate (4.3 g, 15.37 mmol) was dissolved in dry dichloromethane (270 mL). Triethylamine (5.36 mL, 2.5 eq.) was added, and the mixture was chilled to 0°C. Propane-1-sulfonyl chloride (3.63 mL, 32.3 mmol, 2.1 eq.) was then added via syringe, and a precipitate resulted. Once the addition was complete, the mixture was allowed to warm to room temperature, and the starting material was consumed as determined by TLC (3:1 hexane:ethyl acetate). The mixture was then diluted with dichloromethane (200 mL), washed with 2M aqueous HCl (2 X 100 mL), saturated NaHCO₃ solution, dried over sodium sulfate and concentrated. The resulting residue was purified on a 65 Biotage chromatography system (40% ethyl acetate/hexane) to produce benzyl 6-chloro-2-fluoro-3-(N-(propylsulfonyl)propylsulfonamido)benzoate (5.5 g, 72%) as an oil that slowly solidified upon standing. NMR (CDCl₃, 400 MHz) δ 7.28-7.45 (m, 7H), 5.42 (s, 2H), 3.58-3.66 (m, 2H), 3.43-3.52 (m, 2H), 1.08 (t, 6H, J=8).

### Example L

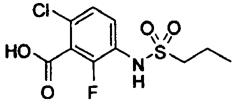

### 6-chloro-2-fluoro-3-(propylsulfonamido)benzoic acid

Benzyl 6-chloro-2-fluoro-3-(N-(propylsulfonyl)propylsulfonamido)benzoate (5.4 g, 10.98 mmol) was dissolved in THF (100 mL) and 1M aqueous KOH (100 mL). This mixture was refluxed for 16 hours and then allowed to cool to room temperature. The mixture was then acidified to a pH of 2 with 2M aqueous HCl and extracted with EtOAc (2 X). The extracts were washed with water, dried over sodium sulfate and concentrated to a solid that was triturated with hexanes/ether to give 6-chloro-2-fluoro-3-(propylsulfonamido)benzoic acid (2.2 g, 68%) as a solid. NMR (DMSO-d₆, 400 MHz) δ 9.93 (s, 1H), 7.49 (t, 1H, J=8), 7.38 (dd, 1H, J = 8, 2), 3.11-3.16 (m, 2H), 1.68-1.78 (m, 2H), 0.97 (t, 3H, J = 8).

### Example M

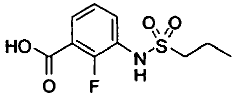

### 2-fluoro-3-(propylsulfonamido)benzoic acid

6-Chloro-2-fluoro-3-(propylsulfonamido)benzoic acid (0.5 g, 1.69 mmol) was dissolved in methanol (15 mL), and Pearlman's catalyst (one weight equivalent, 0.5 g, 20% Pd(OH)₂ on carbon, 50% by weight water) was added. This mixture was subjected to a balloon of hydrogen for 3 hours and then filtered through GF/F filter paper. The filtrate was concentrated to 2-fluoro-3-(propylsulfonamido)benzoic acid (396 mg, 90%) as a solid. MS (M-H+) 262. NMR (DMSO-d₆, 400 MHz) δ 13.36 (s, 1H), 9.76 (s, 1H), 7.58-7.70 (m, 2H), 7.26 (t, 1H, J = 8), 3.10 (t, 2H, J = 8), 1.69-1.80 (m, 2H), 0.98 (t, 3H, J = 8).

### Example N

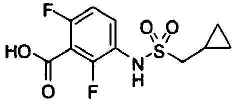

### 3-(cyclopropylmethylsulfonamido)-2,6-difluorobenzoic acid

Step A: Cyclopropylmethanesulfonyl chloride (1.27 g, 8.20 mmol) was added to a mixture of 3-amino-2,6-difluorobenzoic acid (0.430 g, 2.48 mmol), triethylamine (1.52 mL, 10.9 mmol) and CH₂Cl₂ (12 mL, 0.2M) cooled to 0°C. The reaction mixture was allowed to warm to room temperature and stirred for 1 hour. The mixture was then partitioned between saturated NaHCO₃ (75 mL) and ethyl acetate (50 mL). The aqueous layer was washed with ethyl acetate (50 mL) and then acidified to a pH of 1 with concentrated HC1. The acidified aqueous layer was extracted twice with ethyl acetate (2 X 50 mL), and the combined ethyl acetate extracts were dried (Na₂SO₄) filtered and concentrated to provide crude 3-(1-cyclopropyl-N-(cyclopropylmethylsulfonyl)methylsulfonamido)-2,6-difluorobenzoic acid (380 mg, 37%).

Step B: A solution of 1N NaOH (2.78 mL, 2.78 mmol) was added to a solution of crude 3-(1-cyclopropyl-N-(cyclopropylmethylsulfonyl)methylsulfonamido)-2,6-difluorobenzoic acid (380 mg, 0.928 mmol) in 4:1 THF/MeOH (5 mL, 0.2M). The reaction mixture was stirred at room temperature for 1 hour, after which most of the organic solvents were removed. 1N HCl (3 mL) was slowly added to the mixture to acidify to a pH of 1. The acidified aqueous layer was extracted with ethyl acetate (75 mL). The ethyl acetate extract was washed with water (2 X 20 mL), brine (20 mL), dried (Na₂SO₄), filtered and concentrated. Trituration of the residue with Et₂O afforded 3-(cyclopropylmethylsulfonamido)-2,6-difluorobenzoic acid as a solid (139 mg, 51%). ¹H NMR (400 MHz, d₆-DMSO) δ 9.76 (s, 1H), 7.60-7.54 (m, 1H), 7.22-7.16 (m, 1H), 3.10 (d, J = 7.0 Hz, 2H), 1.10-0.99 (m, 1H), 0.58-0.53 (m, 2H), 0.36-0.31 (m, 2H); *mlz* (APCI-neg) M-1 = 289.9.

### Example O

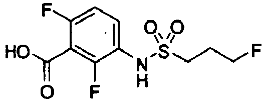

### 2,6-difluoro-3-(3-fluoropropylsulfonamido)benzoic acid

Methyl 2,6-difluoro-3-(N-(3-fluoropropylsulfonyl)-3-fluoropropylsulfonamido)benzoate was made according to the general procedure for Example B, substituting 3-fluoropropyl sulfonyl chloride for propane-1-sulfonyl chloride. ¹H NMR (400 MHz, DMSO-d₆) δ 8.05-7.99 (m, 1H), 7.44 (t, 1H), 4.62 (t, 2H), 4.50 (t, 2H), 3.93 (s, 3H), 3.89-3.74 (m, 4H), 2.26-2.11 (m, 4H).

2,6-Difluoro-3-(3-fluoropropylsulfonamido)benzoic acid was prepared according to the general procedure for Example C, substituting methyl 2,6-difluoro-3-(N-(3-fluoropropylsulfonyl)-3-fluoropropylsulfonamido)benzoate for methyl 2,6-difluoro-3-(N-(propylsulfonyl)-propylsulfonamido)benzoate. ¹H NMR (500 MHz, DMSO-d₆) δ 14.05 (br s, 1H), 9.71 (s, 1H), 7.56-7.50 (m, 1H), 7.20 (t, 1H), 3.12-3.08 (m, 2H), 1.73-1.66 (m, 2H), 1.39 (sx, 2H), 0.87 (t, 3H).

### Example P

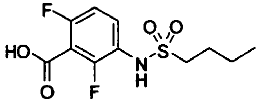

### 3-(butylsulfonamido)-2,6-difluorobenzoic acid

Methyl 2,6-difluoro-3-(N-(butylsulfonyl)-butylsulfonamido)benzoate was made according to the general procedure for Example B, substituting butane-1-sulfonyl chloride for propane-1-sulfonyl chloride. ¹H NMR (500 MHz, DMSO-d₆) δ 7.99-7.94 (m, 1H), 7.42 (t, 1H), 3.92 (s, 3H), 3.74-3.62 (m, 4H), 1.81-1.68 (m, 4H), 1.42 (sx, 4H), 0.89 (t, 6H).

3-(Butylsulfonamido)-2,6-difluorobenzoic acid was prepared according to the general procedure for Example C, substituting methyl 2,6-difluoro-3-(N-(butylsulfonyl)-butylsulfonamido)benzoate for methyl 2,6-difluoro-3-(N-(propylsulfonyl)-propylsulfonamido)benzoate. ¹H NMR (400 MHz, DMSO-d₆) δ 14.05 (br s, 1H), 9.71 (s, 1H), 7.56-7.50 (m, 1H), 7.20 (t, 1H), 3.12-3.08 (m, 2H), 1.73-1.66 (m, 2H), 1.39 (sx, 2H), 0.87 (t, 3H).

### Example Q

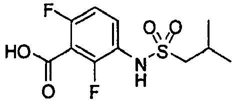

### 2,6.difluoro-3-(2-methylpropylsulfonamido)benzoic acid

Methyl-2,6-difluoro-3-(N-(2-methylpropylsulfonyl)-2-methylpropylsulfonamido)benzoate was made according to the general procedure for Example B, substituting 2-methylpropyl sulfonyl chloride for propane-1-sulfonyl chloride. *m*/*z* (LC-MS) M+1 = 428.4.

2,6-Difluoro-3-(2-methylpropylsulfonamido)benzoic acid was prepared according to the general procedure for Example C, substituting methyl-2,6-difluoro-3-(N-(2-methylpropylsulfonyl)-2-methylpropylsulfonamido)benzoate for methyl 2,6-difluoro-3-(N-(propylsulfonyl)-propylsulfonamido)benzoate. ¹H NMR (400 MHz, DMSO-d₆) δ 14.01 (s, 1H), 9.71 (s, 1H), 7.56 (dd, 1H), 7.22(dd , 1H), 3.02 (d, 2H), 2.18-2.15 (m, 1H), 1.03 (d, 6H); *m*/z (LC-MS) M+1 = 294.3.

### Reference Example R

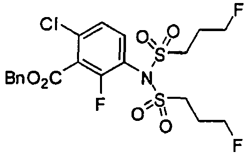

### benzyl 6-chloro-2-fluoro-3-(3-fluoro-N-(3-fluoropropylsulfonyl)propylsulfonamido)benzoate

Benzyl 6-chloro-2-fluoro-3-(3-fluoro-N-(3-fluoropropylsulfonyl)propylsulfonamido)benzoate (92%) was prepared according to the general procedure for Example K, Step B substituting 3-fluoropropane-1-sulfonyl chloride for propane-1-sulfonyl chloride.

### Example S

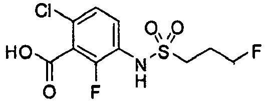

### 6-chloro-2-fluoro-3-(3-fluoropropylsulfonamido)benzoic acid

6-Chloro-2-fluoro-3-(3-fluoropropylsulfonamido)benzoic acid (71%) was prepared according to the general procedure for Example L substituting benzyl 6-chloro-2-fluoro-3-(3-fluoro-N-(3-fluoropropylsulfonyl)propylsulfonamido)benzoate for benzyl 6-chloro-2-fluoro-3-(N-(propylsulfonyl)propylsulfonamido)benzoate.

### Example T

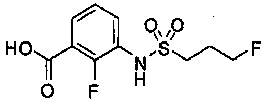

### 2-fluoro-3-(3-fluoropropylsulfonamido)benzoic acid

2-Fluoro-3-(3-fluoropropylsulfonamido)benzoic acid (81%) was prepared according to the general procedure for Example M substituting 6-chloro-2-fluoro-3-(3-fluoropropylsulfonamido)benzoic acid for 6-chloro-2-fluoro-3-(propylsulfonamido)benzoic acid.

### Reference Example U

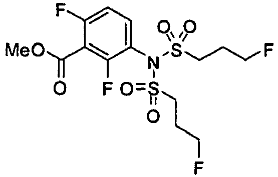

### methyl 2,6-difluoro-3-(3-fluoro-N-(3-fluoropropylsulfonyl)propylsulfonamido)benzoate

3-Fluoropropane-1-sulfonyl chloride (14.3 mL, 129 mmol) was slowly added to a solution of methyl 3-mnino-2,6-difluorobenzoate (24.1 g, 129 mmol) and pyridine (31.2 mL, 386 mmol) in CH₂Cl₂ (360 mL). The reaction mixture was stirred for over two days at room temperature. The reaction mixture was diluted with methylene chloride. The reaction mixture was then washed with an aqueous solution of saturated sodium bicarbonate, 1N HCl, and brine, then dried (Na₂SO₄), filtered and concentrated to an oil to give methyl 2,6-difluoro-3-(3-fluoro-N-(3-fluoropropylsulfonyl)propylsulfonamido)benzoate (38.1 g). ¹H NMR (400 MHz, CDCl₃, ppm) 7.69 (dt, 1H), 7.00 (dt, 1H), 6.55 (s, 1H), 4.56 (dd, 2H), 3.28-3.17 (m, 2H), 2.32-2.15 (m, 2H).

### Example V

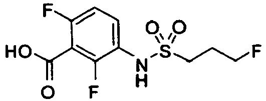

### 2,6-difluoro-3-(3-fluoropropylsulfonamido)benzoic acid

2,6-Difluoro-3-(N-(3-fluoropropylsulfonyl)propylsulfonamido)benzoate (38 g, 120 mmol) was dissolved in 5:2 THF/MeOH (250 mL), and a solution of lithium hydroxide (8.77g, 366 mmol) in water (50 mL) was added. The reaction mixture was stirred at room temperature for four hours. The majority of the organic solvents were then removed in vacuo. 2.5N HCl (500 mL) was slowly added to the mixture, and the resulting solid was filtered and rinsed with cold ether to give 2,6-difluoro-3-(3-fluoropropylsulfonamido)benzoic acid as a solid (29.3 g, 81% yield). ¹H NMR (400 MHz, CDCl₃ ppm) 9.85 (s, 1H), 7.54 (dt, 1H), 7.21 (dt, 1H), 4.54 (td, 2H), 2.20-2.00 (m, 2H), 3.24-3.18 (m, 2H).

### Example W

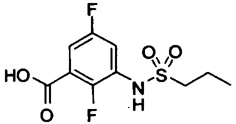

### 2,5-difluoro-3-(propylsulfonamido)benzoic acid

Step A: 2,5-Difluorobenzoic acid (2.01 g, 9.90 mmol, 31.3% yield) was dissolved in concentrated sulfuric acid (25 mL) and cooled to 0°C. Nitric Acid (1.46 mL, 34.8 mmol) was added, and the reaction mixture was stirred at room temperature for one hour. The solution was extracted with DCM (3 X), and the combined organic layers were dried over sodium sulfate and concentrated. The residue was purified by column chromatography (1:1 hexanes:1%HCOOH/EtOAc) giving 2,5-difluoro-3-nitrobenzoic acid (2.01 g, 31.3%) as a solid.

Step B: 2,5-Difluoro-3-nitrobenzoic acid (2.00 g, 9.847 mmol) was dissolved in MeOH (60 mL). TMSCI (6.220 mL, 49.24 mmol) was added, and the reaction mixture was stirred at reflux for 4 hours. The reaction mixture was concentrated to about 20 mL, and the crystals produced were filtered and dried under high vacuum providing methyl 2,5-difluoro-3-nitrobenzoate (1.55 g, 72.4%) as a crystalline solid.

Step C: Methyl 3-amino-2,5-difluorobenzoate (96.5%) was prepared according to the general procedure for Example B, Step B, substituting methyl 2,5-difluoro-3-nitrobenzoate for methyl 2,6-difluoro-3-nitrobenzoate.

Step D: Methyl 2,5-difluoro-3-(N-(propylsulfonyl)propylsulfonamido)benzoate was prepared according to the general procedure for Example B, Step C, substituting methyl 3-amino-2,5-difluorobenzoate for methyl 3-amino-2,6-difluorobenzoate.

Step E: 2,5-Difluoro-3-(propylsulfonamido)benzoic acid (83.8%, two steps) was prepared according to the general procedure for Example C substituting methyl 2,5-difluoro-3-(N-(propylsulfonyl)propylsulfonamido)benzoate for methyl 2,6-difluoro-3-(N-(propylsulfonyl)propylsulfonamido)benzoate. ¹H NMR (400 MHz, d₆-DMSO) δ 13.67 (br s, 1H), 10.07 (s, 1H), 7.46-7.50 (m, 1H), 7.38-7.42 (m, 1H), 3.17-3.21 (m, 2H), 1.70-1.76 (m, 2H), 0.95-0.99 (m, 3H); *m*/*z* (APCI-neg) M-1 = 278.1.

### Example X

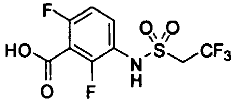

### 2,6-difluoro-3-(2,2,2-trifluoroethylsulfonamido)benzoic acid

Step A: 2,2,2-Trifluoroethyl-sulfonyl chloride (459 mL, 4.15 mmol) was slowly added to a solution of methyl 3-amino-2,6-difluorobenzoate (311 g, 1.66 mmol) and pyridine (0.806 mL, 9.97 mmol) in dichloromethane (8.92 mL, 139 mmol), while applying external cooling using an acetone dry ice bath. The reaction mixture was stirred for 45 minutes, and the dry ice bath was removed. The reaction mixture was kept stirring for another hour. The mixture was diluted with EtOAc (100 mL), washed with water (2 X 25 mL) and brine (25 mL), dried (Na₂SO₄), filtered, and then concentrated to an oil. The crude product was purified by column chromatography, eluting with 15% EtOAc/hexane to afford methyl 2,6-difluoro-3-(2-trifluoroethylsulfonamido) benzoate as a solid (513 mg, 92.6% yield). ¹H NMR (400 MHz, d₆-DMSO) δ 8.10-8.01 (m, 1H), 7.48 (t, 1H), 4.68 (s, 2H), 4.58 (s, 2H), 3.98 (s, 3H).

Step B: 2,6-Difluoro-3-(2-trifluoroethylsulfonamido)benzoic acid was prepared according to the general procedure for Example C, substituting methyl 2,6-difluoro-3-(2-trifluoroethylsulfonamido) benzoate for methyl 2,6-difluoro-3-(N-(propylsulfonyl)-propylsulfonamido)benzoate. ¹H NMR (500 MHz, d₆-DMSO) δ 14.08 (br s, 1H), 9.75 (s, 1H), 7.58-7.52 (m, 1H), 7.25 (t, 1H), 3.15-3.11 (s, 2H).

### Example Y

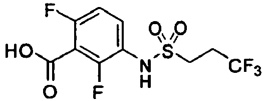

### 2,6-difluoro-3-(3,3,3-trifluoropropylsulfonamido)benzoic acid

Step A: Methyl 2,6-difluoro-3-(N-(3,3,3-trifluoropropylsulfonyl)-3,3,3-trifluoropropyl-sulfonamido) benzoate was made according to the general procedure for Example B, substituting 3,3,3-trifluoropropyl sulfonyl chloride for propane-1-sulfonyl chloride. ¹H NMR (400 MHz, d₆-DMSO) δ 8.05-7.99 (m, 1H), 7.44 (t, 1H), 4.62 (t, 2H), 4.50 (t, 2H), 3.93 (s, 3H), 3.89-3.74 (m, 4H), 2.26-2.11 (m, 4H).

Step B: 2,6-Difluoro-3-(3,3,3-trifluoropropylsulfonamido)benzoic acid was prepared according to the general procedure for Example C, substituting methyl 2,6-difluoro-3-(N-(3,3,3-trifluoropropylsulfonyl)-3,3,3-trifluoropropylsulfonamido)benzoate for methyl 2,6-difluoro-3-(N-(propylsulfonyl)-propylsulfonamido)benzoate. ¹H NMR (500 MHz, d₆-DMSO) δ 14.05 (br s, 1H), 9.71 (s, 1H), 7.56-7.50 (m, 1H), 7.20 (t, 1H), 3.12-3.08 (m, 2H), 1.73-1.66 (m, 2H).

### Example Z

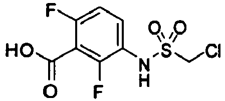

### 2,6-difluoro-3-(2-chloromethylsulfonamido)benzoic acid

Step A: Methyl 2,6-difluoro-3-(N-(2-chloromethylsulfonyl)-2-choromethylsulfonamido) benzoate was made according to the general procedure for Example B, substituting 2-chloromethyl sulfonyl chloride for propane-1-sulfonyl chloride. ¹H NMR (400 MHz, d₆-DMSO) δ 8.08-7.97 (m, 1H), 7.45 (t, 1H), 4.65 (s, 2H), 4.55 (s, 2H), 4.02(s, 3H).

Step B: 2,6-Difluoro-3-(2-chloromethylsulfonamido)benzoic acid was prepared according to the general procedure for Example C, substituting methyl 2,6-difluoro-3-(N-(2-chloromethylsulfonyl)-2-chloromethylsulfonamido)benzoate for methyl 2,6-difluoro-3-(N-(propylsulfonyl)-propylsulfonamido)benzoate. ¹H NMR (500 MHz, d₆-DMSO) δ 14.10 (br s, 1H), 9.78 (s, 1H), 7.62-7.56 (m, 1H), 7.28 (t, 1H), 3.19-3.15 (s, 2H).

### Reference Example AB

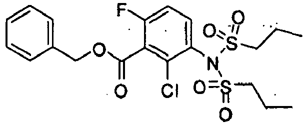

### benzyl 2-chloro-6-fluoro-3-(N-(propylsulfonyl)propylsulfonamido)benzoate

Step A: Benzyl 3-amino-2-chloro-6-fluorobenzoate (56%) was prepared according to the general procedure for Example K, substituting 2-chloro-4-fluoroaniline for 4-chloro-2-fluoroaniline. ¹H NMR (400 MHz, d₆-DMSO) δ 7.48-7.32 (m, 5H), 7.11-7.05 (t, 1H), 6.94-6.89 (q, 1H), 5.53-5.49 (s, 2H), 5.41-5.39 (s, 2H).

Step B: Benzyl 3-amino-2-chloro-6-fluorobenzoate (330 mg, 1.2 mmol) was dissolved in dry dichloromethane (11.8 mL). Triethylamine (0.494 mL, 3.54 mmol) was added, and the mixture was chilled to 0°C. Propane-1-sulfonyl chloride (0.332 mL, 2.95 mmol) was then added via syringe. Once the addition was complete, the mixture was allowed to warm to ambient temperature and stir for 16 hours. The mixture was diluted with dichloromethane (11 mL) and washed with water (2 X 50 mL) and brine (25 mL), dried over sodium sulfate, and concentrated. The resulting residue was applied directly to a silica gel column and eluted with a gradient (5% to 40%) of ethyl acetate-hexanes to provide benzyl 2-chloro-6-fluoro-3-(N-(propylsulfonyl)propylsulfonamido)benzoate (413 mg, 0.840 mmol, 71.1% yield). ¹H NMR (400 MHz, d₆-DMSO) δ 8.00-7.94 (q, 1H), 7.59-7.52 (t, 1H), 7.50-7.35 (m, 5H), 5.48-5.44 (s, 2H), 3.80-3.60 (m, 4H), 1.89-1.75 (m, 4H), 1.05-0.98 (t, 6H).

### Example AC

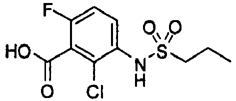

### 2-chloro-6-fluoro-3-(propylsulfonamido)benzoic acid

Step A: Benzyl 2-chloro-6-fluoro-3-(N-(propylsulfonyl)propylsulfonamido)benzoate (413.2 mg, 0.840 mmol) was dissolved in THF (8.4 mL) and 2.0M aqueous LiOH (1.26 mL). The mixture was refluxed for 16 hours and then allowed to cool to ambient temperature. The mixture was acidified to a pH of 0 with 1.0M HCl (5.0 mL) and then adjusted to a pH of 4 using saturated sodium bicarbonate. The mixture was extracted with EtOAc (2 X). The extracts were washed with water (2 X) and brine (1 X), dried over sodium sulfate and concentrated to afford benzyl 2-chloro-6-fluoro-3-(propylsulfonamido)benzoate (174.5 mg, 0.4523 mmol, 53.9% yield). MS (APCI-neg) *m*/*z* = 384.1 (M-H).

Step B: Benzyl 2-chloro-6-fluoro-3-(propylsulfonamido)benzoate (174.5 mg, 0.4523 mmol) was dissolved in 3:1 dioxane:water (7.5 mL) and treated with barium hydroxide (100.7 mg, 0.5879 mmol). The reaction mixture was heated to 80°C for 16 hours and then allowed to cool to ambient temperature. The mixture was acidified to a pH of 0 with concentrated HCl. The reaction mixture was allowed to stir for 10 minutes, after which the pH was adjusted to a pH of 4 using saturated sodium bicarbonate. The mixture was extracted with EtOAc (2 X). The extracts were washed with water (2 X) and brine (1 X), dried over sodium sulfate, and concentrated to afford 2-chloro-6-fluoro-3-(propylsulfonamido)benzoic acid (75.7 mg, 0.2560 mmol, 56.6% yield). MS (APCI-neg) m/z = 293.9 (M-H).

### Example AD

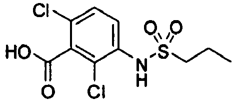

### 2,6-dichloro-3-(propylsulfonamido)benzoic acid

Step A: 2,6-Dichloro-3-nitrobenzoic acid (2.13 g, 9.03 mmol) was dissolved in 2:1 THF:saturated aqueous NH₄Cl and cooled to 0°C. The mixture was treated with zinc (11.8 g, 181 mmol). The reaction mixture was allowed to warm to ambient temperature and stir for 24 hours. The reaction mixture was filtered through GF/F paper while rinsing with THF. The mixture was acidified to a pH of 1 using 1.0M HCl and extracted with 15% 2-propanol:DCM (3 X). The extracts were washed with water and brine, dried over sodium sulfate and concentrated to afford 3-amino-2,6-dichlorobenzoic acid (1.40 g, 6.82 mmol, 75.5% yield). MS (APCI-neg) *mlz* = 203.6 (M-H).

Step B: 3-Amino-2,6-dichlorobenzoic acid (1.40 g, 6.82 mmol) was dissolved in dry dichloromethane (66.7 mL). Triethylamine (4.09 mL, 29.4 mmol) was added, and the mixture was chilled to 0°C. Propane-1-sulfonyl chloride (2.48 mL, 22 mmol) was then added via syringe. Once the addition was complete, the mixture was allowed to warm to ambient temperature and stir for 1 hour. The mixture was concentrated in vacuo and diluted with diethyl ether. The mixture was washed with 0.25M NaOH (80 mL), and the aqueous layer was acidified to a pH of 1 using 1.0M HCl. The aqueous layer was extracted with 15% 2-propanol:DCM (2 X 300 mL). The organic layer was collected, dried over sodium sulfate, and concentrated to afford 2,6-dichloro-3-(propylsulfonamido)benzoic acid (1.55 g, 4.96 mmol, 74.4% yield). ¹H NMR (400 MHz, d₆-DMSO) δ 9.77-9.75 (s, 1H), 7.84-7.80 (d, 1H), 7.71-7.68 (d, 1H), 3.82-3.72 (m, 2H), 1.89-1.70 (m, 2H), 1.05-1.03 (m, 3H).

### Example 1

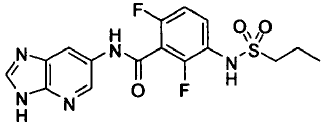

### 2,6-difluoro-N-(3H-imidazo[4,5-b]pyridin-6-yl)-3-(propylsulfonamido)benzamide

Step A: 2-Chloro-3,5-dinitropyridine (6.8 g, 33.41 mmol) was taken up in ethanol (200 mL), and ammonium hydroxide solution (19.5 mL, 167 mmol, 5 eq.) was then dripped in slowly, which resulted in a mixture/precipitate. An exotherm was observed, so the reaction mixture was placed in an ice bath for 10 minutes and then removed. After stirring for about 20 minutes, the solids were collected by filtration and dried under vacuum. 3,5-Dinitropyridin-2-amine (4.8 g, 78%) was obtained as a solid. ¹H NMR (400 MHz, DMSO-d₆) δ 9.26 (br s, 1H), 9.17-9.18 (m, 1H), 8.95-8.97 (m, 1H), 8.70 (br s, 1H).

Step B: 3,5-Dinitropyridin-2-amine (4.0 g, 21.7 mmol) was suspended in methanol (150 mL), and a 20% aqueous solution of ammonium sulfide (31.7 mL, 109 mmol, 5 eq.) was then added. A mixture resulted as the temperature was raised to 75°C for 30 minutes. The mixture was allowed to cool and then placed in an ice bath, where a precipitate formed. The solids were collected by filtration, yielding 5-nitropyridine-2,3-diamine (3.4 g, 100%) as a solid. ¹H NMR (400 MHz, DMSO-d₆) δ 8.28-8.29 (m, 1H), 7.35-7.37 (m, 1H), 6.99 (br s, 2H), 5.32 (br s, 2H).

Step C: 5-Nitropyridine-2,3-diamine (0.050 g, 0.32 mmol) was dissolved in formic acid (10 mL) and heated to 100°C for 6 hours. The reaction was diluted with water (10 mL) and brought to a pH of 7 with 3N NaOH. The aqueous portion was extracted with 25% isopropyl alcohol ("IPA")/DCM (6 X), dried over Na₂SO₄ and concentrated to give 6-nitro-3H-imidazo[4,5-b]pyridine (52 mg, 98%) as a solid.

Step D: 6-Nitro-3H-imidazo[4,5-b]pyridine (0.026 g, 0.158 mmol) was taken up as a slurry in EtOH. 10% Pd/C (0.00843 g, 0.00792 mmol) was added, and hydrogen gas was bubbled through for 10 minutes. The reaction was stirred under a balloon of hydrogen at room temperature overnight. The reaction was filtered through celite, and the filtrate was concentrated to give 3H-imidazo[4,5-b]pyridin-6-amine as a solid that was used without further purification.

Step E: 3H-Imidazo[4,5-b]pyridin-6-amine (29 mg, 0.22 mmol), 2,6-difluoro-3-(propylsulfonamido)benzoic acid (60 mg, 0.22 mmol, 1 eq.), EDCI (46 mg, 0.24 mmol, 1.1 eq.), and 1-hydroxybenzotriazole ("HOBT")·H₂O (33 mg, 0.22 mmol, 1 eq.) were combined in dry DMF (2 mL) and stirred at room temperature for 16 hours. The reaction mixture was then diluted with brine and extracted with ethyl acetate ("EtOAc"; 2 X). The extracts were washed with water (1 X), dried over sodium sulfate and concentrated under reduced pressure. The resulting crude product was purified by preparative TLC (2 X 0.5 mm plates, 10% MeOH/DCM) to give 2,6-difluoro-N-(3H-imidazo[4,5-b]pyridin-6-yl)-3-(propylsulfonamido)benzamide (7.4 mg, 9%). ¹H NMR (400 MHz, DMSO-d₆) δ 12.63 (br s, 1H), 11.03 (br s, 1H), 9.81 (br s, 1H), 8.43-8.53 (m, 3H), 7.53-7.59 (m, 1H), 7.26-7.30 (m, 1H), 3.11-3.15 (m, 2H), 1.74-1.80 (m, 2H), 0.98-1.02 (m, 3H); *m*/*z* (APCI-pos) M+1 = 394.3.

### Example 2

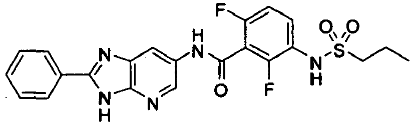

### 2,6-difluoro-N-(2-phenyl-3H-imidazo[4,5-b]pyridin-6-yl)-3-(propylsulfonamido)benzamide

Step A: 5-Nitropyridine-2,3-diamine (100 mg, 0.65 mmol, see Example 1) and benzoic acid (87 mg, 0.71 mmol, 1.1 eq.) were combined in POCl₃ (5 mL) and heated to reflux for 16 hours. After cooling to room temperature, the mixture was concentrated under reduced pressure, and the resulting residue was taken up in saturated sodium bicarbonate solution, and extracted with 25% IPA/DCM (2 X). The extracts were dried over sodium sulfate and concentrated to 6-nitro-2-phenyl-3H-imidazo[4,5-b]pyridine (67 mg, 43%) as a solid. ¹H NMR (400 MHz, DMSO-d₆) δ 9.24-9.27 (m, 1H), 8.81 (br s, 1H), 8.27-8.32 (m, 2H), 7.61-7.67 (m, 3H); *m*/*z* (APCI-neg) M-1 = 239.3.

Step B: 2-Phenyl-3H-imidazo[4,5-b]pyridin-6-amine (71%) was prepared according to the general procedure in Example 1, step D, substituting 6-nitro-2-phenyl-3H-imidazo[4,5-b]pyridine for 6-nitro-3H-imidazo[4,5-b]pyridine, and was carried forward to the next step without further purification.

Step C: 2,6-Difluoro-N-(2-phenyl-3H-imidazo[4,5-b]pyridin-6-yl)-3-(propylsulfonamido)benzamide (10%) was prepared according to the general procedure in Example 1, Step E, substituting 2-phenyl-3H-imidazo[4,5-b]pyridin-6-amine for 3H-imidazo[4,5-b]pyridin-6-amine. ¹H NMR (400 MHz, DMSO-d₆) δ 13.61 and 13.17 (broad singlets, 1H), 11.17 and 11.07 (broad singlets, 1H), 9.84 (br s, 1H), 8.42-8.57 (m, 2H), 8.17-8.26 (m, 2H), 7.53-7.64 (m, 4H), 7.25-7.33 (m, 1H), 3.10-3.17 (m, 2H), 1.71-1.83 (m, 2H), 0.98-1.03 (m, 3H); *m*/*z* (APCI-neg) M-1 = 470.2, (APCI-pos) M+1 = 472.1.

Step D: Alternative formation of 2,6-difluoro-N-(2-phenyl-3H-imidazo[4,5-b]pyridin-6-yl)-3-(propylsulfonamido)benzamide: A reaction vial containing 2-phenyl-3H-imidazo[4,5-b]pyridin-6-amine (5.3 mg, 0.026 mmol) was taken up in dry toluene (0.25 mL). Trimethyl aluminum (36 mL, 0.075 mmol, 2M in toluene) was then added by syringe, and the mixture was stirred at room temperature for 20 minutes. 2,6-Difluoro-3-(N-(propylsulfonyl)propylsulfonamido)-benzoate was added to this mixture, and the mixture was warmed to 90°C for 4 hours. The mixture was then allowed to stir at room temperature overnight. The mixture was then quenched with 30% aqueous sodium potassium tartrate, extracted with EtOAc (2 X), dried over sodium sulfate and concentrated to crude 2,6-difluoro-N-(2-phenyl-3H-imidazo[4,5-b]pyridin-6-yl)-3-(N-(propylsulfonyl)propylsulfonamido)benzamide. *m*/*z* (APCI-neg) M-1 = 470.1 (M-SO₂Pr), (APCI-pos) M+1 = 577.9. The crude product was then dissolved in methanol (0.5 mL), and aqueous 2M potassium carbonate (0.5 mL) was added. The mixture was warmed to 60°C for 4 hours and then stirred at room temperature overnight. The mixture was then diluted with EtOAc (5 mL), washed with 10% aqueous citric acid solution, dried over sodium sulfate and concentrated to give 2,6-difluoro-N-(2-phenyl-3H-imidazo[4,5-b]pyridin-6-yl)-3-(propylsulfonamido)benzamide. (APCI-pos) M+1 = 472.1.

Step E: Alternative formation of 2,6-difluoro-N-(2-phenyl-3H-imidazo[4,5-b]pyridin-6-yl)-3-(propylsulfonamido)benzamide: 2,6-Difluoro-N-(2-phenyl-3H-imidazo[4,5-b]pyridin-6-yl)-3-(N-(propylsulfonyl)propylsulfonamido)benzamide was prepared according to the general procedure in Example 1, Step E, substituting 2-phenyl-3H-imidazo[4,5-b]pyridin-6-amine for 3H-imidazo[4,5-b]pyridin-6-amine and 2,6-difluoro-3-(N-(propylsulfonyl)propylsulfonamido)benzoic acid for 2,6-difluoro-3-(propylsulfonamido)benzoic acid. (APCI-neg) M-1 = 470.1 (M-SO₂Pr), (APCI-pos) M+1 = 577.9. This material was then taken up in methanol (0.5 mL) and 2M aqueous potassium carbonate (0.5 mL), and the mixture was warmed to 60°C for 3 hours. The mixture was then diluted with EtOAc, washed with 10% aqueous citric acid, dried and concentrated to give 2,6-difluoro-N-(2-phenyl-3H-imidazo[4,5-b]pyridin-6-yl)-3-(propylsulfonamido)benzamide. (APCI-pos) M+1 = 472.1.

### Example 3

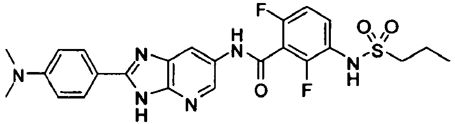

### N-(2-(4-(dimethylamino)phenyl)-3H-imidazo[45-b]pyridin-6-yl)-2,6-difluoro-3-(propylsulfonamido)benzamide

Step A: N,N-Dimethyl-4-(6-nitro-3H-imidazo[4,5-b]pyridin-2-yl)aniline (97%) was prepared according to the general procedure in Example 2, Step A, substituting 4-(dimethylamino)benzoic acid for benzoic acid. *m*/*z* (LC-MS) M+1 = 284.3.

Step B: N,N-Dimethyl-4-(6-nitro-3H-imidazo[4,5-b]pyridin-2-yl)aniline (155 mg, 0.55 mmol) in ethanol (10 mL) and water (3 mL), Fe (122 mg, 2.19 mmol) and NH₄Cl (293 mg, 5.47 mmol) were stirred at 80°C for 4 hours. Then the mixture was cooled down to room temperature and diluted with 20% MeOH in CH₂Cl₂. The mixture was then filtrated through a celite pad and concentrated. The mixture was used in the next step without further purification. *m*/*z* (LC-MS) M+1 = 254.3.

Step C: N-(2-(4-(Dimethylamino)phenyl)-3H-imidazo[4,5-b]pyridin-6-yl)-2,6-difluoro-3-(propylsulfonamido)benzamide (26%) was prepared according to the general procedure in Example 1, Step E, substituting 2-(4-(dimethylamino)phenyl)-3H-imidazo[4,5-b]pyridin-6-amine for 3H-imidazo[4,5-b]pyridin-6-amine. *m*/*z* (LC-MS) M+1 = 515.5.

### Example 4

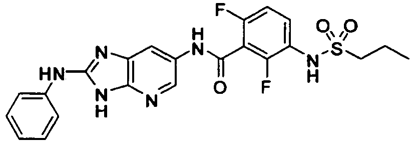

### 2,6-difluoro-N-(2-(phenylamino)-3H-imidazo[4,5-b]pyridin-6-yl)-3-(propylsulfonamido)benzamide

Step A: A scintillation vial was charged with 5-nitropyridine-2,3-diamine (44 mg, 0.285 mmol), phenyl isothiocyanate (250 µL, 2.09 mmol) and THF (5 mL). The reaction vessel was sealed and heated to 70°C for 30 minutes. The vessel was then cooled to room temperature, and PS-carbodiimide (1.25 mmol/g; 1.20 g, 1.50 mmol) was added. The vessel was re-sealed and heated to 70°C for 24 hours. The reaction mixture was filtered through GF/F paper, and the solids were rinsed with excess CH₂Cl₂ and MeOH alternately. The filtrate was concentrated in vacuo, and the residue was triturated with 5% MeOH/CH₂Cl₂ to afford 6-nitro-N-phenyl-3H-imidazo[4,5-b]pyridin-2-amine.

Step B: N2-Phenyl-3H-imidazo[4,5-b]pyridine-2,6-diamine was prepared according to Example 1, Step D, substituting 6-nitro-N-phenyl-3H-imidazo[4,5-b]pyridin-2-amine for 6-ilitro-3H-imidazo[4,5-b]pyridine.

Step C: 2,6-Difluoro-N-(2-(phenylamino)-3H-imidazo[4,5-b]pyridin-6-yl)-3-(propylsulfonamido)benzamide was prepared according to Example 1, Step E, substituting N2-phenyl-3H-imidazo[4,5-b]pyridine-2,6-diamine for 3H-imidazo[4,5-b]pyridin-6-amine. ¹H NMR (400 MHz, CD₃OD) δ 8.19 (br s, 1H), 7.70-7.56 (m, 4H), 7.40-7.33 (m, 3H), 7.16-7.10 (m, 1H), 7.10-7.05 (m, 1H), 3.14-3.08 (m, 2H), 1.92-1.82 (m, 2H), 1.06 (t, J = 7.4 Hz, 3H). *m*/*z* (APCI-pos) M+1 = 487.1.

### Example 5

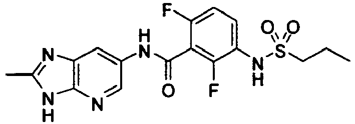

### 2,6-difluoro-N-(2-methyl-3H-imidazo[4,5-b]pyridin-6-yl)-3-(propylsulfonamido)benzamide

Step A: A 10-20 mL Biotage Microwave reaction vial was charged with 5-nitropyridine-2,3-diamine (85 mg, 0.55 mmol, Example 1, Step B) and acetic acid (5 mL). This mixture was subjected to microwave irradiation at 175°C for 75 minutes and then poured into a saturated sodium bicarbonate solution (200 mL). The solution was extracted with 25% IPA/DCM (2 X), and the extracts were dried over sodium sulfate. The extracts were concentrated to a solid, 2-methyl-6-nitro-3H-imidazo[4,5-b]pyridine (85 mg, 87%). ¹H NMR (400 MHz, DMSO-d₆) δ 13.43 (br s, 1H), 9.15-9.17 (m, 1H), 8.67-8.69 (m, 1H), 2.61 (s, 3H); *m*/*z* (APCI-neg) M-1 = 177.1.

Step B: 2-Methyl-3H-imidazo[4,5-b]pyridin-6-amine (87%) was prepared according to the general procedure Example 1, Step D, substituting 2-methyl-6-nitro-3H-imidazo[4,5-b]pyridine for 6-nitro-3H-imidazo[4,5-b]pyridine.

Step C: 2,6-Difluoro-N-(2-methyl-3H-imidazo[4,5-b]pyridin-6-yl)-3-(propylsulfonamido)benzamide (19%) was prepared according to the general procedure in Example 1, Step E, substituting 2-methyl-3H-imidazo[4,5-b]pyridin-6-amine for 3H-imidazo[4,5-b]pyridin-6-amine. ¹H NMR (400 MHz, DMSO-d₆) δ 12.82, 12.42 (broad singlets, 1H), 10.93-11.10 (m, 1H), 9.80 (br s, 1H), 8.21-8.46 (m, 2H), 7.50-7.59 (m, 1H), 7.23-7.29 (m, 1H), 3.08-3.19 (m, 3H), 1.72-1.83 (m, 2h), 0.96 - 1.03 (m, 3H); *m*/*z* (APCI-neg) M-1 = 408.1, (APCI-pos) M+1 = 410.1.

### Example 6

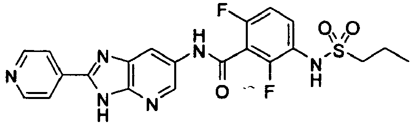

### 2,6-difluoro-3-(propylsulfonamido)-N-(2-(pyridin-4-yl)-3H-imidazo[4.5-b]pyridin-6-yl)benzamide

Step A: A 2-5 mL Biotage Microwave reaction vial was charged with 5-nitropyridine-2,3-diamine (100 mg, 0.65 mmol), isonicotinic acid (80 mg, 0.65 mmol, 1 eq.), and triphenyl phosphite (204 µL, 0.78 mmol, 1.2 eq.) in pyridine (3 mL). This mixture was heated in the microwave at 200°C for 15 minutes and then concentrated under reduced pressure. Preparative TLC of the resulting material (4 X 1.0 mm plates, 100% EtOAc as the eluant) afforded 6-nitro-2-(pyridin-4-yl)-3H-imidazo[4,5-b]pyridine (40 mg, 25%) as a solid. ¹H NMR (400 MHz, DMSO-d₆) δ 9.30-9.31 (m, 1H), 8.91-8.94 (br m, 1H), 8.83-8.88 (br m, 2H), 8.18-8.21 (m, 3H); *m*/*z* (APCI-neg) M-1 = 240.4, (APCI-pos) M+1 = 242.2.

Step B: 2-(Pyridin-4-yl)-3H-imidazo[4,5-b]pyridin-6-amine (50%) was prepared according to the general procedure in Example 1, Step D (using 1 weight equivalent of 20% Pd(OH)₂ as the catalyst), substituting 6-nitro-2-(pyridin-4-yl)-3H-imidazo[4,5-b]pyridine for 6-Nitro-3H-imidazo[4,5-b]pyridine.

Step C: 2,6-Difluoro-3-(propylsulfonamido)-N-(2-(pyridin-4-yl)-3H-imidazo[4,5-b]pyridin-6-yl)benzamide (35%) was prepared according to the general procedure in Example 1, Step E, substituting 2-(pyridin-4-yl)-3H-imidazo[4,5-b]pyridin-6-amine for 3H-imidazo[4,5-b]pyridin-6-amine. ¹H NMR (400 MHz, DMSO-d₆) δ 11.19 (br s, 1H), 9.83 (br s), 1H), 8.76-8.83 (m, 2H), 8.52-8.61 (br m, 2H), 8.09-8.16 br m, 2H), 7.53-7.61 (m, 1H), 7.24-7.31 (m, 1H), 3.08-3.18 (m, 2H), 1.71-1.82 (m, 2H), 0.98-1.03 (m, 3H); *m*/*z* (APCI-neg) M-1 = 471.2, (APCI-pos) M+1 = 473.1.

### Example 7

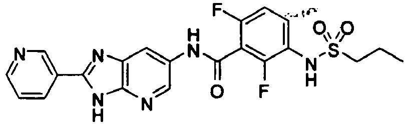

### 2,6-difluoro-3-(propylsulfonamido)-N-(2-(pyridin-3-yl)-3H-imidazo[4,5-b]pyridin-6-y])benzamide

Step A: 6-Nitro-2-(pyridin-3-yl)-3H-imidazo[4,5-b]pyridine (20%) was prepared according to Example 6, Step A, substituting nicotinic acid for isonicotinic acid. ¹H NMR (400 MHz, DMSO-d₆) δ 9.43-9.45 (m, 1H), 9.27-9.29 (m, 1H), 8.90 (br s, 1H), 8.78-8.82 (m, 1H), 8.59-8.63 (m, 1H), 7.65-7.71 (m, 1H); *m*/*z* (APCI-neg) M-1 = 240.4.

Step B: 2-(Pyridin-3-yl)-3H-imidazo[4,5-b]pyridin-6-amine (61%) was prepared according to Example 6, Step B, substituting 6-nitro-2-(pyridin-3-yl)-3H-imidazo[4,5-b]pyridine for 6-nitro-2-(pyridin-4-yl)-3H-imidazo[4,5-b]pyridine.

Step C: 2,6-Difluoro-3-(propylsulfonamido)-N-(2-(pyridin-3-yl)-3H-imidazo[4,5-b]pyridin-6-yl)benzamide (42%) was prepared according to the general procedure in Example 1, Step E, substituting 2-(pyridin-3-yl)-3H-imidazo[4,5-b]pyridin-6-amine for 3H-imidazo[4,5-b]pyridin-6-amine. ¹H NMR (400 MHz, DMSO-d₆) δ 13.81 and 13.37 (broad singlets, 1H), 11.07-11.22 (br m, 1H), 9.77-9.86 (br s, 1H), 9.34-9.41 (br s, 1H), 8.71-8.74 (m, 1H), 8.45-8.61 (br m, 3H), 7.54-7.66 (m, 2H), 7.26-7.31 (m, 1H), 3.09-3.19 (m, 2H), 1.73-1.83 (m, 2H), 0.97-1.04 (m, 3H); *m*/*z* (APCI-neg) M-1 = 471.2, (APCI-pos) M+1 = 473.1.

### Example 8

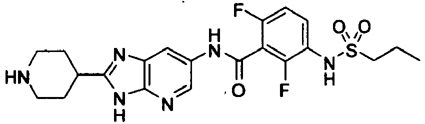

### 2,6-difluoro-N-(2-(piperidin-4-yl)-3 H-imidazo[4,5-b]pyridin-6-yl)-3-(propylsulfonamido)benzamide

Step A: Benzyl 4-(6-nitro-3H-imidazo[4,5-b]pyridin-2-yl)piperidine-1-carboxylate (34%) was prepared according to the general procedure in Example 6, Step 1, substituting 1-(benzyloxycarbonyl)piperidine-4-carboxylic acid for isonicotinic acid. ¹H NMR (400 MHz, DMSO-d₆) δ 13.53 (br s, 1H), 9.17-9.21 (m, 1H), 8.70-8.76 (br s, 1H), 7.30-7.42 (m, 5H), 5.11 (s, 2H), 4.05-4.14 (m, 2H), 2.96-3.28 (m, 3H), 2.02-2.12 (m, 2H), 1.69-1.82 (m, 2H); *mlz* (APCI-neg) M-1 = 380.4.

Step B: Benzyl 4-(6-nitro-3H-imidazo[4,5-b]pyridin-2-yl)piperidine-1-carboxylate (80 mg, 0.21 mmol) was dissolved in methanol (2 mL). Tin (II) chloride dihydrate (237 mg, 1.05 mmol, 5 eq.) was added, and the mixture was warmed to 70°C for 2 hours. The mixture was then allowed to cool to room temperature. The reaction mixture was diluted with EtOAc and washed once with saturated sodium bicarbonate solution (an emulsion formed, which was filtered through GF/F filter paper). The organics were isolated, dried and concentrated under reduced pressure. Preparative TLC (2 X 0.5 mm plates, 10% MeOH/DCM as the eluant) afforded benzyl 4-(6-amino-3H-imidazo[4,5-b]pyridin-2-yl)piperidine-1-carboxylate (42 mg, 57%) as a solid. ¹H NMR (400 MHz, DMSO-d₆) δ 7.70-7.73 (m, 1H), 7.29-7.41 (m, 5H), 6.97-7.01 (br s, 1H), 5.11 (s, 2H), 4.02-4.11 (m, 2H), 3.15-3.18 (m, 1H), 2.93-3.07 (m, 2H), 1.94-2.02 (m, 2H), 1.63-1.75 (m, 2H); *mlz* (APCI-neg) M-1 = 350.4, (APCI-pos) M+1 = 352.1.

Step C: Benzyl 4-(6-(2,6-difluoro-3-(propylsulfonamido)benzamido)-3H-imidazo[4,5-b]pyridin-2-yl)piperidine-1-carboxylate (46%) was prepared according to the general procedure in Example 1, Step E, substituting benzyl 4-(6-amino-3H-imidazo[4,5-b]pyridin-2-yl)piperidine-1-carboxylate for 3H-imidazo[4,5-b]pyridin-6-amine. ¹H NMR (400 MHz, DMSO-d₆) δ 12.87 and 12.46 (br singlets, 1H), 11.07 and 10.97 (br singlets), 9.78-9.83 (br s, 1H), 8.39-8.42 (m, 1H), 8.28 and 8.46 (br singlets, 1H), 7.28-7.59 (m , 7H), 5.11 (s, 2H), 4.05-4.14 (m, 2H), 2.96-3.19 (m, 5H), 2.00-2.08 (m, 2H), 1.69-1.81 (m, 4H), 0.96-1.04 (m, 3H); *m*/*z* (APCI-neg) M-1 = 611.3, (APCI-pos) M+1 = 613.1.

Step D: Benzyl 4-(6-(2,6-difluoro-3-(propylsulfonamido)benzamido)-3H-imidazo[4,5-b]pyridin-2-yl)piperidine-1-carboxylate (29 mg, 0.047 mmol) was dissolved in methanol (approximately 1 mL), and 20% palladium hydroxide (30 mg, 1 eq.) was added. The mixture was hydrogenated under a balloon of hydrogen for 1.5 hours. The mixture was filtered through GF/F paper, and the filtrate was concentrated to 2,6-difluoro-N-(2-(piperidin-4-yl)-3H-imidazo[4,5-b]pyridin-6-yl)-3-(propylsulfonamido)benzamide (14 mg, 62%). ¹H NMR (400 MHz, DMSO-d₆) δ 12.57 (very broad s, 1H), 11.02 (br s, 1H), 8.33-8.49 (m, 3H), 7.49-7.56 (m, 1H), 7.16-7.23 (m, 1H), 4.05-4.15 (m, 2H), 2.87-3.06 (m, 4H), 2.09-2.15 (m, 2H), 1.69-1.94 (m, 4H), 0.95-1.02 (m, 3H); *m*/*z* (APCI-neg) M-1 = 477.3, (APCI-pos) M+1 = 479.2.

### Example 9

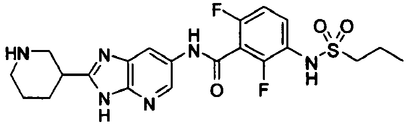

### 2,6-difluoro-N-(2-(piperidin-3-yl)-3H-imidazo[4.5-b]pyridin-6-yl)-3-(propylsulfonamido)benzamide

Step A: Benzyl 3-(6-nitro-3H-imidazo[4,5-b]pyridin-2-yl)piperidine-1-carboxylate (27%) was prepared according to the general procedure in Example 6, Step 1, substituting 1-(benzyloxycarbonyl)piperidine-3-carboxylic acid for isonicotinic acid. ¹H NMR (400 MHz, DMSO-d₆) δ 13.61 (br s, 1H), 9.18-9.21 (m, 1H), 8.71-8.77 (br s, 1H), 7.27-7.41 (m, 5H), 5.10 (s, 2H), 4.25-4.38 (m, 1H), 3.91-4.02 (m, 1H), 3.06-3.18 (m, 1H), 2.93-3.07 (m, 1H), 2.16 -2.25 (m, 1H), 1.77-1.91 (m, 2H), 1.47-1.60 (m, 2H); *m*/*z* (APCI-neg) M-1 = 380.4, (APCI-pos) M+1 = 382.0.

Step B: Benzyl 3-(6-amino-3H-imidazo[4,5-b]pyridin-2-yl)piperidine-1-carboxylate (75%) was prepared according to the general procedure in Example 8, Step B, substituting benzyl 3-(6-nitro-3H-imidazo[4,5-b]pyridin-2-yl)piperidine-1-carboxylate for benzyl 4-(6-nitro-3H-imidazo[4,5-b]pyridin-2-yl)piperidine-1-carboxylate. *m*/*z* (APCI-neg) M-1 = 350.3, (APCI-pos) M+1 = 352.2.

Step C: Benzyl 3-(6-(2,6-difluoro-3-(propylsulfonamido)benzamido)-3H-imidazo[4,5-b]pyridin-2-yl)piperidine-1-carboxylate (25%) was prepared according to the general procedure in Example 1, Step E, substituting 3-(6-amino-3H-imidazo[4,5-b]pyridin-2-yl)piperidine-1-carboxylate for 3H-imidazo[4,5-b]pyridin-6-amine. *m*/*z* (APCI-neg) M-1 = 611.2, (APCI-pos) M+1 =613.1.

Step D: 2,6-Difluoro-N-(2-(piperidin-3-yl)-3H-imidazo[4,5-b]pyridin-6-yl)-3-(propylsulfonamido)benzamide (53%) was prepared according to the general procedure in Example 8, Step D, substituting benzyl 3-(6-(2,6-difluoro-3-(propylsulfonamido)benzamido)-3H-imidazo[4,5-b]pyridin-2-yl)piperidine-1-carboxylate for benzyl 4-(6-(2,6-difluoro-3-(propylsulfonamido)benzamido)-3H-imidazo[4,5-b]pyridin-2-yl)piperidine-1-carboxylate.
¹H NMR (400 MHz, DMSO-d₆) δ 10.97 (s, 1H), 8.42-8.44 (m, 1H), 8.35-8.37 (m, 1H), 7.47-7.56)m, m, 1H), 7.15-7.21 (m, 1H), 3.51 (s, 2H), 2.88-3.06 (m, 4H), 2.61-2.69 (m, 1H), 2.09-2.16 (m, 1H), 1.68-1.82 (m, 4H), 0.95-1.01 (m, 3H); *m*/*z* (APCI-neg) M-1 = 477.3, (APCI-pos) M+1 = 479.2.

### Example 10

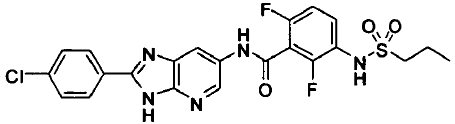

### N-(2-(4-chlorophenyl)-3H-imidazo[4.5-b]pyridin-6-yl)-2,6-difluoro-3-(propylsulfonamido)benzamide

Step A: 2-(4-Chlorophenyl)-6-nitro-3H-imidazo[4,5-b]pyridine (49%) was prepared as in the general procedure in Example 2, Step A, substituting 4-chlorobenzoic acid for benzoic acid. ¹H NMR (400 MHz, DMSO-d₆) δ 9.25-9.26 (m, 1H), 8.81-8.32 (m, 1H), 8.27-8.32 (m, 2H), 7.70-7.74 (m, 2H); *m*/*z* (APCI-neg) M-1 = 273.3.

Step B: 2-(4-Chlorophenyl)-6-nitro-3H-imidazo[4,5-b]pyridine (50 mg, 0.182 mmol) was dissolved in THF (2 mL), and a saturated ammonium chloride solution (2 mL) was added followed by zinc powder (119 mg, 1.82 mmol, 10 eq.). This mixture was vigorously stirred at room temperature for 15 minutes, when TLC indicated complete consumption of starting material. The reaction mixture was then filtered through GF/F filter paper. The filtrate was diluted with water and extracted with EtOAc (with a little methanol, 2 X). The extracts were dried over sodium sulfate and concentrated to 2-(4-chlorophenyl)-3H-imidazo[4,5-b]pyridin-6-amine (41 mg, 92%) as a solid.

Step C: N-(2-(4-Chlorophenyl)-3H-imidazo[4,5-b]pyridin-6-yl)-2,6-difluoro-3-(propylsulfonamido)benzamide (20%) was prepared according to the general procedure in Example 1, Step E, substituting 2-(4-chlorophenyl)-3H-imidazo[4,5-b]pyridin-6-amine for 3H-imidazo[4,5-b]pyridin-6-amine. ¹H NMR (400 MHz, DMSO-d₆) δ 13.67 and 13.25 (broad singlets, 1H), 11.18 and 11.07 (broad singlets, 1H), 9.81 (br s, 1H), 8.42-8.56 (m, 2H), 8.16 - 8.28 (m, 2H), 7.63 - 7.70 (br m, 2H), 7.52-7.60 (m, 1H), 7.26-7.32 (m, 1H), 3.11-3.17 (m, 2H), 1.72-1.82 (m, 2H), 0.98-1.04 (m, 3H); *m*/*z* (APCI-neg) M-1 = 506.2, 504.2, (APCI-pos) M+1 = 506.1, 508.1.

### Example 11

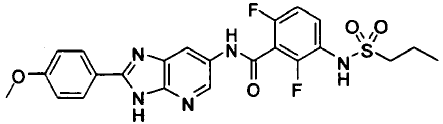

### 2,6-difluoro-N-(2-(4-methoxyphenyl)-3H-imidazo[4,5-b]pyridin-6-yl)-3-(propylsulfonamido)benzamide

Step A: 2-(4-Methoxyphenyl)-6-nitro-3H-imidazo[4,5-b]pyridine (64%) was prepared as in the general procedure in Example 2, Step A, substituting 4-methoxybenzoic acid for benzoic acid. ¹H NMR (400 MHz, DMSO-d₆) δ 9.19-9.22 (m, 1H), 8.72 (br s, 1H), 8.21-8.26 (m, 2H), 7.16-7.20 (m, 2H), 3.87 (s, 3H); *m*/*z* (APCI-neg) M-1 = 269.3.

Step B: 2-(4-Methoxyphenyl)-3H-imidazo[4,5-b]pyridin-6-amine (96%) was prepared as in the general procedure in Example 10, Step B, substituting 2-(4-methoxyphenyl)-6-nitro-3H-imidazo[4,5-b]pyridine for 2-(4-chlorophenyl)-6-nitro-3H-imidazo[4,5-b]pyridine.

Step C: 2,6-Difluoro-N-(2-(4-methoxyphenyl)-3H-imidazo[4,5-b]pyridin-6-yl)-3-(propylsulfonamido)benzamide (22%) was prepared according to the general procedure in Example 1, Step E, substituting 2-(4-methoxyphenyl)-3H-imidazo[4,5-b]pyridin-6-amine for 3H-imidazo[4,5-b]pyridin-6-amine. ¹H NMR (400 MHz, MeOH-d₄) δ 8.57 (br s, 1H), 8.44 (m, 1H), 8.08-8.13 (m, 2H), 7.62-7.69 (m, 1H), 7.10-7.17 (m, 3H), 3.90 (s, 3H), 3.08-3.14 (m, 2H), 1.82-1.93 (m, 2H), 1.03-1.09 (m, 3H); *m*/*z* (APCI-neg) M-1 = 500.3, (APCI-pos) M+1 = 502.1.

### Example 12

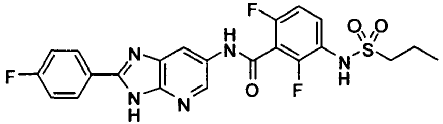

### 2,6-difluoro-N-(2-(4-fluorophenyl)-3H-imidazo[4,5-b]pyridin-6-yl)-3-(propylsulfonamido)benzamide

Step A: 2-(4-Fluorophenyl)-6-nitro-3H-imidazo[4,5-b]pyridine (30%) was prepared according to the general procedure in Example 2, Step A, substituting 4-fluorobenzoic acid for benzoic acid. ¹H NMR (400 MHz, MeOH-d₄) δ 8.17-8.22 (m, 2H), 7.96-7.99 (m, 1H), 7.63-7.72 (m, 2H), 7.24-7.38 (m, 3H), 7.12-7.18 (m, 1H), 3.10-3.14 (m, 2H), 1.79-1.93 (m, 2H), 1.01-1.09 (m, 3H); *m*/*z* (APCI-neg) M-1 = 480.2, (APCI-pos) M+1 = 490.1.

Step B: 2-(4-Fluorophenyl)-3H-imidazo[4,5-b]pyridin-6-amine (59%) was prepared according to Example 10, Step B, substituting 2-(4-fluorophenyl)-6-nitro-3H-imidazo[4,5-b]pyridine for 2-(4-chlorophenyl)-6-nitro-3H-imidazo[4,5-b]pyridine.

Step C: 2,6-Difluoro-N-(2-(4-fluorophenyl)-3H-imidazo[4,5-b]pyridin-6-yl)-3-(propylsulfonamido)benzamide (30%) was prepared according to the general procedure in Example 1, Step E, substituting 2-(4-fluorophenyl)-3H-imidazo[4,5-b]pyridin-6-amine for 3H-imidazo[4,5-b]pyridin-6-amine. ¹H NMR (400 MHz, MeOH-d₄) δ 8.17-8.22 (m, 2H), 7.96-7.99 (m, 1H), 7.63-7.72 (m, 2H), 7.24-7.38 (m, 3H), 7.12-7.18 (m, 1H), 3.10-3.14 (m, 2H), 1.79-1.93 (m, 2H), 1.01-1.09 (m, 3H); *m*/*z* (APCI-neg) M-1 = 480.2, (APCI-pos) M+1 = 490.1.

### Example 13

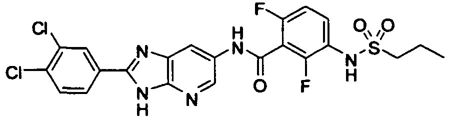

### N-(2-(3,4-dichlorophenyl)-3H-imidazo[4,5-b]pyridin-6-yl)-2,6-difluoro-3-(propylsulfonamido)benzamide

Step A: 2-(3,4-Dichlorophenyl)-6-nitro-3H-imidazo[4,5-b]pyridine was prepared according to the general procedure in Example 2, Step A, substituting 3,4-dichlorobenzoic acid for benzoic acid. *m*/*z* (LC-MS) M+1 = 310.1.

Step B: 2-(3,4-Dichlorophenyl)-3H-imidazo[4,5-b]pyridin-6-amine was prepared according to the general procedure in Example 3, Step B, substituting 2-(3,4-dichlorophenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 = 280.1.

Step C: N-(2-(3,4-Dichlorophenyl)-3H-imidazo[4,5-b]pyridin-6-yl)-2,6-difluoro-3-(propylsulfonamido)benzamide (19%) was prepared according to the general procedure in Example 1, Step E, substituting 2-(3,4-dichlorophenyl)-3H-imidazo[4,5-b]pyridin-6-amine for 3H-imidazo[4,5-b]pyridin-6-amine. *m*/*z* (LC-MS) M+1 = 541.3.

### Example 14

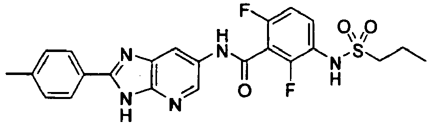

### 2,6-difluoro-3-(propylsulfonamido)-N-(2-p-tolyl-3H-imidazo[4,5-b]pyridin-6-yl)benzamide

Step A: 6-Nitro-2-p-tolyl-3H-imidazo[4,5-b]pyridine (82%) was prepared according to the general procedure in Example 2, Step A, substituting p-toluic acid for benzoic acid. *m*/*z* (LC-MS) M+1 = 255.2.

Step B: 2-p-Tolyl-3H-imidazo[4,5-b]pyridin-6-amine was prepared according to the general procedure in Example 3, Step B, substituting 6-nitro-2-p-tolyl-3H-imidazo[4,5-b]pyridine for N,N-dimethyl-4-(6-nitro-3H-imidazo[4,5-b]pyridin-2-yl)aniline.

Step C: 2,6-Difluoro-3-(propylsulfonamido)-N-(2-p-tolyl-3H-imidazo[4,5-b]pyridin-6-yl)benzamide (30%) was prepared according to the general procedure in Example 1, Step E, substituting 2-p-tolyl-3H-imidazo[4,5-b]pyridin-6-amine for 3H-imidazo[4,5-b]pyridin-6-amine. *m*/*z* (LC-MS) M+1 = 486.5.

### Example 15

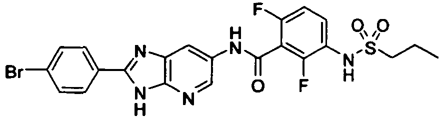

### N-(2-(4-bromophenyl)-3H-imidazo[4,5-b]pyridin-6-yl)-2,6-difluoro-3-(propylsulfonamido)benzamide

Step A: 2-(4-Bromophenyl)-6-nitro-3H-imidazo[4,5-b]pyridine (59%) was prepared according to the general procedure in Example 2, Step A, substituting 4-bromobenzoic acid for benzoic acid. *m*/*z* (LC-MS) M+1 = 321.

Step B: 2-(4-Bromophenyl)-3H-imidazo[4,5-b]pyridin-6-amine was prepared according to the general procedure in Example 3, Step B, substituting 2-(4-bromophenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 = 291.

Step C: N-(2-(4-Bromophenyl)-3H-imidazo[4,5-b]pyridin-6-yl)-2,6-difluoro-3-(propylsulfonamido)benzamide (54%) was prepared according to the general procedure in Example 1, Step E, substituting 2-(4-bromophenyl)-3H-imidazo[4,5-b]pyridin-6-amine for 3H-imidazo[4,5-b]pyridin-6-amine. *m*/*z* (LC-MS) M+1 = 552.

### Example 16

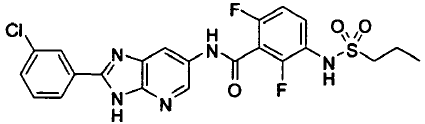

### N-(2-(3-chlorophenyl)-3H-imidazo[4,5-b]pyridin-6-yl)-2,6-difluoro-3-(propylsulfonamido)benzamide

Step A: 2-(3-Chlorophenyl)-6-nitro-3H-imidazo[4,5-b]pyridine (83%) was prepared according to the general procedure in Example 2, Step A, substituting 3-chlorobenzoic acid for benzoic acid. *m*/*z* (LC-MS) M+1 = 275.6.

Step B: 2-(3-Chlorophenyl)-3H-imidazo[4,5-b]pyridin-6-amine was prepared according to the general procedure in Example 3, Step B, substituting 2-(3-chlorophenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 = 245.6.

Step C: N-(2-(3-Chlorophenyl)-3H-imidazo[4,5-b]pyridin-6-yl)-2,6-difluoro-3-(propylsulfonamido)benzamide (18%) was prepared according to the general procedure in Example 1, Step E, substituting 2-(3-chlorophenyl)-3H-imidazo[4,5-b]pyridin-6-amine for 3H-imidazo[4,5-b]pyridin-6-amine. *m*/*z* (LC-MS) M+1 = 506.9.

### Example 17

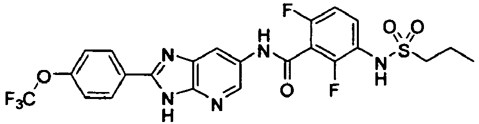

### 2,6-difluoro-3-(propylsulfonamido)-N-(2-(4-(trifluoromethoxy)phenyl)-3H-imidazo[4,5-b]pyridin-6-yl)benzamide

Step A: 6-Nitro-2-(3-(trifluoromethoxy)phenyl)-3H-imidazo[4,5-b]pyridine (75%) was prepared according to the general procedure in Example 2, Step A, substituting 4-trifluoromethoxybenzoic acid for benzoic acid. *m*/*z* (LC-MS) M+1 = 325.2.

Step B: 2-(3-(Trifluoromethoxy)phenyl)-3H-imidazo[4,5-b]pyridin-6-amine was prepared according to the general procedure in Example 3, Step B, substituting 6-nitro-2-(3-(trifluoromethoxy)phenyl)-3H-imidazo[4,5-b]pyridine for N,N-dimethyl-4-(6-nitro-3H-imidazo[4,5-b]pyridin-2-yl)aniline. *m*/*z* (LC-MS) M+1 = 295.2.

Step C: 2,6-Difluoro-3-(propylsulfonamido)-N-(2-(4-(trifluoromethoxy)phenyl)-3H-imidazo[4,5-b]pyridin-6-yl)benzamide (28%) was prepared according to the general procedure in Example 1, Step E, substituting 2-(3-(trifluoromethoxy)phenyl)-3H-imidazo[4,5-b]pyridin-6-amine for 3H-imidazo[4,5-b]pyridin-6-amine. *m*/*z* (LC-MS) M+1 = 556.5.

### Example 18

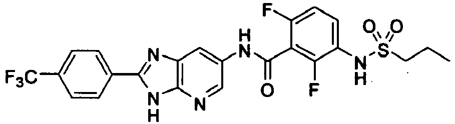

### 2,6-difluoro-3-(propylsulfonamido)-N-(2-(4-(trifluoromethyl)phenyl)-3H-imidazo[4,5-b]pyridin-6-yl)benzamide

Step A: 6-Nitro-2-(4-(trifluoromethyl)phenyl)-3H-imidazo[4,5-b]pyridine (64%) was prepared according to the general procedure in Example 2, Step A, substituting 4-trifluoromethylbenzoic acid for benzoic acid. *m*/*z* (LC-MS) M+1 = 309.2.

Step B: 2-(4-(Trifluoromethyl)phenyl)-3H-imidazo[4,5-b]pyridin-6-amine was prepared according to the general procedure in Example 3, Step B, substituting 6-nitro-2-(4-(trifluoromethyl)phenyl)-3H-imidazo[4,5-b]pyridine for N,N-dimethyl-4-(6-nitro-3H-imidazo[4,5-b]pyridin-2-yl)aniline. *m*/*z* (LC-MS) M+1 = 279.2.

Step C: 2,6-Difluoro-3-(propylsulfonamido)-N-(2-(4-(trifluoromethyl)phenyl)-3H-imidazo[4,5-b]pyridin-6-yl)benzamide (27%) was prepared according to the general procedure in Example 1, Step E, substituting 2-(4-(trifluoromethyl)phenyl)-3H-imidazo[4,5-b]pyridin-6-amine for 3H-imidazo[4,5-b]pyridin-6-amine. *m*/*z* (LC-MS) M+1 = 540.4.

### Example 19

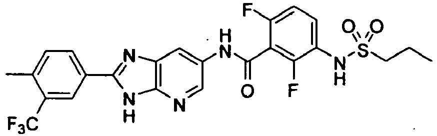

### 2,6-difluoro-N-(2-(4-methyl-3-(trifluoromethyl)phenyl)-3H-imidazo[4,5-b]pyridin-6-yl)-3-(propylsulfonamido)benzamide

Step A: 2-(4-Methyl-3-(trifluoromethyl)phenyl)-6-nitro-3H-imidazo[4,5-b]pyridine (87%) was prepared according to the general procedure in Example 2, Step A, substituting 4-methyl-3-(trifluoromethyl)benzoic acid for benzoic acid. *m*/*z* (LC-MS) M+1 = 323.

Step B: 2-(4-Methyl-3-(trifluoromethyl)phenyl)-3H-imidazo[4,5-b]pyridin-6-amine was prepared according to the general procedure in Example 3, Step B, substituting 2-(4-methyl-3-(trifluoromethyl)phenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 = 293.

Step C: 2,6-Difluoro-N-(2-(4-methyl-3-(trifluoromethyl)phenyl)-3H-imidazo[4,5-b]pyridin-6-yl)-3-(propylsulfonamido)benzamide (30%) was prepared according to the general procedure in Example 1, Step E, substituting 2-(4-methyl-3-(trifluoromethyl)phenyl)-3H-imidazo[4,5-b]pyridin-6-amine for 3H-imidazo[4,5-b]pyridin-6-amine. *m*/*z* (LC-MS) M+1 = 554.

### Example 20

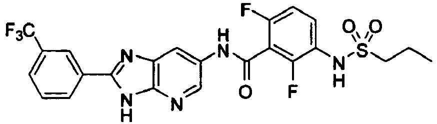

### 2,6-difluoro-3-(propylsulfonamido)-N-(2-(3-(trifluoromethyl)phenyl)-3H-imidazo[4,5-b]pyridin-6-yl)benzamide

Step A: 6-Nitro-2-(3-(trifluoromethyl)phenyl)-3H-imidazo[4,5-b]pyridine (78%) was prepared according to the general procedure in Example 2, Step A, substituting 3-(trifluoromethyl)benzoic acid for benzoic acid. *m*/*z* (LC-MS) M+1 = 309.2.

Step B: 2-(3-(Trifluoromethyl)phenyl)-3H-imidazo[4,5-b]pyridin-6-amine was prepared according to the general procedure in Example 3, Step B, substituting 6-nitro-2-(3-(trifluoromethyl)phenyl)-3H-imidazo[4,5-b]pyridine for N,N-dimethyl-4-(6-nitro-3H-imidazo[4,5-b]pyridin-2-yl)aniline. *m*/*z* (LC-MS) M+1 = 279.2.

Step C: 2,6-Difluoro-3-(propylsulfonamido)-N-(2-(3-(trifluoromethyl) phenyl)-3H-imidazo[4,5-b]pyridin-6-yl)benzamide (27%) was prepared according to the general procedure in Example 1, Step E, substituting 2-(3-(trifluoromethyl)phenyl)-3H-imidazo[4,5-b]pyridin-6-amine for 3H-imidazo[4,5-b]pyridin-6-amine. *m*/*z* (LC-MS) M+1 = 540.4.

### Example 21

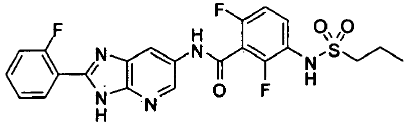

### 2,6-difluoro-N-(2-(2-fluorophenyl)-3H-imidazo[4,5-b]pyridin-6-yl)-3-(propylsulfonamido)benzamide

Step A: 2-(2-Fluorophenyl)-6-nitro-3H-imidazo[4,5-b]pyridine (88%) was prepared according to the general procedure in Example 2, Step A, substituting 2-fluorobenzoic acid for benzoic acid. *m*/*z* (LC-MS) M+1 = 259.2.

Step B: 2-(2-Fluorophenyl)-3H-imidazo[4,5-b]pyridin-6-amine was prepared according to the general procedure in Example 3, Step B, substituting 2-(2-fluorophenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 = 229.2.

Step C: 2,6-Difluoro-N-(2-(2-fluorophenyl)-3H-imidazo[4,5-b]pyridin-6-yl)-3-(propylsulfonamido)benzamide (43%) was prepared according to the general procedure in Example 1, Step E, substituting 2-(2-fluorophenyl)-3H-imidazo[4,5-b]pyridin-6-amine for 3H-imidazo[4,5-b]pyridin-6-amine. *m*/*z* (LC-MS) M+1 = 490.4.

### Example 22

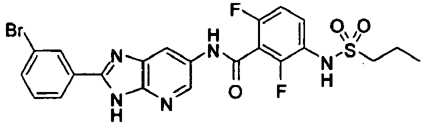

### N-(2-(3-bromophenyl)-3H-imidazo[4,5-b]pyridin-6-yl)-2,6-difluoro-3-(propylsulfonamido)benzamide

Step A: 2-(3-Bromophenyl)-6-nitro-3H-imidazo[4,5-b]pyridine (71%) was prepared according to the general procedure in Example 2, Step A, substituting 3-bromobenzoic acid for benzoic acid. *m*/*z* (LC-MS) M+1 = 320.1.

Step B: 2-(3-Bromophenyl)-3H-imidazo[4,5-b]pyridin-6-amine was prepared according to the general procedure in Example 3, Step B, substituting 2-(3-bromophenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 = 290.1.

Step C: N-(2-(3-Bromophenyl)-3H-imidazo[4,5-b]pyridin-6-yl)-2,6-difluoro-3-(propylsulfonamido)benzamide (17%) was prepared according to the general procedure in Example 1, Step E, substituting 2-(3-bromophenyl)-3H-imidazo[4,5-b]pyridin-6-amine for 3H-imidazo[4,5-b]pyridin-6-amine. *m*/*z* (LC-MS) M+1 = 551.4.

### Example 23

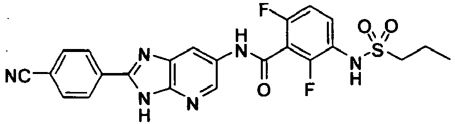

### N-(2-(4-cvanophenyl)-3H-imidazo[4,5-b]pyridin-6-yl)-2,6-difluoro-3-(propylsulfonamido)benzamide

Step A: 4-(6-Nitro-3H-imidazo[4,5-b]pyridin-2-yl)benzonitrile (71%) was prepared according to the general procedure in Example 2, Step A, substituting 4-cyanobenzoic acid for benzoic acid. *m*/*z* (LC-MS) M+1 = 266.2.

Step B: 4-(6-Amino-3H-imidazo[4,5-b]pyridin-2-yl)benzonitrile was prepared according to the general procedure in Example 3, Step B, substituting 4-(6-nitro-3H-imidazo[4,5-b]pyridin-2-yl)benzonitrile for N,N-dimethyl-4-(6-nitro-3H-imidazo[4,5-b]pyridin-2-yl)aniline. *m*/*z* (LC-MS) M+1 = 236.2.

Step C: N-(2-(4-Cyanophenyl)-3H-imidazo[4,5-b]pyridin-6-yl)-2,6-difluoro-3-(propylsulfonamido)benzamide (21%) was prepared according to the general procedure in Example 1, Step E, substituting 4-(6-amino-3H-imidazo[4,5-b]pyridin-2-yl)benzonitrile for 3H-imidazo[4,5-b]pyridin-6-amine. *m*/*z* (LC-MS) M+1 = 497.4.

### Example 24

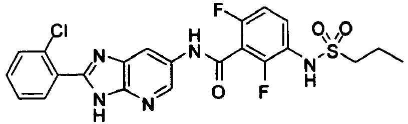

### N-(2-(2-chlorophenyl)-3H-imidazo[4,5-b]pyridin-6-yl)-2,6-difluoro-3-(propylsulfonamido)benzamide

Step A: 2-(2-Chlorophenyl)-6-nitro-3H-imidazo[4,5-b]pyridine (88%) was prepared according to the general procedure in Example 2, Step A, substituting 2-chlorobenzoic acid for benzoic acid. *m*/*z* (LC-MS) M+1 = 275.6.

Step B: 2-(2-Chlorophenyl)-3H-imidazo[4,5-b]pyridin-6-amine was prepared according to the general procedure in Example 3, Step B, substituting 2-(2-chlorophenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =245.6.

Step C: N-(2-(2-Chlorophenyl)-3H-imidazo[4,5-b]pyridin-6-yl)-2,6-difluoro-3-(propylsulfonamido)benzamide (32%) was prepared according to the general procedure in Example 1, Step E, substituting 2-(2-chlorophenyl)-3H-imidazo[4,5-b]pyridin-6-amine for 3H-imidazo[4,5-b]pyridin-6-amine. *m*/*z* (LC-MS) M+1 = 506.9.

### Example 25

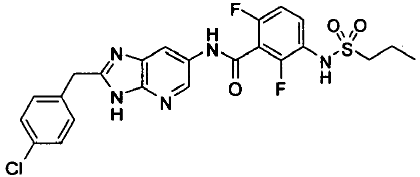

### N-(2-(4-chlorobenzyl)-3H-imidazo[4,5-b]pyridin-6-yl)-2,6-difluoro-3-(propylsulfonamido)benzamide

Step A: 2-(4-Chlorobenzyl)-6-nitro-3H-imidazo[4,5-b]pyridine (99%) was prepared according to the general procedure in Example 2, Step A, substituting 4-chlorophenyl acetic acid for benzoic acid. *m*/*z* (LC-MS) M+1 = 289.7.

Step B: 2-(4-Chlorobenzyl)-3H-imidazo[4,5-b]pyridin-6-amine was prepared according to the general procedure in Example 3, Step B, substituting 2-(4-chlorobenzyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =259.7.

Step C: N-(2-(4-Chlorobenzyl)-3H-imidazo[4,5-b]pyridin-6-yl)-2,6-difluoro-3-(propylsulfonamido)benzamide (52%) was prepared according to the general procedure in Example 1, Step E, substituting 2-(4-chlorobenzyl)-3H-imidazo[4,5-b]pyridin-6-amine for 3H-imidazo[4,5-b]pyridin-6-amine. *m*/*z* (LC-MS) M+1 = 520.9.

### Example 26

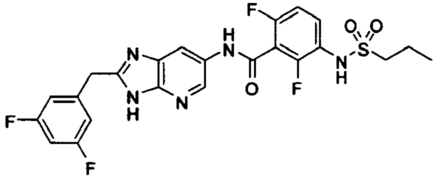

### N-(2-(3,5-difluorobenzyl)-3H-imidazo[4,5-b]pyridin-6-yl)-2,6-difluoro-3-(propylsulfonamido)benzamide

Step A: 2-(3,5-Difluorobenzyl)-6-nitro-3H-imidazo[4,5-b]pyridine (91%) was prepared according to the general procedure in Example 2, Step A, substituting 4-chlorophenyl acetic acid for benzoic acid. *m*/*z* (LC-MS) M+1 = 291.2.

Step B: 2-(3,5-Difluorobenzyl)-3H-imidazo[4,5-b]pyridin-6-amine was prepared according to the general procedure in Example 3, Step B, substituting 2-(3,5-difluorobenzyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =261.2.

Step C: N-(2-(3,5-Difluorobenzyl)-3H-imidazo[4,5-b]pyridin-6-yl)-2,6-difluoro-3-(propylsulfonamido)benzamide (23%) was prepared according to the general procedure in Example 1, Step E, substituting 2-(3,5-difluorobenzyl)-3H-imidazo[4,5-b]pyridin-6-amine for 3H-imidazo[4,5-b]pyridin-6-amine. *m*/*z* (LC-MS) M+1 = 522.4.

### Example 27

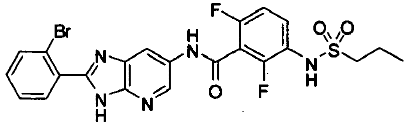

### N-(2-(2-bromophenyl)-3H-imidazo[4,5-b]pyridin-6-yl)-2,6-difluoro-3-(propylsulfonamido)benzamide

Step A: 2-(2-Bromophenyl)-6-nitro-3H-imidazo[4,5-b]pyridine (81%) was prepared according to the general procedure in Example 2, Step A, substituting 2-bromobenzoic acid for benzoic acid. *m*/*z* (LC-MS) M+1 = 320.1.

Step B: 2-(2-Bromophenyl)-3H-imidazo[4,5-b]pyridin-6-amine was prepared according to the general procedure in Example 3, Step B, substituting 2-(2-bromophenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =290.1.

Step C: N-(2-(2-Bromophenyl)-3H-imidazo[4,5-b]pyridin-6-yl)-2,6-difluoro-3-(propylsulfonamido)benzamide (30%) was prepared according to the general procedure in Example 1, Step E, substituting 2-(2-bromophenyl)-3H-imidazo[4,5-b]pyridin-6-amine for 3H-imidazo[4,5-b]pyridin-6-amine. *m*/*z* (LC-MS) M+1 = 551.4.

### Example 28

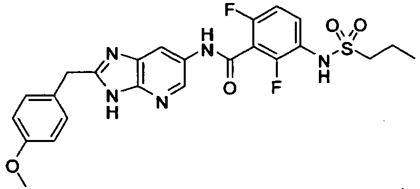

### 2,6-difluoro-N-(2-(4-methoxybenzyl)-3H-imidazo[4,5-b]pyridin-6-yl)-3-(propylsulfonamido)benzamide

Step A: 2-(4-Methoxybenzyl)-6-nitro-3H-imidazo[4,5-b]pyridine (90%) was prepared according to the general procedure in Example 2, Step A, substituting 2-bromobenzoic acid for benzoic acid. *m*/*z* (LC-MS) M+1 = 285.2.

Step B: 2-(4-Methoxybenzyl)-3H-imidazo[4,5-b]pyridin-6-amine was prepared according to the general procedure in Example 3, Step B, substituting 2-(4-methoxybenzyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 = 255.2.

Step C: 2,6-Difluoro-N-(2-(4-methoxybenzyl)-3H-imidazo[4,5-b]pyridin-6-yl)-3-(propylsulfonamido)benzamide (47%) was prepared according to the general procedure in Example 1, Step E, substituting 2-(4-methoxybenzyl)-3H-imidazo[4,5-b]pyridin-6-amine for 3H-imidazo[4,5-b]pyridin-6-amine. *m*/*z* (LC-MS) M+1 = 516.5.

### Example 29

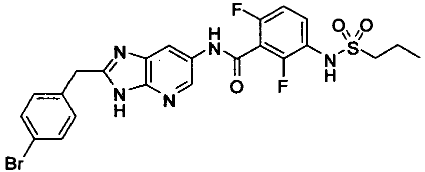

### N-(2-(4-bromobenzyl)-3H-imidazo[4,5-b]pyridin-6-yl)-2,6-difluoro-3-(propylsulfonamido)benzamide

Step A: 2-(4-Bromobenzyl)-6-nitro-3H-imidazo[4,5-b]pyridine (70%) was prepared according to the general procedure in Example 2, Step A, substituting 4-bromophenyl acetic acid for benzoic acid. *m*/*z* (LC-MS) M+1 = 334.1.

Step B: 2-(4-Bromobenzyl)-3H-imidazo[4,5-b]pyridin-6-amine was prepared according to the general procedure in Example 3, Step B, substituting 2-(4-bromobenzyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =304.2.

Step C: N-(2-(4-Bromobenzyl)-3H-imidazo[4,5-b]pyridin-6-yl)-2,6-difluoro-3-(propylsulfonamido)benzamide (70%) was prepared according to the general procedure in Example 1, Step E, substituting 2-(4-bromobenzyl)-3H-imidazo[4,5-b]pyridin-6-amine for 3H-imidazo[4,5-b]pyridin-6-amine. *m*/*z* (LC-MS) M+1 = 565.4.

### Example 30

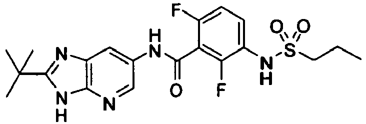

### N-(2-tert-butyl-3H-imidazo[4,5-b]pyridin-6-yl)-2,6-difluoro-3-(propylsulfonamido)benzamide

Step A: 2-tert-Butyl-6-nitro-3H-imidazo[4,5-b]pyridine (54%) was prepared according to the general procedure in Example 2, Step A, substituting pivalic acid for benzoic acid. *m*/*z* (LC-MS) M+1 = 221.2.

Step B: 2-tert-Butyl-3H-imidazo[4,5-b]pyridin-6-amine was prepared according to the general procedure in Example 3, Step B, substituting 2-tert-butyl-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =191.2.

Step C: N-(2-tert-Butyl-3H-imidazo[4,5-b]pyridin-6-yl)-2,6-difluoro-3-(propylsulfonamido)benzamide (33%) was prepared according to the general procedure in Example 1, Step E, substituting 2-tert-butyl-3H-imidazo[4,5-b]pyridin-6-amine for 3H-imidazo[4,5-b]pyridin-6-amine. *m*/*z* (LC-MS) M+1 = 452.4.

### Example 31

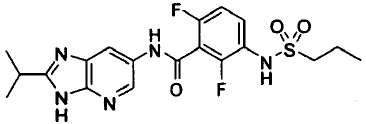

### 2,6-difluoro-N-(2-isopropyl-3H-imidazo[4,5-b]pyridin-6-yl)-3-(propylsulfonamido)benzamide

Step A: 2-Isopropyl-6-nitro-3H-imidazo[4,5-b]pyridine (87%) was prepared according to the general procedure in Example 2, Step A, substituting isobutyric acid for benzoic acid. *m*/*z* (LC-MS) M+1 = 207.2.

Step B: 2-Isopropyl-3H-imidazo[4,5-b]pyridin-6-amine was prepared according to the general procedure in Example 3, Step B, substituting 2-isopropyl-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =177.2.

Step C: 2,6-Difluoro-N-(2-isopropyl-3H-imidazo[4,5-b]pyridin-6-yl)-3-(propylsulfonamido)benzamide (71%) was prepared according to the general procedure in Example 1, Step E, substituting 2-isopropyl-3H-imidazo[4,5-b]pyridin-6-amine for 3H-imidazo[4,5-b]pyridin-6-amine. *m*/*z* (LC-MS) M+1 = 438.4.

### Example 32

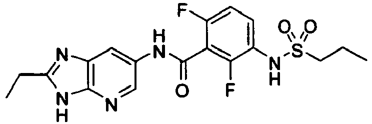

### N-(2-ethyl-3H-imidazo[4,5-b]pyridin-6-yl)-2 6-difluoro-3-(propylsulfonamido)benzamide

Step A: 2-Ethyl-6-nitro-3H-imidazo[4,5-b]pyridine (80%) was prepared according to the general procedure in Example 2, Step A, substituting propionic acid for benzoic acid. *m*/*z* (LC-MS) M+1 = 193.1.

Step B: 2-Ethyl-3H-imidazo[4,5-b]pyridin-6-amine was prepared according to the general procedure in Example 3, Step B, substituting 2-ethyl-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =163.1.

Step C: N-(2-Ethyl-3H-imidazo[4,5-b]pyridin-6-yl)-2,6-difluoro-3-(propylsulfonamido)benzamide (33%) was prepared according to the general procedure in Example 1, Step E, substituting 2-ethyl-3H-imidazo[4,5-b]pyridin-6-amine for 3H-imidazo[4,5-b]pyridin-6-amine. *m*/*z* (LC-MS) M+1 = 424.4.

### Example 33

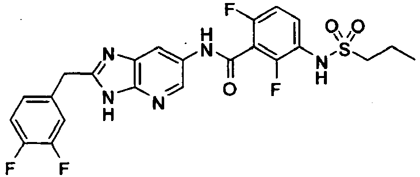

### N-(2-(3,4-difluorobenzyl)-3H-imidazo[4,5-b]pyridin-6-yl)-2,6-difluoro-3-(propylsulfonamido)benzamide

Step A: 2-(3,4-Difluorobenzyl)-6-nitro-3H-imidazo[4,5-b]pyridine (82%) was prepared according to the general procedure in Example 2, Step A, substituting 3,4-difluorophenyl acetic acid for benzoic acid. *m*/*z* (LC-MS) M+1 = 291.2.

Step B: 2-(3,4-Difluorobenzyl)-3H-imidazo[4,5-b]pyridin-6-amine was prepared according to the general procedure in Example 3, Step B, substituting 2-(3,4-difluorobenzyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =261.2.

Step C: N-(2-(3,4-Difluorobenzyl)-3H-imidazo[4,5-b]pyridin-6-yl)-2,6-difluoro-3-(propylsulfonamido)benzamide (40%) was prepared according to the general procedure in Example 1, Step E, substituting 2-(3,4-difluorobenzyl)-3H-imidazo[4,5-b]pyridin-6-amine for 3H-imidazo[4,5-b]pyridin-6-amine. *m*/*z* (LC=MS) M+1 = 522.4.

### Example 34

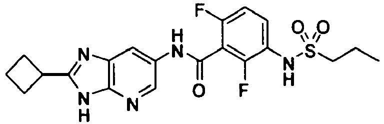

### N-(2-cyclobutyl-3H-imidazo[4,5-b]pyridin-6-yl)-2,6-difluoro-3-(propylsulfonamido)benzamide

Step A: 2-Cyclopropyl-6-nitro-3H-imidazo[4,5-b]pyridine (70%) was prepared according to the general procedure in Example 2, Step A, substituting cyclobutanecarboxylic acid for benzoic acid. *m*/*z* (LC-MS) M+1 = 219.2.

Step B: 2-Cyclopropyl-3H-imidazo[4,5-b]pyridin-6-amine was prepared according to the general procedure in Example 3, Step B, substituting 2-cyclopropyl-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =189.2.

Step C: N-(2-Cyclopropyl-3H-imidazo[4,5-b]pyridin-6-yl)-2,6-difluoro-3-(propylsulfonamido)benzamide (79%) was prepared according to the general procedure in Example 1, Step E, substituting 2-cyclopropyl-3H-imidazo[4,5-b]pyridin-6-amine for 3H-imidazo[4,5-b]pyridin-6-amine. *m*/*z* (LC-MS) M+1 = 450.4.

### Example 35

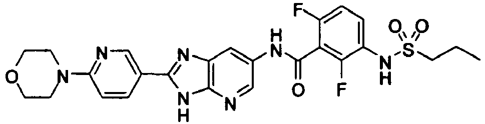

### 2,6-difluoro-N-(2-(6-morpholinopyridin-3-yl)-3H-imidazo[4,5-b]pyridin-6-yl)-3-(propylsulfonamido)benzamide

Step A: 4-(5-(6-Nitro-3H-imidazo[4,5-b]pyridin-2-yl)pyridin-2-yl)morpholine (99%) was prepared according to the general procedure in Example 2, Step A, substituting 6-morpholinonicotinic acid for benzoic acid. *m*/*z* (LC-MS) M+1 = 327.3.

Step B: 2-(6-Morpholinopyridin-3-yl)-3H-imidazo[4,5-b]pyridin-6-amine was prepared according to the general procedure in Example 3, Step B, substituting 4-(5-(6-nitro-3H-imidazo[4,5-b]pyridin-2-yl)pyridin-2-yl)morpholine for N,N-dimethyl-4-(6-nitro-3H-imidazo[4,5-b]pyridin-2-yl)aniline. *m*/*z* (LC-MS) M+1 =297.3.

Step C: 2,6-Difluoro-N-(2-(6-morpholinopyridin-3-yl)-3H-imidazo[4,5-b]pyridin-6-yl)-3-(propylsulfonamido)benzamide (8%) was prepared according to the general procedure in Example 1, Step E, substituting 2-(6-morpholinopyridin-3-yl)-3H-imidazo[4,5-b]pyridin-6-amine for 3H-imidazo[4,5-b]pyridin-6-amine. *m*/*z* (LC-MS) M+1 = 558.5.

### Example 36

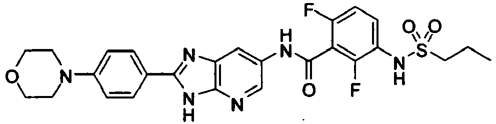

### 2,6-difluoro-N-(2-(4-morpholinophenyl)-3H-imidazo[4,5-b]pyridin-6-yl)-3-(propylsulfonamido)benzamide

Step A: 4-(4-(6-Nitro-3H-imidazo[4,5-b]pyridin-2-yl)phenyl)morpholine (99%) was prepared according to the general procedure in Example 2, Step A, substituting 4-morpholinobenzoic acid for benzoic acid. *m*/*z* (LC-MS) M+1 = 326.3.

Step B: 2-(4-Morpholinophenyl)-3H-imidazo[4,5-b]pyridin-6-amine was prepared according to the general procedure in Example 3, Step B, substituting 4-(4-(6-nitro-3H-imidazo[4,5-b]pyridin-2-yl)phenyl)morpholine for N,N-dimethyl-4-(6-nitro-3H-imidazo[4,5-b]pyridin-2-yl)aniline. *m*/*z* (LC-MS) M+1 =296.3.

Step C: 2,6-Difluoro-N-(2-(4-morpholinophenyl)-3H-imidazo[4,5-b]pyridin-6-yl)-3-(propylsulfonamido)benzamide (8%) was prepared according to the general procedure in Example 1, Step E, substituting 2-(4-morpholinophenyl)-3H-imidazo[4,5-b]pyridin-6-amine for 3H-imidazo[4,5-b]pyridin-6-amine. *m*/*z* (LC-MS) M+1 = 557.5.

### Example 37

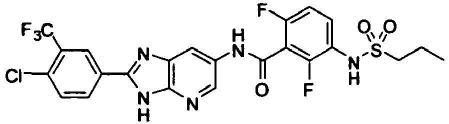

### N-(2-(4-chloro-3-(trifluoromethyl)phenyl)-3H-imidazo[4,5-b]pyridin-6-yl)-2,6-difluoro-3-(propylsulfonamido)benzamide

Step A: 2-(4-Chloro-3-(trifluoromethyl)phenyl)-6-nitro-3H-imidazo[4,5-b]pyridine (91%) was prepared according to the general procedure in Example 2, Step A, substituting 4-chloro-3-trifluoromethyl benzoic acid for benzoic acid. *m*/*z* (LC-MS) M+1 = 343.6.

Step B: 2-(4-Chloro-3-(trifluoromethyl)phenyl)-3H-imidazo[4,5-b]pyridin-6-amine was prepared according to the general procedure in Example 3, Step B, substituting 2-(4-chloro-3-(trifluoromethyl)phenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =312.6.

Step C: N-(2-(4-chloro-3-(trifluoromethyl)phenyl)-3H-imidazo[4,5-b]pyridin-6-yl)-2,6-difluoro-3-(propylsulfonamido)benzamide (14%) was prepared according to the general procedure in Example 1, Step E, substituting 2-(4-chloro-3-(trifluoromethyl)phenyl)-3H-imidazo[4,5-b]pyridin-6-amine for 3H-imidazo[4,5-b]pyridin-6-amine. *m*/*z* (LC-MS) M+1 = 574.9.

### Example 38

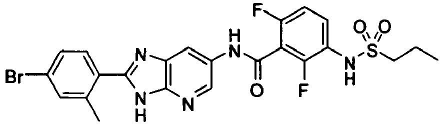

### N-(2-(4-bromo-2-methylphenyl)-3H-imidazo[4,5-b]pyridin-6-yl)-2,6-difluoro-3-(propylsulfonamido)benzamide

Step A: 2-(4-Bromo-2-methylphenyl)-6-nitro-3H-imidazo[4,5-b]pyridine (78%) was prepared according to the general procedure in Example 2, Step A, substituting 4-bromo-2-methyl benzoic acid for benzoic acid. *m*/*z* (LC-MS) M+1 = 334.1.

Step B: 2-(4-Bromo-2-methylphenyl)-3H-imidazo[4,5-b]pyridin-6-amine was prepared according to the general procedure in Example 3, Step B, substituting 2-(4-bromo-2-methylphenyl)-6-nitro-3H-imidazo[4,5-b]pyridine for N,N-dimethyl-4-(6-nitro-3H-imidazo[4,5-b]pyridin-2-yl)aniline. *m*/*z* (LC-MS) M+1 =304.2.

Step C: N-(2-(4-Chloro-3-(trifluoromethyl)phenyl)-3H-imidazo[4,5-b]pyridin-6-yl)-2,6-difluoro-3-(propylsulfonamido)benzamide (21 %) was prepared according to the general procedure in Example 1, Step E, substituting 2-(4-bromo-2-methylphenyl)-3H-imidazo[4,5-b]pyridin-6-amine for 3H-imidazo[4,5-b]pyridin-6-amine. *m*/*z* (LC-MS) M+1 = 565.4.

The following compounds in Table 1 were prepared following the above procedures.

**Table 1**

| Ex. # | Structure | Name | MS / NMR |
|---|---|---|---|
| 39 | 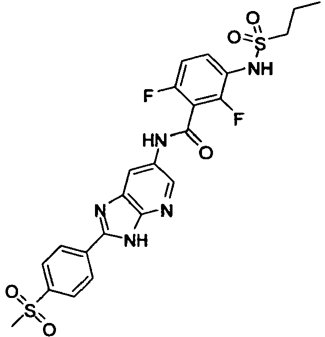 | 2,6-difluoro-N-(2-(4-(methylsulfonyl)phenyl )-3H-imidazo[4,5-b]pyridin-6-yl)-3-(propylsulfonamido)benzamide | MH+550.1 |
| 40 | 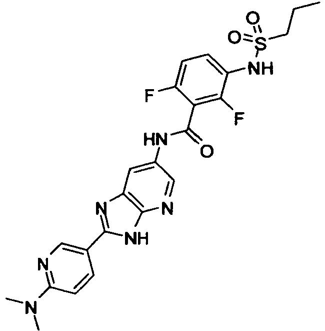 | N-(2-(6-(dimethylamino)pyridin -3-yl)-3H-imidazo[4,5-b]pyridin-6-yl)-2,6-difluoro-3-(propylsulfonamido)benzamide | MH+ 516.2 |
| 41 | 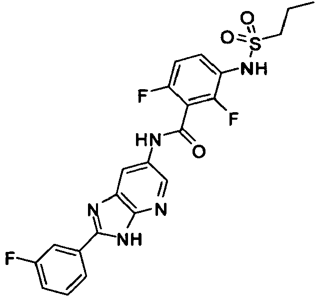 | 2,6-difluoro-N-(2-(3-fluorophenyl)-3H-imidazo[4,5-b]pyridin-6-yl)-3-(propylsulfonamido)benzamide | MH+ 490.1 |
| 42 | 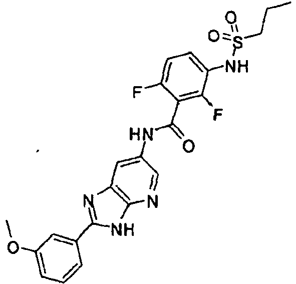 | 2,6-difluoro-N-(2-(3-methoxyphenyl)-3H-imidazo[4,5-b]pyridin-6-yl)-3-(propylsulfonamido)benzamide | MH+ 502.2 |

While the invention has been described in conjunction with the enumerated embodiments, it will be understood that they are not intended to limit the invention to those embodiments. On the contrary, the invention is intended to cover all alternatives, modifications and equivalents, included within the scope of the present invention as defined by the claims. Thus, the foregoing description is considered as illustrative only of the principles of the invention.

The words "comprise," "comprising," "include," "including," and "includes" when used in this specification and in the following claims are intended to specify the presence of stated features, integers, components, or steps, but they do not preclude the presence or addition of one or more other features, integers, components, steps, or groups thereof.

## Claims

1. A compound selected from Formula I: 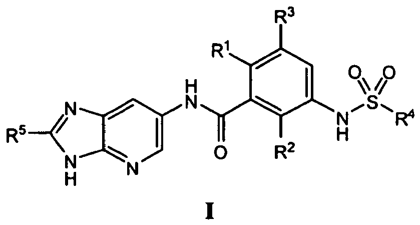 and stereoisomers and pharmaceutically acceptable salts thereof, wherein:
R¹ and R² are independently selected from hydrogen, halogen, CN, C₁-C₃ alkyl, and C₁-C₃ alkoxy;
R³ is hydrogen, halogen or C₁-C₃ alkyl;
R⁴ is C₃-C₅ cycloalkyl, C₁-C₆ alkyl, C₁-C₆ alkenyl, or C₁-C₆ alkynyl, wherein the cycloalkyl, alkyl, alkenyl and alkynyl are optionally substituted with OR^{c}, halogen or C₃-C₄ cycloalkyl;
R⁵ is hydrogen, phenyl optionally substituted with one to three R^{a} groups, -N(R^{c})-phenyl optionally substituted with R^{a}, -CH₂-phenyl optionally substituted with one to three R^{b} groups, a 5-6 membered heteroaryl optionally substituted with one to three R^{e} groups, saturated or partially unsaturated C₃-C₆ cycloalkyl optionally substituted with halogen or C₁-C₄ alkyl, a 5-6 membered heterocyclyl, or C₁-C₆ alkyl optionally substituted with one or more R^{g} groups;
each R^{a} is independently selected from halogen, CN, a 5-6 membered heterocyclyl, NR^{c}R^{d}, -S(O)₂R^{f}, -O(C₁-C₄ alkyl), and C₁-C₄ alkyl, wherein the alkyl or alkoxy are optionally substituted with halogen;
each R^{b} is independently selected from halogen, OH or OCH₃;
each R^{c} and R^{d} are independently selected from hydrogen or C₁-C₄ alkyl;
R^{e} is selected from a 5-6 membered heterocyclyl or NR^{c}R^{d};
R^{f} is selected from C₁-C₄ alkyl or NR^{c}R^{d}; and
each R^{g} is independently selected from halogen, CN, OR^{c}, C₃-C₆ cycloalkyl or NR^{c}R^{d}_{.}

2. A compound of Claim 1, wherein:
R¹, R² and R³ are independently selected from H, halogen or C₁-C₃ alkyl;
R⁴ is C₃-C₄ cycloalkyl, or C₁-C₆ alkyl optionally substituted with OH, halogen or C₃-C₄ cycloalkyl;
R⁵ is hydrogen, phenyl optionally substituted with one to three R^{a} groups, -NH-phenyl, -CH₂-phenyl optionally substituted with one to three R^{b} groups, a 5-6 membered heteroaryl optionally substituted with one to three R^{e} groups, C₃-C₆ cycloalkyl, a 5-6 membered heterocyclyl, or C₁-C₆ alkyl;
each R^{a} is independently selected from halogen, CN, a 5-6 membered heterocyclyl, NR^{c}R^{d}, -S(O)₂R^{f}, -O(C₁-C₄ alkyl), and C₁-C₄ alkyl, wherein the alkyl or alkoxy are optionally substituted with halogen;
each R^{b} is independently selected from halogen, OH or OCH₃;
each R^{c} and R^{d} are independently selected from hydrogen or C₁-C₄ alkyl;
R^{e} is selected from a 5-6 membered heterocyclyl or NR^{c}R^{d}; and
R^{f} is selected from C₁-C₄ alkyl or NR^{c}R^{d}.

3. A compound as claimed in any one of Claims 1 or 2, wherein R¹, R² and R³ are independently selected from hydrogen, halogen or C₁-C₃ alkyl.

4. A compound as claimed in any one of Claims 1 to 3, wherein the residue: 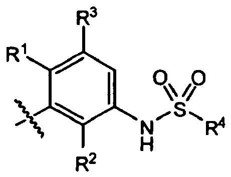 of Formula **I**, wherein the wavy line represents the point of attachment of the residue in Formula **I**, is selected from:

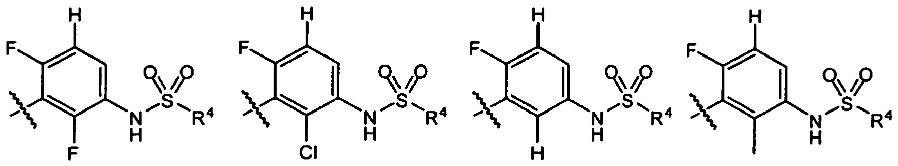

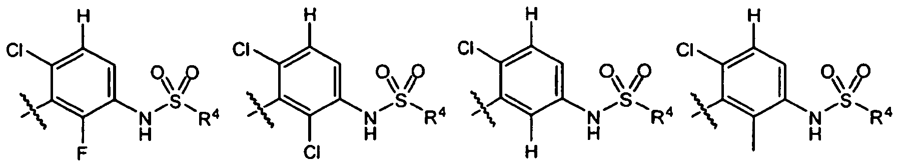

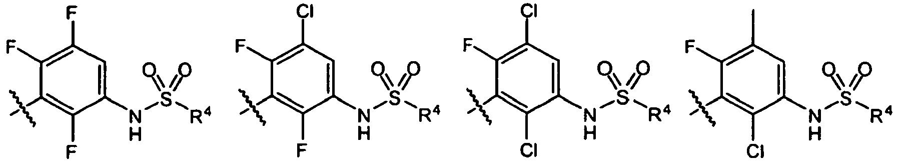

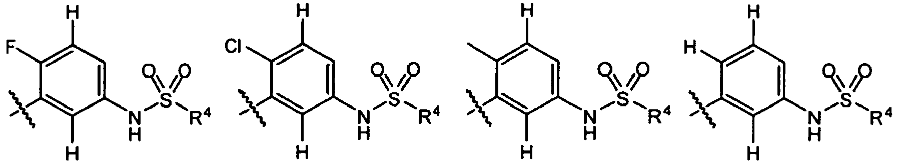

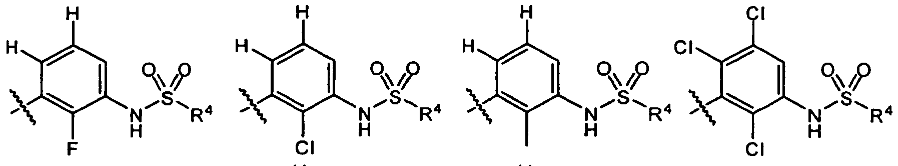

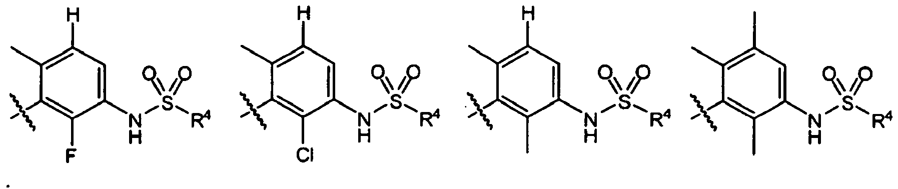

5. A compound as claimed in any one of Claims 1 to 4, wherein
R¹ and R² are F and R³ is hydrogen, or R¹, R² and R³ are F, or
R¹ is F and R² is Cl and R³ is hydrogen, or R¹ is Cl and R² is F and R³ is hydrogen.

6. A compound as claimed in any one of Claims 1 to 4, wherein R¹ is F and R² is methyl and R³ is hydrogen, or R¹ is methyl and R² is F and R³ is hydrogen.

7. A compound as claimed in any one of Claims 1 to 4, wherein R¹ is F and R² and R³ are hydrogen, or R¹ is Cl and R² and R³ are hydrogen.

8. A compound as claimed in any one of Claims 1 to 4, wherein R² is F and R¹ and R³ are hydrogen.

9. A compound as claimed in any one of Claims 1 to 8, wherein R⁴ is propyl, butyl, isobutyl, -CH₂CH₂CH₂F, -CH₂CH₂CF₃ or cyclopropylmethyl.

10. A compound as claimed in any one of Claims 1 to 8, wherein R⁴ is -CF₃, - CH₂CF₃, -CH₂CH₂CH₂F, -CH₂CH₂CF₃, -CF₂CF₃ or -CF₂CF₂CF₃.

11. A compound as claimed in Claim 1, wherein R⁵ is selected from hydrogen, phenyl, 2-fluorophenyl, 2-chlorophenyl, 2-bromophenyl, 3-fluorophenyl, 3-chlorophenyl, 3-bromophenyl, 3-trifluoromethylphenyl, 3-methoxyphenyl, 4-fluorophenyl, 4-chlorophenyl, 4-bromophenyl, 4-cyanophenyl, 4-methylphenyl, 4-trifluoromethylphenyl, 4-methoxyphenyl, 4-trifluoromethoxyphenyl, 4-dimethylaminophenyl, 4-(methylsulfonyl)phenyl, 4-morpholinophenyl, 3,4-dichlorophenyl, 3-trifluoromethyl-4-methylphenyl, 3-trifluoromethyl-4-chlorophenyl, 2-methyl-4-bromophenyl, NH-phenyl, -CH₂-4-chlorophenyl, -CH₂-3,5-difluorophenyl, -CH₂-4-methoxyphenyl, -CH₂-4-bromophenyl, -CH₂-3,4-difluorophenyl, pyridin-3-yl, pyridin-4-yl, 6-morpholinopyridin-3-yl, 6-(dimethylamino)pyridin-3-yl, cyclobutyl, piperdin-3-yl, piperdin-4-yl, methyl, ethyl, isopropyl and tert-butyl.

12. A compound of Formula **I** as defined in Claim 1 and having the structure:

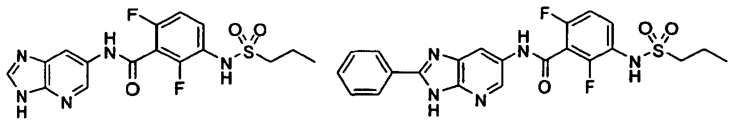

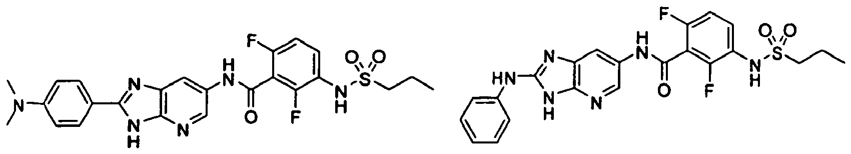

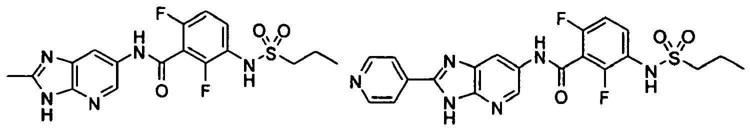

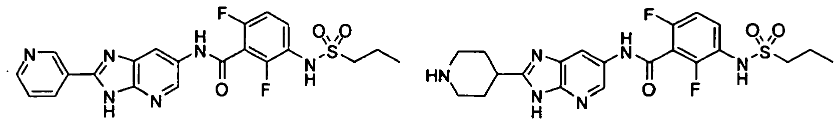

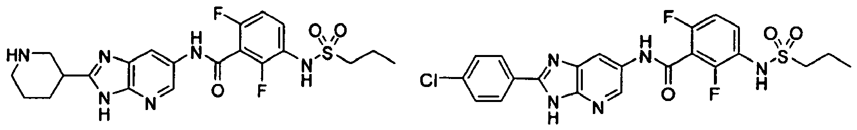

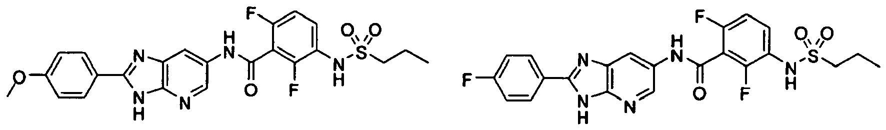

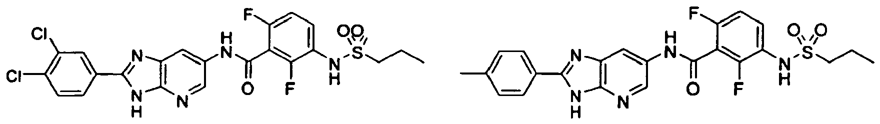

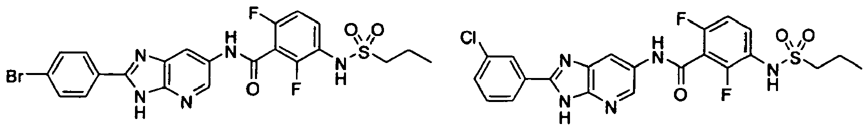

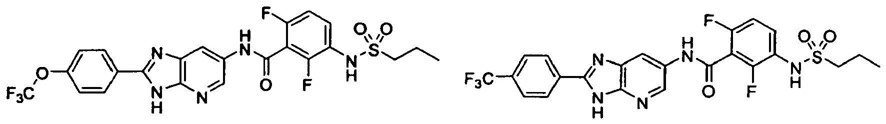

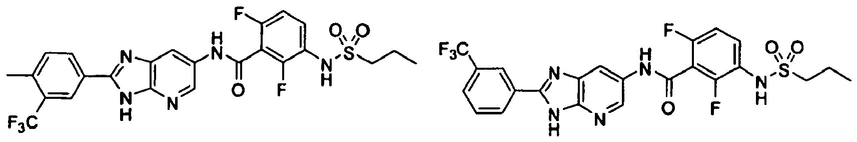

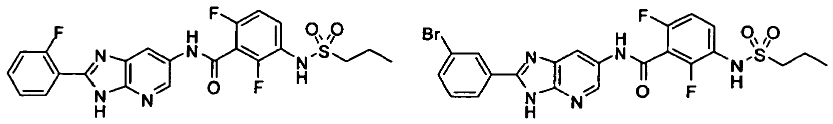

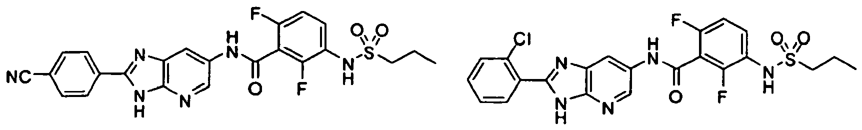

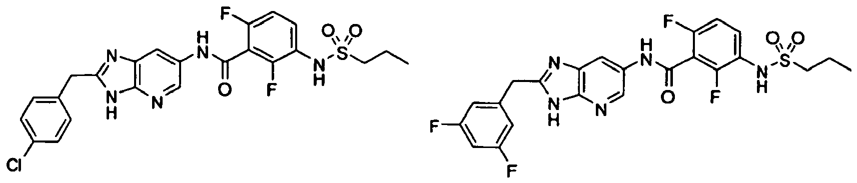

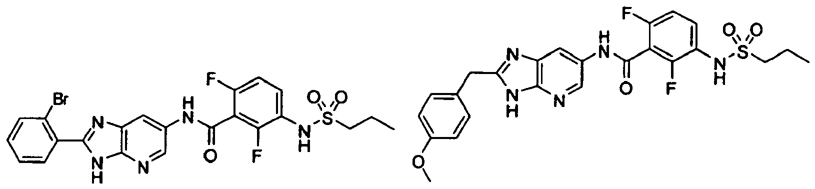

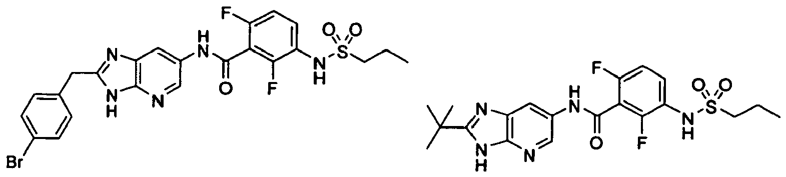

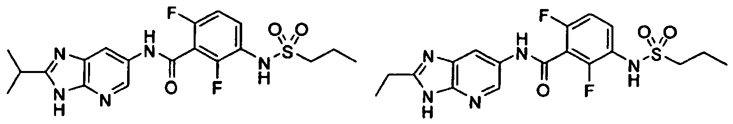

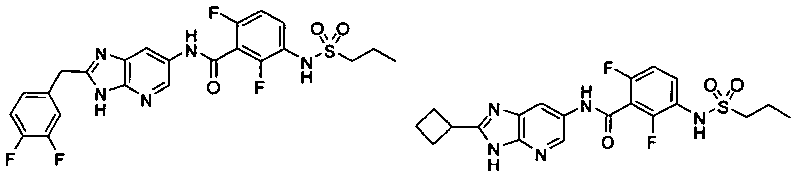

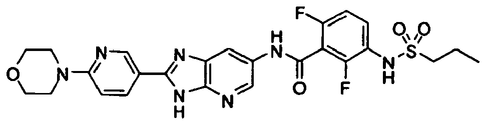

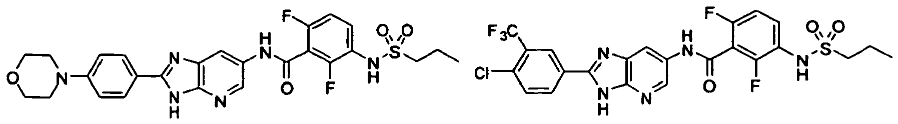

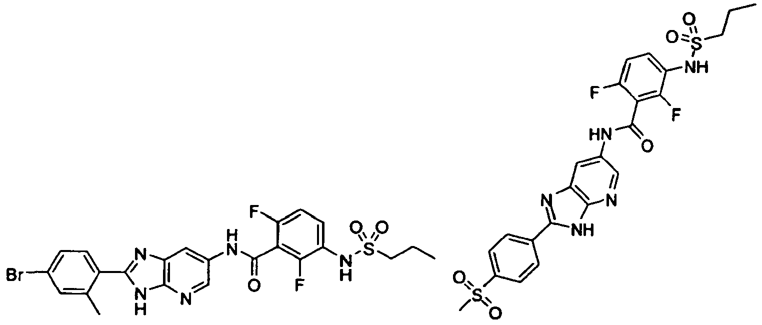

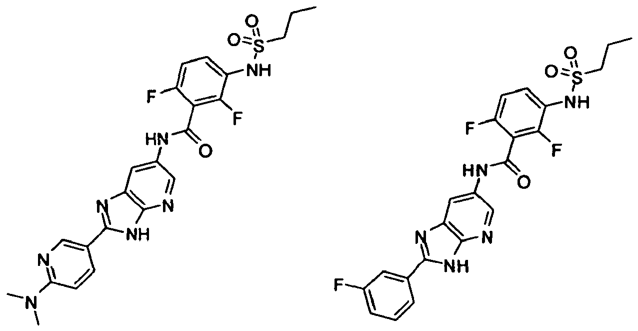

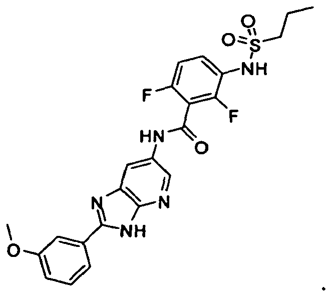

13. A pharmaceutical composition, comprising a compound as claimed in any one of Claims I to 12, and a pharmaceutically acceptable carrier or excipient.

14. A compound as claimed in any one of Claims 1 to 12 for use in therapy.

15. A compound as claimed in any one of Claims 1 to 12 for use in:
the treatment or prevention of cancer; or
the treatment of a hyperproliferative disease; or
the treatment or prevention of a kidney disease.

## Patentansprüche

1. Verbindung, ausgewählt aus Formel I: 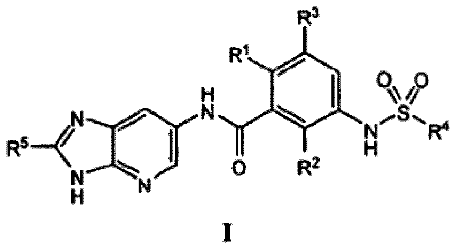 sowie Stereoisomeren und pharmazeutisch annehmbaren Salzen davon, worin:
R¹ und R² unabhängig voneinander aus Wasserstoff, Halogen, CN, C₁-C₃-Alkyl und C₁-C₃-Alkoxy ausgewählt sind ;
R³ Wasserstoff, Halogen oder C₁-C₃-Alkyl ist;
R⁴ C₃-C₅-Cycloalkyl, C₁-C₆-Alkyl, C₁-C₆-Alkenyl oder C₁-C₆-Alkinyl ist, worin das Cycloalkyl, Alkyl, Alkenyl und Alkinyl gegebenenfalls mit OR^{c}, Halogen oder C₃-C₄-Cycloalkyl substituiert sind;
R⁵ Wasserstoff, gegebenenfalls mit einer bis drei R^{a}-Gruppen substituiertes Phenyl, gegebenenfalls mit R^{a} substituiertes -N(R^{c})-Phenyl, gegebenenfalls mit ein bis drei R^{b}-Gruppen substituiertes -CH₂-Phenyl, gegebenenfalls mit ein bis drei R^{e}-Gruppen substituiertes, 5- bis 6-gliedriges Heteroaryl, gegebenenfalls mit Halogen oder C₁-C₄-Alkyl substituiertes, gesättigtes oder teilweise ungesättigtes C₃-C₆-Cycloalkyl, ein 5- bis 6-gliedriges Heterocyclyl oder gegebenenfalls mit einer oder mehr R^{g}-Gruppen substituiertes C₁-C₆-Alkyl ist;
die R^{a} unabhängig voneinander aus Halogen, CN, 5- bis 6-gliedrigem Heterocyclyl, NR^{c}R^{d}, -S(O)₂R^{f}, -O(C₁-C₄-Alkyl) und C₁-C₄-Alkyl ausgewählt sind, wobei das Alkyl oder Alkoxy gegebenenfalls mit Halogen substituiert ist;
die R^{b} unabhängig voneinander aus Halogen, OH und OCH₃ ausgewählt sind;
die R^{c} und R^{d} unabhängig voneinander aus Wasserstoff und C₁-C₄-Alkyl ausgewählt sind;
R^{e} aus 5- bis 6-gliedrigem Heterocyclyl und NR^{c}R^{d} ausgewählt ist;
R^{f} aus C₁-C₄-Alkyl und NR^{c}R^{d} ausgewählt ist; und
die R^{g} unabhängig voneinander aus Halogen, CN, OR^{c}, C₃-C₆-Cycloalkyl und NR^{c}R^{d} ausgewählt sind.

2. Verbindung nach Anspruch 1, worin:
R¹, R² und R³ unabhängig voneinander aus H, Halogen und C₁-C₃-Alkyl ausgewählt sind;
R⁴ C₃-C₄-Cycloalkyl oder C₁-C₆-Alkyl, gegebenenfalls mit OH, Halogen oder C₃-C₄-Cycloalkyl substituiert, ist;
R⁵ Wasserstoff, gegebenenfalls mit ein bis drei R^{a}-Gruppen substituiertes Phenyl, -NH-Phenyl, gegebenenfalls mit ein bis drei R^{b}-Gruppen substituiertes -CH₂-Phenyl, gegebenenfalls mit ein bis drei R^{e}-Gruppen substituiertes, 5- bis 6-gliedriges Heteroaryl, C₃-C₆-Cycloalkyl, 5- bis 6-gliedriges Heterocyclyl oder C₁-C₆-Alkyl ist;
die R^{a} unabhängig voneinander aus Halogen, CN, 5- bis 6-gliedrigem Heterocyclyl, NR^{c}R^{d}, -S(O)₂R^{f}, -O(C₁-C₄-Alkyl) und C₁-C₄-Alkyl ausgewählt sind, wobei das Alkyl oder Alkoxy gegebenenfalls mit Halogen substituiert ist;
die R^{b} unabhängig voneinander aus Halogen, OH und OCH₃ ausgewählt sind;
die R^{c} und R^{d} unabhängig voneinander aus Halogen und C₁-C₄-Alkyl ausgewählt sind;
R^{e} aus 5- bis 6-gliedrigem Heterocyclyl und NR^{c}R^{d} ausgewählt ist; und
R^{f} aus C₁-C₄-Alkyl und NR^{c}R^{d} ausgewählt ist.

3. Verbindung nach einem der Ansprüche 1 oder 2, worin R¹, R² und R³ unabhängig voneinander aus Wasserstoff, Halogen und C₁-C₃-Alkyl ausgewählt sind.

4. Verbindung nach einem der Ansprüche 1 bis 3, worin der Rest 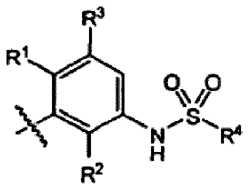 der Formel I, worin die Wellenlinie für den Anbindungspunkt des Rests in Formel I steht, ausgewählt ist aus:

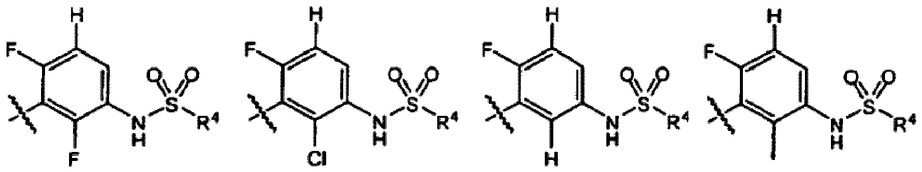

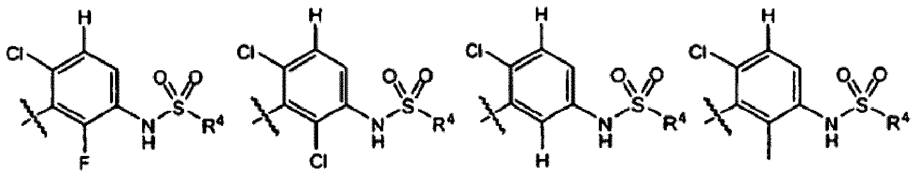

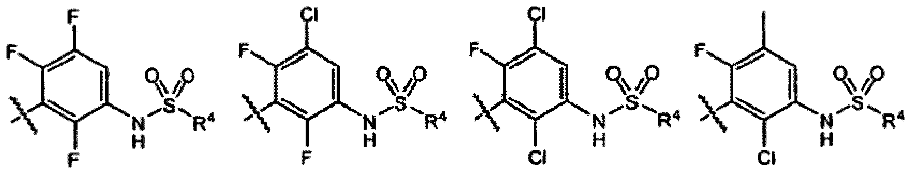

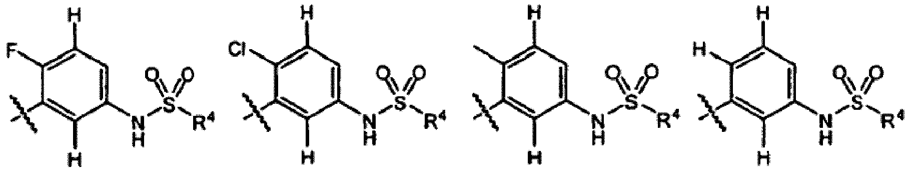

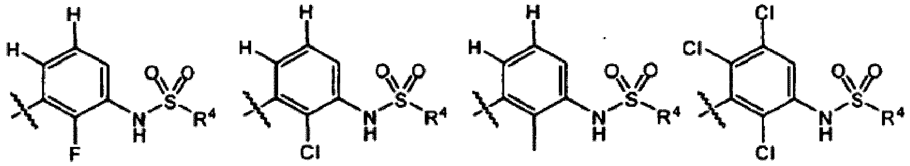

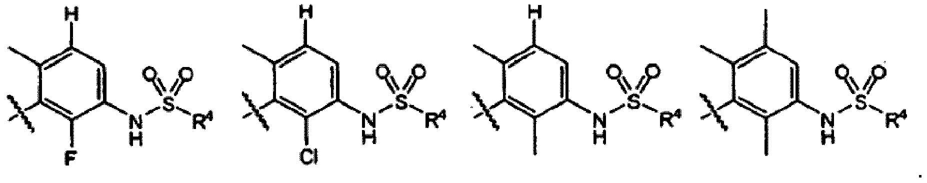

5. Verbindung nach einem der Ansprüche 1 bis 4, worin
R¹ und R² F sind und R³ Wasserstoff ist oder R¹, R² und R³ F sind oder
R¹ F ist und R² Cl ist und R³ Wasserstoff ist oder R¹ Cl ist und R² F ist und R³ Wasserstoff ist.

6. Verbindung nach einem der Ansprüche 1 bis 4, worin R¹ F ist und R² Methyl ist und R³ Wasserstoff ist oder R¹ Methyl ist und R² F ist und R³ Wasserstoff ist.

7. Verbindung nach einem der Ansprüche 1 bis 4, worin R¹ F ist und R² und R³ Wasserstoff sind oder R¹ Cl ist und R² und R³ Wasserstoff sind.

8. Verbindung nach einem der Ansprüche 1 bis 4, worin R² F ist und R¹ und R³ Wasserstoff sind.

9. Verbindung nach einem der Ansprüche 1 bis 8, worin R⁴ Propyl, Butyl, Isobutyl, - CH₂CH₂CH₂F, -CH₂CH₂CF₃ oder Cyclopropylmethyl ist.

10. Verbindung nach einem der Ansprüche 1 bis 8, worin R⁴ -CF₃, -CH₂CF₃, -CH₂CH₂CH₂F, -CH₂CH₂CF₃, -CF₂CF₃ oder -CF₂CF₂CF₃ ist.

11. Verbindung nach Anspruch 1, worin R⁵ aus Wasserstoff, Phenyl, 2-Fluorphenyl, 2-Chlorphenyl, 2-Bromphenyl, 3-Fluorphenyl, 3-Chlorphenyl, 3-Bromphenyl, 3-Trifluormethylphenyl, 3-Methoxyphenyl, 4-Fluorphenyl, 4-Chlorphenyl, 4-Bromphenyl, 4-Cyanophenyl, 4-Methylphenyl, 4-Trifluormethylphenyl, 4-Methoxyphenyl, 4-Trifluormethoxyphenyl, 4-Dimethylaminophenyl, 4-(Methylsulfonyl)phenyl, 4-Morpholinophenyl, 3,4-Dichlorphenyl, 3-Trifluormethyl-4-methylphenyl, 3-Trifluormethyl-4-chlorphenyl, 2-Methyl-4-bromphenyl, -NH-Phenyl, -CH₂-4-Chlorphenyl, -CH₂-3,5-Difluorphenyl, -CH₂-4-Methoxyphenyl, -CH₂-4-Bromphenyl, -CH₂-3,4-Difluorphenyl, Pyridin-3-yl, Pyridin-4-yl, 6-Morpholinopyridin-3-yl, 6-(Dimethylamino)pyridin-3-yl, Cyclobutyl, Piperdin-3-yl, Piperdin-4-yl, Methyl, Ethyl, Isopropyl und tert-Butyl ausgewählt ist.

12. Verbindung der Formel I nach Anspruch 1 mit der folgenden Struktur:

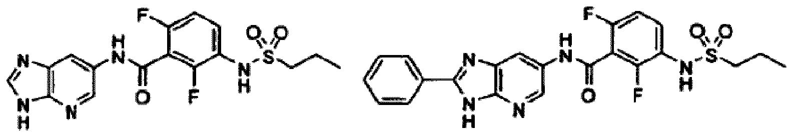

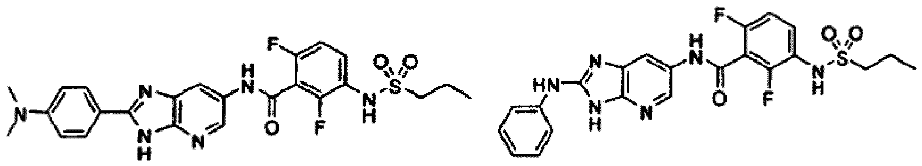

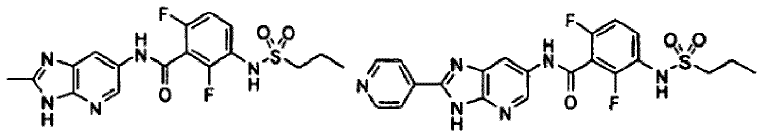

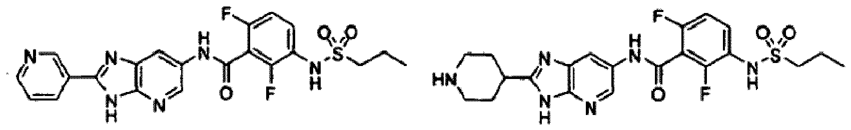

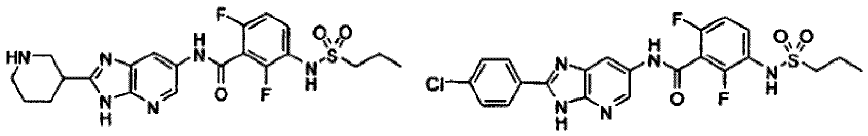

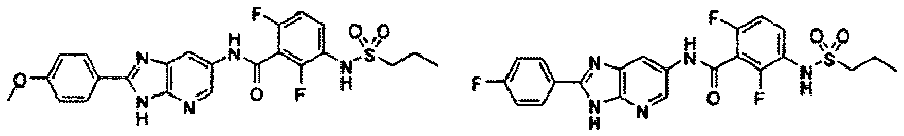

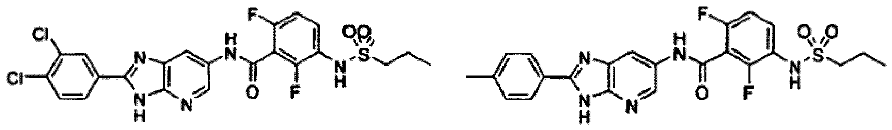

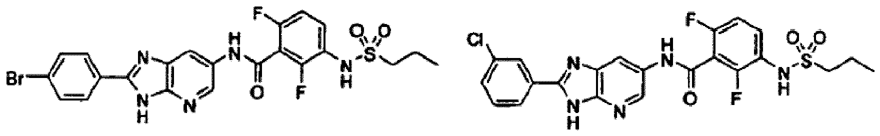

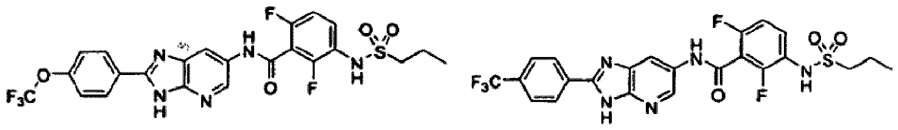

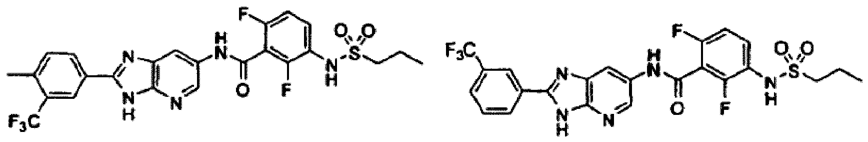

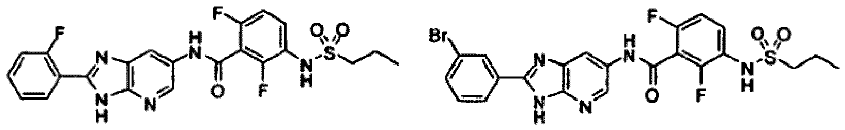

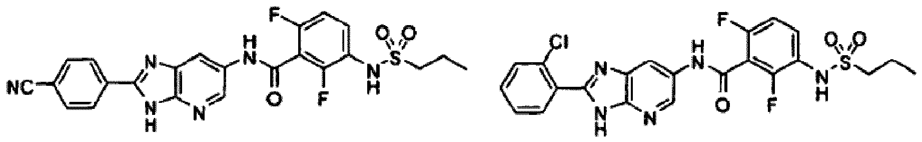

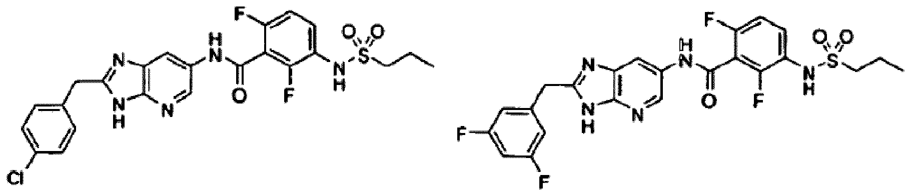

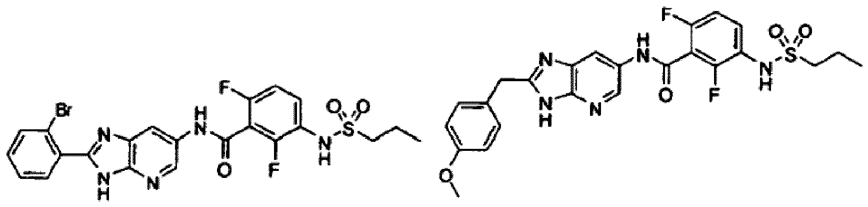

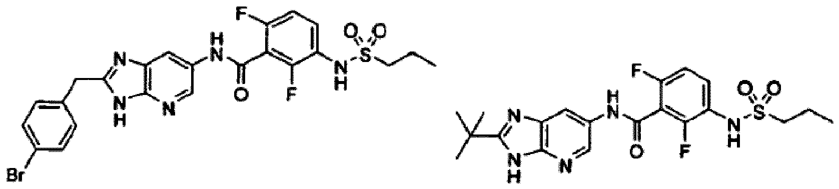

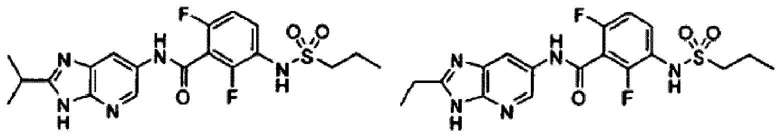

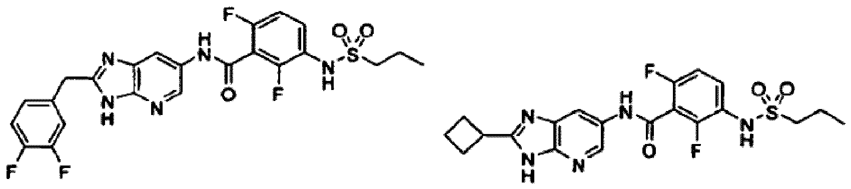

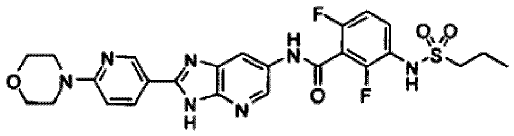

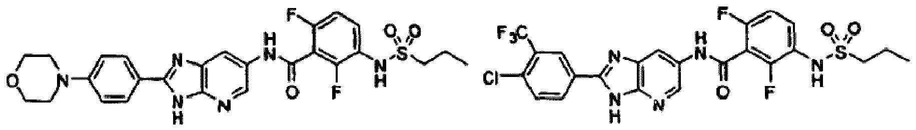

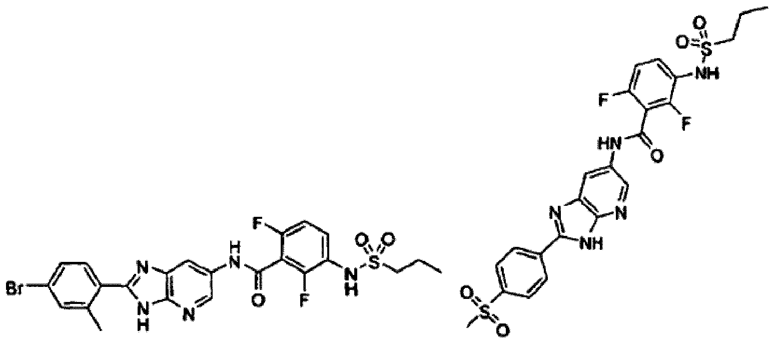

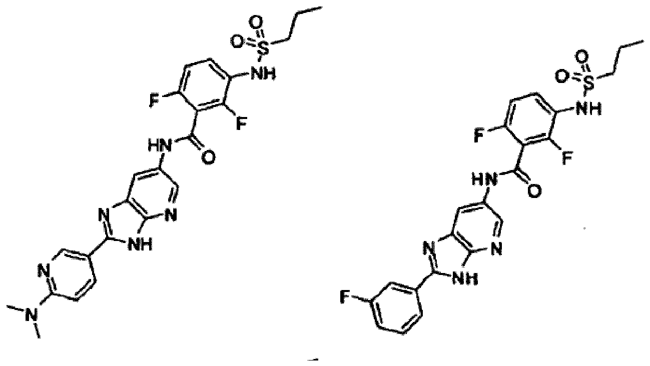

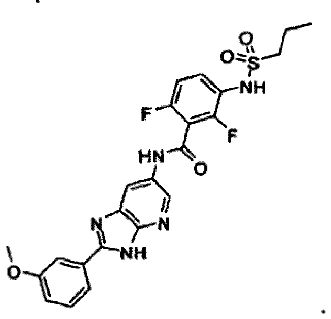

13. Pharmazeutische Zusammensetzung, die eine Verbindung nach einem der Ansprüche 1 bis 12 und einen pharmazeutisch annehmbaren Träger oder Exzipienten umfasst.

14. Verbindung nach einem der Ansprüche 1 bis 12 zur Verwendung in einer Therapie.

15. Verbindung nach einem der Ansprüche 1 bis 12 zur Verwendung
bei der Behandlung von oder Vorbeugung gegen Krebs; oder
bei der Behandlung einer Hyperproliferationserkrankung; oder
bei der Behandlung einer oder Vorbeugung gegen eine Nierenerkrankung.

## Revendications

1. Composé sélectionné de la Formule I : 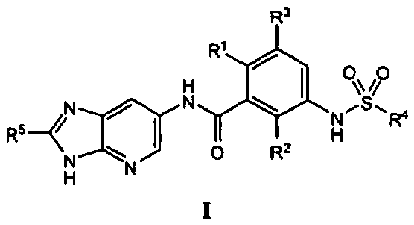 et des stéréoisomères et sels pharmaceutiquement acceptables de celui-ci, où:
R¹ et R² sont indépendamment sélectionnés parmi hydrogène, halogène, CN, alkyleC₁-C₃ et alcoxyC₁-C₃;
R³ est hydrogène, halogène ou alkyleC₁-C₃;
R⁴ est cycloalkyle C₃-C₅, alkyleC₁-C₆, alkényleC₁-C₆ ou alkynyleC₁-C₆, ou cycloalkyle, alkyle, alkényle et alkynyle sont optionnellement substitués par OR^{c}, halogène ou cycloalkyleC₃-C₄ ;
R⁵ est hydrogène, phényle optionnellement substitué par un à trois groupes R^{α}, -N(R^{c})-phényle optionnellement substitué par R^{α}, -CH₂-phényle optionnellement substitué par un à trois groupes R^{b}, un hétéroaryle de 5 à 6 membres optionnellement substitué par un à trois groupes R^{e}, cycloalkyleC₃-C₆ saturé ou partiellement insaturé, optionnellement substitué par halogène ou alkyleC₁-C₄, un hétérocyclyle de 5-6 membres ou alkyleC₁-C₆ optionnellement substitué par un ou plusieurs groupes R^{g};
chaque R^{α} est indépendamment sélectionné parmi halogène, CN, un hétérocyclyle de 5-6 membres, NR^{c}R^{d}, -S(O)₂R^{f}, -O(alkyleC₁-C₄), et alkyleC₁-C₄; où l'alkyle ou l'alkoxy sont optionnellement substitués par halogène;
chaque R^{b} est indépendamment sélectionné parmi halogènen, OH ou OCH₃;
chaque R^{c} et R^{d} sont indépendamment sélectionnés parmi hydrogène ou alkyleC₁-C₄;
R^{e} est sélectionné parmi un hétérocyclyle de 5 à 6 membres ou NR^{c}R^{d};
R^{f} est sélectionné parmi alkyleC₁-C₄ ou NR^{c}R^{d}; et
chaque R^{g} est indépendamment sélectionné parmi halogène, CN, OR^{c}, cycloalkyleC₃-C₆ ou NR^{c}R^{d}.

2. Composé selon la revendication 1, où:
R¹, R² et R³ sont indépendamment sélectionnés parmi H, halogène ou alkyleC₁-C₃;
R⁴ est cycloalkyle C₃-C₄, ou alkyleC₁-C₆ optionnellement substitué par OH, halogène ou cycloalkyle C₃-C₄;
R⁵ est hydrogène, phényle optionnellement substitué par un à trois groupes R^{α}, -NH-phényle, -CH₂-phényle optionnellement substitué par un à trois groupes R^{b}, un hétéroaryle de 5 à 6 membres optionnellement substitué par un à trois groupes R^{e}, cycloalkyleC₃-C₆, un hétérocyclyle de 5-6 membres ou alkyleC₁-C₆;
chaque R^{a} est indépendamment sélectionné parmi halogène, CN, un hétérocyclyle de 5 à 6 membres, NR^{c}R^{d}, -S(O)₂R^{f}, -O(alkyleC₁-C₄), et alkyleC₁-C₄, où l'alkyle ou l'alkoxy sont optionnellement substitués par halogène;
chaque R^{b} est indépendamment sélectionné parmi halogène, OH ou OCH₃;
chaque R^{c} et R^{d} sont indépendamment sélectionnés parmi hydrogène ou alkyle C₁-C₄;
R^{e} est sélectionné parmi un hétérocyclyle de 5-6 membres ou NR^{c}R^{d}; et
R^{f} est sélectionné parmi alkyleC₁-C₄ ou NR^{c}R^{d}.

3. Composé selon l'une quelconque des revendications 1 ou 2, où R¹, R² et R³ sont indépendamment sélectionnés parmi hydrogène, halogène ou alkyleC₁-C₃.

4. Composé selon l'une quelconque des revendications 1 à 3, où le résidu: 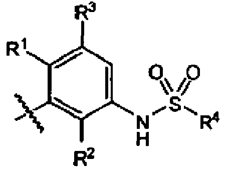 de la Formule I, où la ligne ondulée représente le point d'attachement du résidu dans la Formule I, est sélectionné parmi:

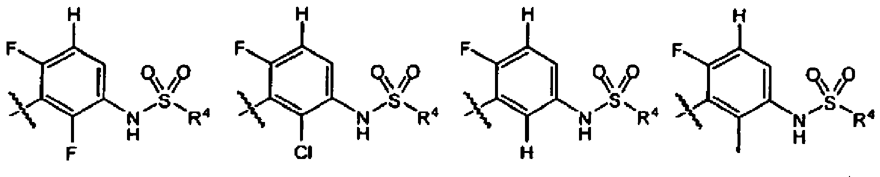

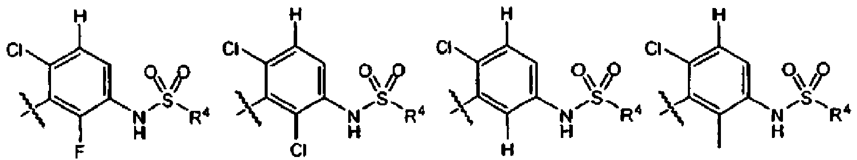

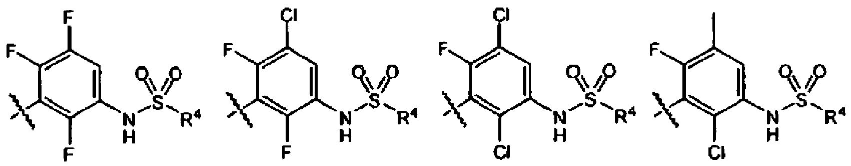

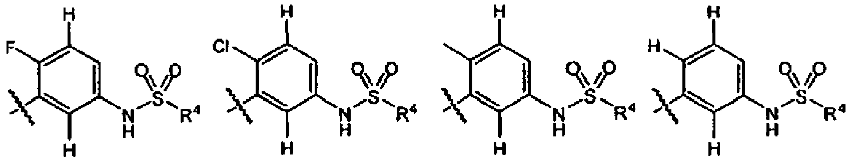

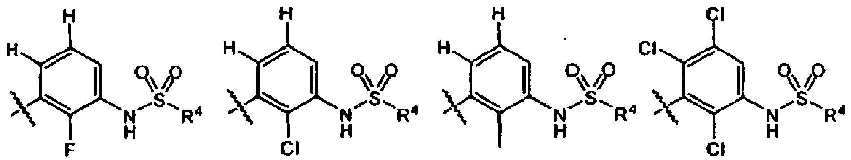

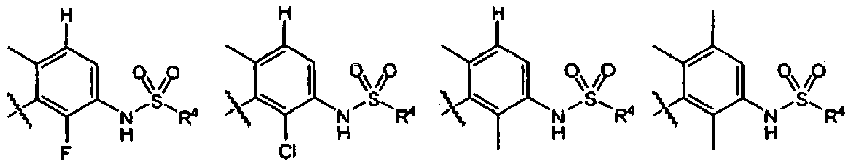

5. Composé selon l'une quelconque des revendications 1 à 4, où
R¹ et R² sont F et R³ est hydrogène, ou bien R¹, R² et R³ sont F, ou bien
R¹ est F et R² est Cl, et R³ est hydrogène, ou bien R¹ est Cl et R² est F et R³ est hydrogène.

6. Composé selon l'une quelconque des revendications 1 à 4, où R¹ est F et R² est méthyle, et R³ est hydrogène, ou bien R¹ est méthyle et R² est F et R³ est hydrogène.

7. Composé selon l'une quelconque des revendications 1 à 4, où R¹ est F, et R² et R³ sont hydrogène, ou bien R¹ est C1 et R² et R³ sont hydrogène.

8. Composé selon l'une quelconque des revendications 1 à 4, où R² est F, et R¹ et R³ sont hydrogène.

9. Composé selon l'une quelconque des revendications 1 à 8, où R⁴ est propyle, butyle, isobutyle, -CH₂CH₂CH₂F, -CH₂CH₂CF₃ ou cyclopropylméthyle.

10. Composé selon l'une quelconque des revendications 1 à 8, où R⁴ est -CF₃, -CH₂CF₃, -CH₂CH₂CH₂F, -CH₂CH₂CF₃, -CF₂CF₃ ou -CF₂CF₂CF₃.

11. Composé selon la revendication 1, où R⁵ est sélectionné parmi hydrogène, phényle, 2-fluorophényle, 2-chlorophényle, 2-bromophényle, 3-fluorophényle, 3-chlorophényle, 3-bromophényle, 3-trifluorométhylphényle, 3-méthoxyphényle, 4-fluorophényle, 4-chlorophényle, 4-bromophényle, 4-cyanophényle, 4-méthylphényle, 4-trifluorométhylphényle, 4-méthoxyphényle, 4-trifluoro-méthoxyphényle, 4-diméthyl-aminophényle, 4-(méthylsulfonyl)phényle, 4-morpholino-phényle, 3,4-dichlorophényle, 3-trifluorométhyl-4-méthylphényle, 3-trifluorométhyl-4-chlorophényle, 2-méthyl-4-bromophényle, -NH-phényle, -CH₂-4-chlorophényle, -CH₂-3,5-difluorophényle, - CH₂-4-méthoxyphényle, -CH₂-4- bromophényle, -CH₂-3,4-difluorophényle, pyridin-3-yle, pyridin-4-yle, 6-morpholinopyridin-3-yle, 6-(diméthylamino)pyridin-3-yle, cyclobutyle, pipéridin-3-yle, pipéridin-4-yle, méthyle, éthyle, isopropyle et tert-butyle.

12. Composé selon la Formule I tel que défini dans la revendication 1 et ayant la structure:

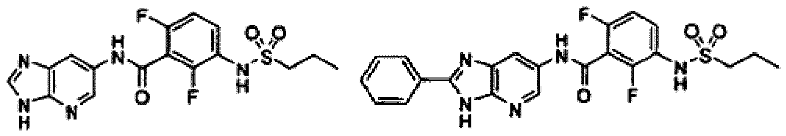

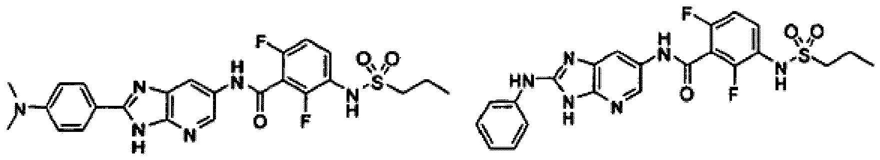

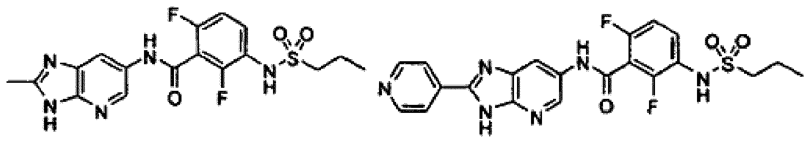

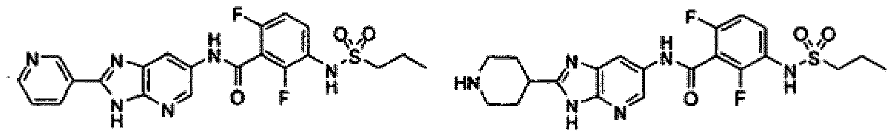

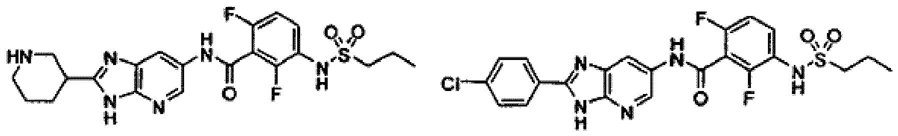

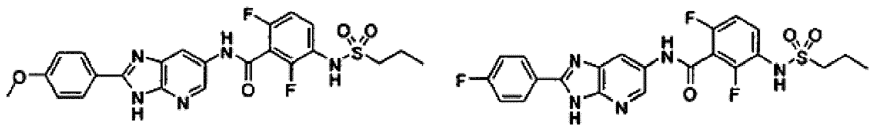

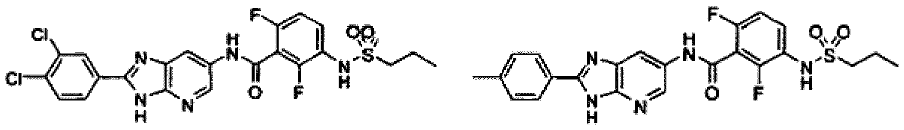

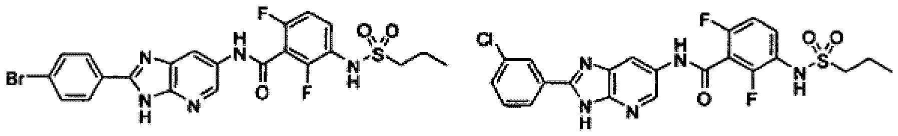

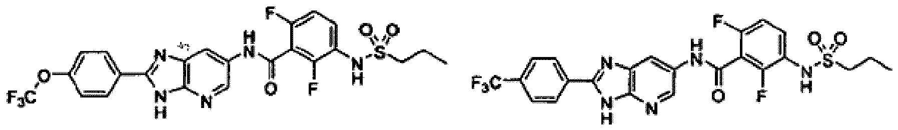

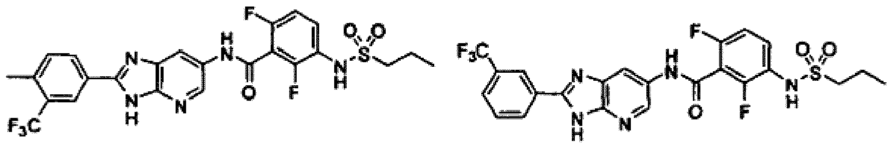

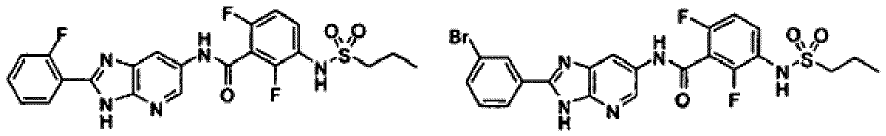

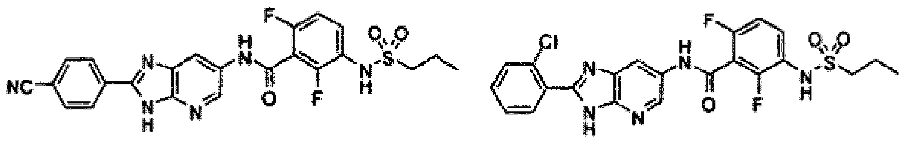

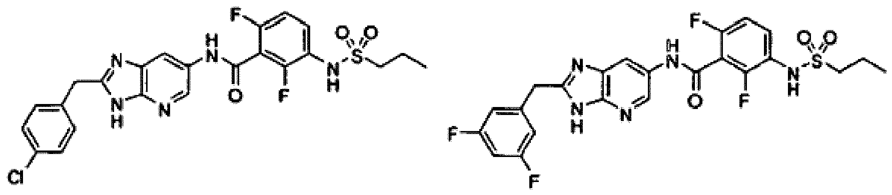

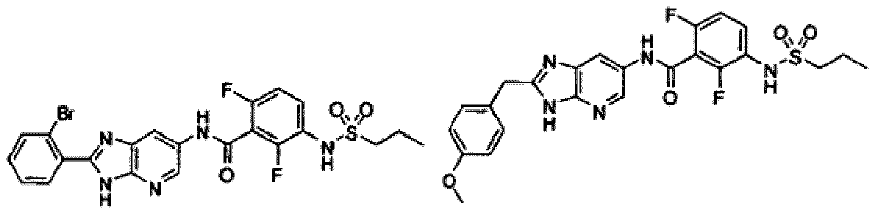

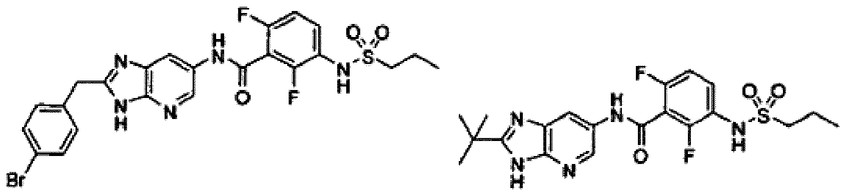

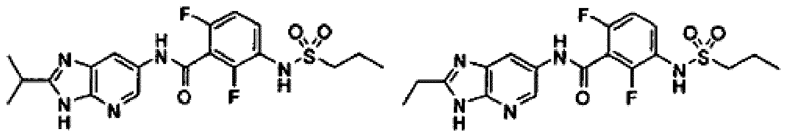

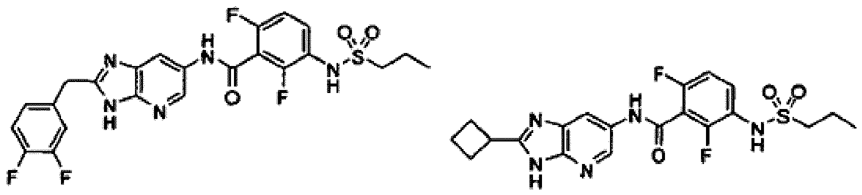

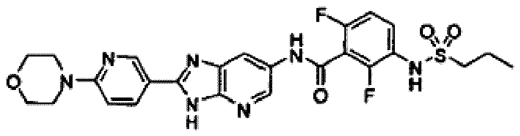

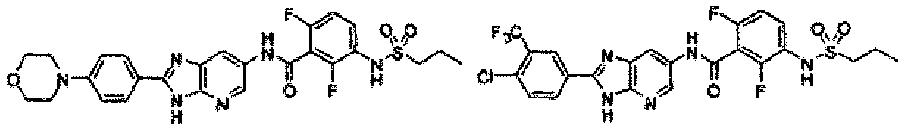

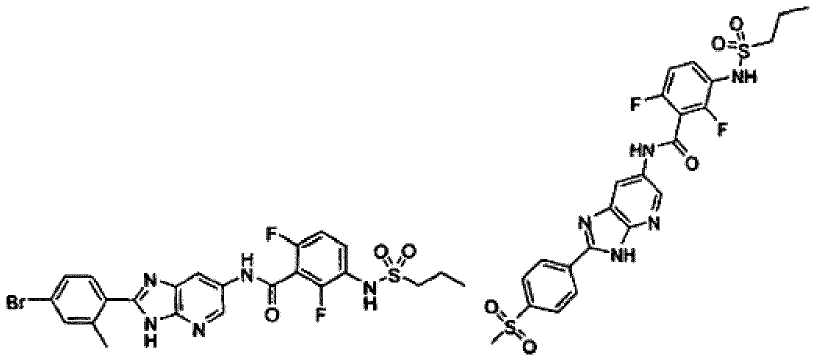

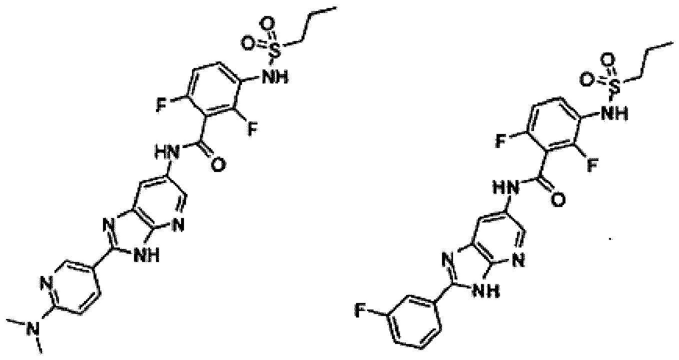

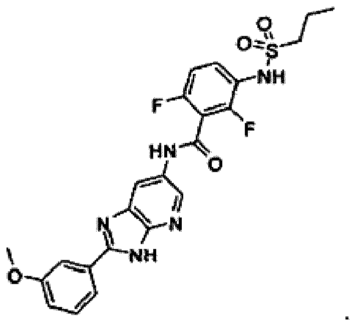

13. Composition pharmaceutique, comprenant un composé tel que revendiqué dans l'une quelconque des revendications 1 à 12, et un support ou excipient pharmaceutiquement acceptable.

14. Composé selon l'une quelconque des revendications 1 à 12 pour utilisation en thérapie.

15. Composé selon l'une quelconque des revendications 1 à 12 pour utilisation dans
le traitement ou la prévention du cancer; ou
le traitement d'une maladie hyperproliférante; ou
le traitement ou la prévention d'une maladie des reins.
